# EUROPEAN PATENT APPLICATION

(11) **EP 4 817 536 A1**
(43) Date of publication of application: **07.10.2026**
(21) Application number: 25793931.4
(22) Date of filing: 22.04.2025
(51) Int. Cl.: A61B 17/34, A61B 34/20

(54) **INTERVENTIONAL MEDICAL SYSTEM**

(30) Priority: 23.04.2024 CN 202410494718; 29.09.2024 CN 202411383994
(71) Applicant: Wuhan United Imaging Surgical Co., Ltd., Wuhan, Hubei 430206 (CN)
(72) Inventor: KE, Xianfeng, Wuhan, Hubei 430206 (CN); XIE, Qiang, Wuhan, Hubei 430206 (CN); ZHU, Longquan, Wuhan, Hubei 430206 (CN); QIAN, Shusheng, Wuhan, Hubei 430206 (CN); GE, Zhuang, Wuhan, Hubei 430206 (CN); LU, Zhuangzhuang, Wuhan, Hubei 430206 (CN)
(74) Representative: Wang, Bo
(86) International application number: PCT/CN2025/090479
(87) International publication number: WO 2025/223419

(57) **Abstract**

Provided is an interventional medical system (140, 200), which comprises a processor (110) and an operation execution end (240), wherein the operation execution end (240) comprises a surgical execution system (241), and the processor (110) is configured for: performing spatial registration on the interventional medical system (140, 200) to acquire registration information (910); acquiring related information and surgical information of a patient, and determining an operation plan of the surgical execution system (241) on the basis of the registration information, the related information, and the surgical information (920); and controlling the surgical execution system (241) to perform an operation on the basis of the surgical information and the operation plan (930).

## Description

### CROSS REFERENCE RELATED TO APPLICATION

This application claims a priority of Chinese Application No. 202410494718.4, filed on April 23, 2024 and Chinese Application No. 202411383994. X, filed on September 29, 2024, the contents of each of which are hereby incorporated by reference.

### FIELD OF THE INVENTION

The present disclosure relates to the field of medical instrument technology, and in particular, to an interventional medical system.

### BACKGROUND

Radioactive interventional puncture surgery is based on diagnostic imaging and, under image guidance, is used for the non-surgical treatment of diseases such as lung cancer, liver cancer, and mediastinal tumors, or for obtaining histological, physiological, and biochemical materials to clarify the nature of lesions. The radioactive interventional puncture surgery includes puncture biopsy, ablation, particle implantation, etc. Among these, puncture biopsy is the gold standard for tumor diagnosis, while ablation and particle implantation offer advantages such as minimally invasive procedures and favorable prognostic outcomes for tumor treatment. However, in traditional radioactive interventional puncture surgery, repeated scanning and confirmation of adjusted positions are required, resulting in low puncture accuracy.

Therefore, it is desirable to provide an interventional medical system that improves surgical precision and reduces the difficulty of surgical procedures.

### SUMMARY

According to an aspect of the present disclosure, an interventional medical system is provided. The system comprises a processor and an operation end effector. The operation end effector comprises a surgical execution system. The processor is configured to perform a spatial registration on the interventional medical system to obtain registration information, acquire relevant information and surgical information of a patient, determine a procedure plan for the surgical execution system based on the registration information, the relevant information, and the surgical information, and control the surgical execution system to perform a procedure based on the surgical information and the procedure plan.

According to an aspect of the present disclosure, an interventional medical system is provided. The system includes a processor. The interventional medical system is configured to be used with a medical radiation imaging system. The medical radiation imaging system comprises a scanning table, and the processor is configured to in response to a start signal, perform a spatial registration on the interventional medical system, and in response to completion of the spatial registration, control the scanning table to move into an imaging area of the medical radiation imaging system to a second position. The second position is a position of the scanning table when the medical radiation imaging system scans a patient. The processor is also configured to in response to completion of scanning the patient by the medical radiation imaging system, control the scanning table to move to an execution position, the patient undergoing a procedure at the execution position.

According to an aspect of the present disclosure, an interventional medical system is provided. The system comprises a processor and an operation end effector. The operation end effector comprises a surgical execution system. The processor is configured to acquire relevant information and surgical information of a patient, determine a procedure plan based at least on the relevant information and the surgical information, and control the surgical execution system to perform a procedure based on the surgical information and the procedure plan. The surgical information comprises a surgical mode, the procedure plan comprises a navigation positioning point, the procedure comprises a surgical procedure, the surgical execution system comprises a surgical operation assembly. The controlling the surgical execution system to perform a procedure based on the surgical information and the procedure plan comprises controlling the surgical operation assembly to move to the navigation positioning point, and after the surgical operation assembly moves to the navigation positioning point, controlling the surgical operation assembly to perform the surgical procedure based on the surgical mode.

### BRIEF DESCRIPTION OF THE DRAWINGS

The present disclosure will be further illustrated by way of exemplary embodiments, which are described in detail by means of the accompanying drawings. These embodiments are not limiting, and in these embodiments, the same numbering denotes the same structure, wherein:
FIG. 1 is a schematic diagram illustrating an exemplary application scenario of an interventional medical system according to some embodiments of the present disclosure.
FIG. 2 is a block diagram illustrating an interventional medical system according to some embodiments of the present disclosure.
FIG. 3A is a schematic diagram illustrating an active control end of an interventional medical system according to some embodiments of the present disclosure.
FIG. 3B is a schematic diagram illustrating an operation end effector of an interventional medical system according to some embodiments of the present disclosure.
FIG. 4 is a structural schematic diagram illustrating a master manipulator according to some embodiments of the present disclosure.
FIG. 5 is a structural schematic diagram illustrating a surgical execution arm according to some embodiments of the present disclosure.
FIG. 6 is a partial structural schematic diagram illustrating a base according to some embodiments of the present disclosure.
FIG. 7A is a schematic diagram illustrating reference coordinate systems of an operation end effector according to some embodiments of the present disclosure.
FIG. 7B is a schematic diagram illustrating reference coordinate systems of an operation end effector according to some embodiments of the present disclosure.
FIG. 8 is a schematic diagram illustrating transformations between reference coordinate systems according to some embodiments of the present disclosure.
FIG. 9 is a schematic flowchart illustrating an exemplary workflow of an interventional medical system according to some embodiments of the present disclosure.
FIGS. 10 and 11 are schematic flowcharts illustrating a process for determining a first mapping relationship according to some embodiments of the present disclosure.
FIG. 12 is a schematic diagram illustrating an operation interface for an optical imaging system to image a patient according to some embodiments of the present disclosure.
FIG. 13 is a schematic flowchart illustrating a process for determining target joint parameters according to some embodiments of the present disclosure.
FIG. 14 is a schematic flowchart illustrating a process for determining an executable path according to some embodiments of the present disclosure.
FIG. 15 is a schematic flowchart illustrating a process for determining an initial planned surgical pose according to some embodiments of the present disclosure.
FIG. 16 is a schematic flowchart illustrating a process for determining an initial planned auxiliary pose according to some embodiments of the present disclosure.
FIG. 17 is a schematic flowchart illustrating a process for controlling a surgical execution system based on a control command according to some embodiments of the present disclosure.
FIG. 18 is a schematic diagram illustrating master-slave control feedback according to some embodiments of the present disclosure.
FIG. 19 is a schematic diagram illustrating an operation interface for an auxiliary procedure according to some embodiments of the present disclosure.
FIG. 20 is a schematic diagram illustrating an operation interface for surgical preparation according to some embodiments of the present disclosure.
FIG. 21 is a schematic diagram illustrating an operation interface for a manual control mode according to some embodiments of the present disclosure.
FIG. 22 is a schematic diagram illustrating an operation interface when a master manipulator controls a surgical execution system according to some embodiments of the present disclosure.
FIG. 23 is an exemplary flowchart illustrating a user controlling an interventional medical system to perform a procedure, according to some embodiments of the present disclosure.

### DETAILED DESCRIPTION

In order to more clearly illustrate the technical solutions of the embodiments of the present disclosure, the accompanying drawings required to be used in the description of the embodiments are briefly described below. Obviously, the accompanying drawings in the following description are only some examples or embodiments of the present disclosure, and it is possible for a person of ordinary skill in the art to apply the present disclosure to other similar scenarios in accordance with these drawings without creative labor. The present disclosure may be applied to other similar scenarios based on these drawings without creative labor. Unless obviously obtained from the context or the context illustrates otherwise, the same numeral in the drawings refers to the same structure or operation.

It should be understood that the terms "system", "medical device" as used herein, "unit" and/or "module" as used herein is a way to distinguish between different components, elements, parts, sections or assemblies at different levels. However, said words may be replaced by other expressions if other words accomplish the same purpose.

As shown in the present disclosure and in the claims, unless the context clearly suggests an exception, the words "one," "a ", "an", and/or "the" do not refer specifically to the singular, but can also include the plural. Generally, the terms "including" and "comprising" suggest only the inclusion of clearly identified steps and elements. In general, the terms "including" and "comprising" only suggest the inclusion of explicitly identified steps and elements that do not constitute an exclusive list, and the method or medical device can also include other steps or elements.

Flowcharts are used in the present disclosure to illustrate operations performed by a system according to embodiments of the present disclosure. It should be appreciated that the preceding or following operations are not necessarily performed in an exact sequence. Instead, steps may be processed in reverse order or simultaneously. Also, it is possible to add other operations to these processes or remove a step or steps from them.

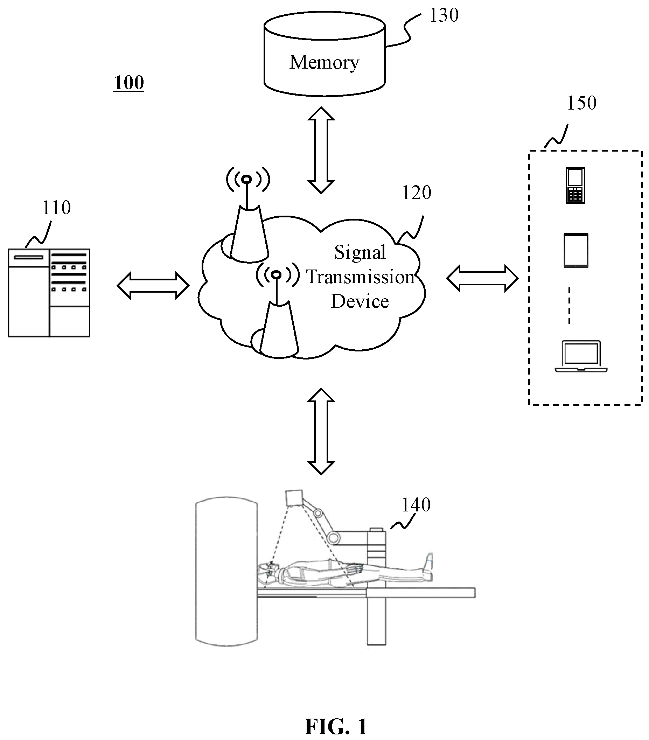

FIG. 1 is a schematic diagram illustrating an exemplary application scenario of an interventional medical system according to some embodiments of the present disclosure.

As shown in FIG. 1, in some embodiments, the application scenario 100 of an interventional medical system may include a processor 110, a signal transmission device 120, a memory 130, an interventional medical system 140, and a terminal device 150. In some embodiments, the processor 110 may be connected via the signal transmission device 120 to the memory 130, the interventional medical system 140, and/or the terminal device 150 to access and/or receive data and information. For example, the processor 110 may receive information related to the interventional medical system 140 (e.g., a procedure plan, control commands, enable information, etc.) via the signal transmission device 120. The application scenario 100 may control the procedure of the interventional medical system 140 by implementing the methods and/or processes disclosed in this specification.

The processor 110 may be used to process data and/or information from at least one component of the application scenario 100 or an external data source (e.g., a cloud data center). The processor 110 may be connected via the signal transmission device 120 to the memory 130, the interventional medical system 140, and/or the terminal device 150 to access and/or receive data and information. For example, the processor 110 may receive information related to operational control of the interventional medical system 140 (e.g., a procedure plan, control commands, enable information, etc.) via the signal transmission device 120. In other embodiments, the processor 110 may send parameters related to the operational control of the interventional medical system 140 (e.g., a first optical image, a second optical image, target joint parameters, control parameters of an auxiliary procedure , etc.) to the terminal device 150 via the signal transmission device 120.

In some embodiments, the processor 110 is included in the interventional medical system 140. In some embodiments, the processor 110 may be configured to perform spatial registration on an interventional medical system 200 to obtain registration information. In some embodiments, the processor 110 may be configured to acquire relevant information and surgical information of a patient. In some embodiments, the processor 110 may be further configured to determine a procedure plan based on the registration information, the relevant information, and the surgical information. In some embodiments, the processor 110 may be further configured to control a surgical execution system to perform a procedure based on the procedure plan and the surgical information. In some embodiments, the processor 110 may be further configured to acquire a control command from an active control end in the interventional medical system 140 and control the surgical execution system to perform a procedure based on the control command.

In some embodiments, the processor 110 may include one or more processing engines (e.g., a single-chip processing engine or a multi-chip processing engine). For example, the processor 110 may include a Central Processing Unit (CPU). The processor 110 may process data, information, and/or processing results obtained from other devices or system components, and execute program instructions based on these data, information, and/or processing results to perform one or more functions described in this specification.

The signal transmission device 120 may connect various components of the application scenario 100 (e.g., the memory 130, the interventional medical system 140, the terminal device 150, etc.) and/or connect the application scenario 100 to external resources. The signal transmission device 120 enables communication between the components of the application scenario 100 and with other parts outside the application scenario 100, facilitating the exchange of data and/or information. In some embodiments, the memory 130 may be connected with the signal transmission device 120 to communicate with one or more components of the application scenario 100 (e.g., the processor 110, the interventional medical system 140, the terminal device 150). In some embodiments, the signal transmission device 120 may also include a network. In some embodiments, the network may include a Local Area Network (LAN), a Wide Area Network (WAN), a wired network, a wireless network, etc., or any combination thereof. In FIG. 1, the signal transmission device 120 includes a network, which is for illustrative purposes only and does not limit the embodiments of the present disclosure. It may be understood that the signal transmission device 120 may transmit signals through other media. For example, the signal transmission device 120 may include data transmission cables.

The memory 130 may be used to store data and/or instructions. In some embodiments, the memory 130 may store data and/or instructions used or executed by the processor 110 to perform the exemplary methods described in the present disclosure. For example, the memory 130 may store information generated by the interventional medical system 140 (e.g., a procedure plan).

In some embodiments, the memory 130 may be part of the processor 110. In some embodiments, the memory 130 may include mass storage, removable storage, volatile read-write memory, read-only memory (ROM), etc. In some embodiments, the memory 130 may be implemented on a cloud platform. In some embodiments, the memory 130 may be connected with the signal transmission device 120 to communicate with one or more components of the application scenario 100 (e.g., the processor 110, the interventional medical system 140, the terminal device 150).

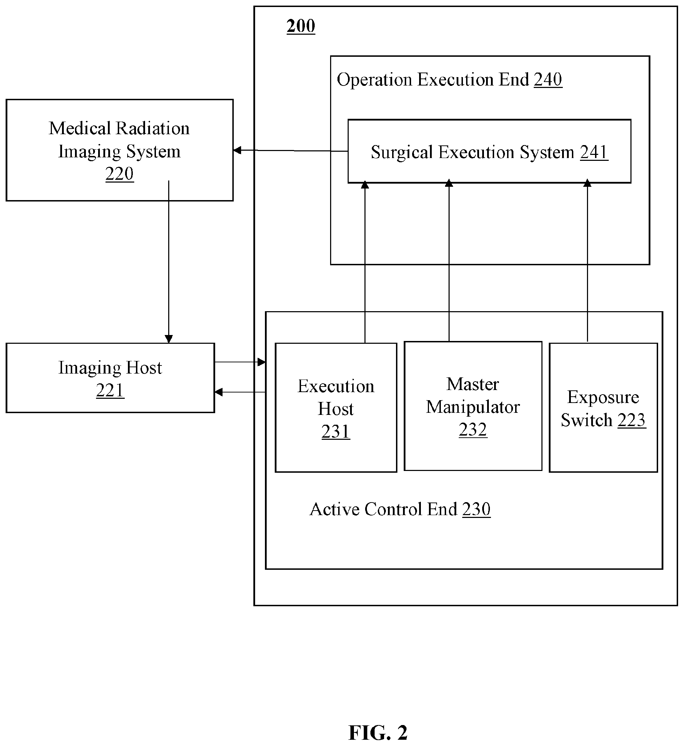

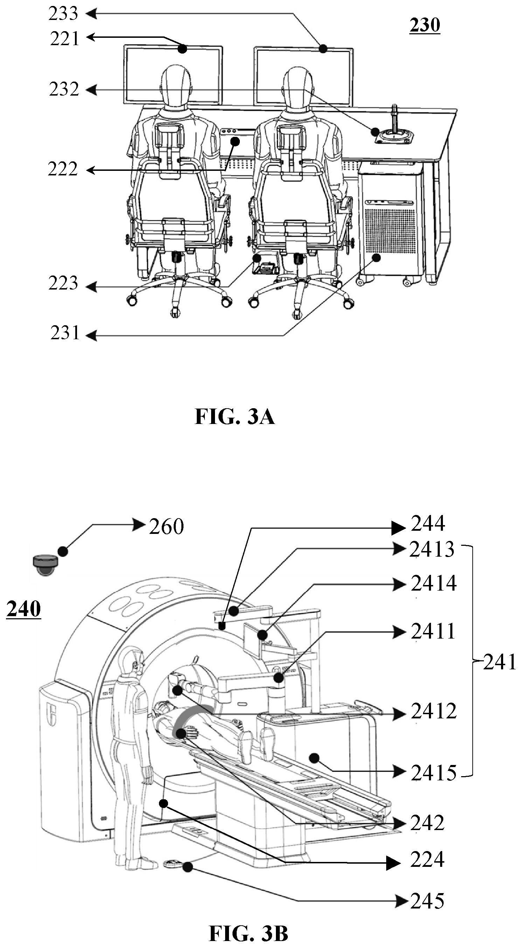

In some embodiments, the interventional medical system 140 may be used to perform one or more procedures on a patient for a surgery. In some embodiments, the interventional medical system 140 is configured to operate in conjunction with a medical radiation imaging system. In some embodiments, the interventional medical system 140 may include a surgical execution system. The interventional medical system 140 may acquire one or more medical images of the patient via the medical radiation imaging system, and may also control the surgical execution system to perform a corresponding procedure on the patient. In some embodiments, the processor 110 may be part of the interventional medical system 140. For more details on the interventional medical system 140, please refer to the subsequent descriptions of FIG. 2, FIG. 3A, FIG. 3B and related descriptions thereof, which will not be repeated here.

The terminal device 150 may include one or more terminal devices or software. In some embodiments, the terminal device 150 may include a mobile phone, a tablet computer, a laptop computer, a monitor, etc. In some embodiments, a user may view information and/or input data and/or instructions via the terminal device 150. In some embodiments, the terminal device 150 may include a signal transmitter and a signal receiver configured to communicate with the interventional medical system 140 to obtain information related to an observation object and imaging.

In some embodiments, the terminal device 150 may be fixed and/or mobile. For example, the terminal device 150 may be directly installed on the processor 110 and/or the interventional medical system 140, becoming part of the processor 110 and/or the interventional medical system 140. As another example, the terminal device 150 may be a movable device, and a user may carry the terminal device 150 at a location relatively distant from the processor 110, and the interventional medical system 140, and the terminal device 150 may be connected and/or communicate with the processor 110 and the interventional medical system 140 via the signal transmission device 120.

It should be noted that the above description of the application scenario of the interventional medical system is for convenience only and does not limit the present disclosure to the scope of the illustrated embodiments. It may be understood that for those skilled in the art, after understanding the principle of the system, they may arbitrarily combine the various components, or form sub-components to connect with other components, without departing from this principle. In some embodiments, the processor and memory disclosed in FIG. 1 may be different units in one component, or one component may perform the functions of two or more of the above components. For example, each component may share a single storage unit, or each may have its own. Such variations are within the scope of protection of the present disclosure.

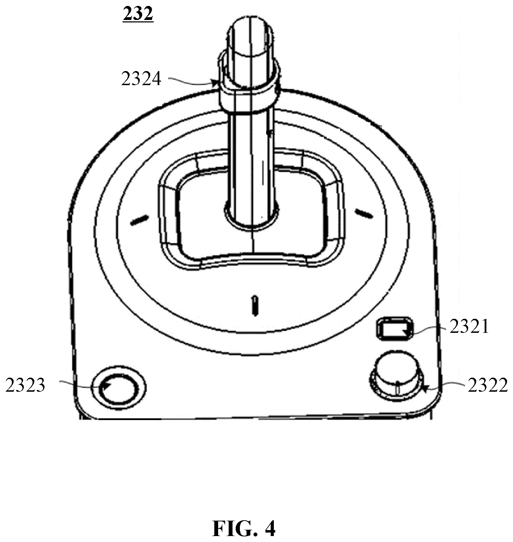

FIG. 2 is a block diagram illustrating an interventional medical system according to some embodiments of the present disclosure. FIG. 3A is a schematic diagram illustrating an active control end of an interventional medical system according to some embodiments of the present disclosure. FIG. 3B is a schematic diagram illustrating an operation end effector of an interventional medical system according to some embodiments of the present disclosure. FIG. 4 is a structural schematic diagram illustrating a master manipulator according to some embodiments of the present disclosure.

Referring to FIG. 2, FIG. 3A, and FIG. 3B, some embodiments of the present disclosure provide an interventional medical system 200. In some embodiments, the interventional medical system 200 may be configured to operate in conjunction with a medical radiation imaging system 220.

The interventional medical system 200 is used to perform one or more procedures on a patient. In some embodiments, the interventional medical system 200 may include an active control end 230 and an operation end effector 240 that are communicatively connected. A user (e.g., medical staff, etc.) may control the operation end effector 240 via the active control end 230 to perform a corresponding procedure for a surgery on the patient. In some embodiments, the active control end 230 and the operation end effector 240 may be connected via a control signal, and the type of the control signal may include, but is not limited to, an electrical signal, an optical signal, an acoustic signal, etc. In some embodiments, the interventional medical system 200 may further include a processor. The processor is configured to at least perform the following operations including performing spatial registration on the interventional medical system 200 to obtain registration information; acquiring relevant information and surgical information of a patient, determining a procedure plan based on the registration information, the relevant information, and the surgical information, and controlling a surgical execution system 241 to perform a procedure based on the procedure plan and the surgical information. In some embodiments, the processor is further configured to perform the following operations including acquiring a control command from the active control end 230, and controlling the surgical execution system 241 to perform a procedure based on the control command.

In some embodiments, the processor may include at least one of a first processor disposed in the active control end 230 and a second processor disposed in the operation end effector 240. The first processor and the second processor may cooperate to perform the above operations, or the first processor or the second processor may perform the above operations alone.

In some embodiments, the first processor and the second processor may cooperate to perform the following operations, including performing spatial registration on the interventional medical system 200 to obtain registration information, acquiring relevant information and surgical information of a patient, and determining a procedure plan based on the registration information, the relevant information, and the surgical information. For example, the first processor may acquire a first coordinate of a reference point (e.g., a reference point integrated into an end effector 2412 mentioned later, etc.) of the operation end effector 240 in a coordinate system corresponding to the medical radiation imaging system 220 (e.g., a second coordinate system O₂ mentioned later). The second processor acquires pose information of the operation end effector 240 (e.g., joint angles of a surgical execution arm 2411, etc.) to determine a second coordinate of the reference point in a coordinate system corresponding to the operation end effector 240 (e.g., a third coordinate system O3, etc.). The first processor sends the first coordinate to the second processor, and the second processor performs spatial registration based on the first coordinate and the second coordinate. The provision of the second processor eliminates the need for the first processor to directly obtain position information of the surgical execution system 241, reduces connecting cables between the active control end 230 and the operation end effector 240, allows for more flexible and varied positioning of the active control end 230, enables remote placement of the active control end 230, and allows users to operate remotely.

In some embodiments, the first processor may be further configured to acquire relevant information and surgical information of a patient, and determine a procedure plan based on the registration information, the relevant information, and the surgical information. In some embodiments, the second processor may be further configured to control the surgical execution system 241 to perform a procedure based on the procedure plan and the surgical information, and acquire a control command from the active control end 230 and control the surgical execution system 241 to perform a procedure based on the control command.

In some embodiments, the active control end 230 may be disposed at a location remote from the operation end effector 240 to avoid the influence of the operation end effector 240 or its working environment on the user. For example, the operation end effector 240 may be disposed in a radiology room, and the active control end 230 may be disposed outside the radiology room or in an adjacent room, thereby avoiding radiation exposure to the user during patient imaging by the medical radiation imaging system 220.

The active control end 230 is used to control the operation end effector 240. In some embodiments, the active control end 230 may be an integrated unit comprising the processor 110, the memory 130, and the terminal device 150. In some embodiments, the active control end 230 may include an execution host 231. The first processor may be disposed in the execution host 231, and the first processor may be configured to acquire relevant information and surgical information of a patient, and determine a procedure plan based on the registration information, the relevant information, and the surgical information. In some embodiments, the medical radiation imaging system 220 may be signally connected with an imaging host 221, and the active control end 230 may be signally connected with the imaging host 221. The second processor may be disposed in the imaging host 221, or in both the operation end effector 240 and the imaging host 221 simultaneously. The second processor may also be configured to acquire a control command from a master manipulator 232 and control the surgical execution system 241 to perform a surgical procedure based on the control command. For details regarding the imaging host 221, please refer to the subsequent description related to the imaging host 221.

In some embodiments, the active control end 230 may further include an execution display 233 (e.g., the terminal device 150). A user may view information and/or input data and/or instructions via the execution display 233 to facilitate control of the interventional medical system 200. For example, a user may input surgical information via the execution display 233. As another example, a user may view a first optical image via the execution display 233. As still another example, a user may input a control command via the execution display 233. In some embodiments, the execution display 233 may include an input device, such as a keyboard, a touch screen, etc.

In some embodiments, the active control end 230 may further include a master manipulator 232. The master manipulator 232 is signally connected with the surgical execution system 241 of the operation end effector 240. The master manipulator 232 is configured to receive a user operation to generate a control command. A user may generate a corresponding control command by operating the master manipulator 232, thereby controlling the surgical execution system 241 (e.g., the surgical execution arm 2411 and the end effector 2412, etc.) to perform a corresponding procedure.

Referring to FIG. 4, in some embodiments, the master manipulator 232 is provided with a release button 2321. The release button 2321 may be used to quickly release an execution instrument of a surgical operation assembly after a surgical procedure is completed.

In some embodiments, the active control end 230 may further include an exposure switch 223. The exposure switch 223 is configured to issue first enable information upon a user operation. The first enable information may be used to control exposure of the medical radiation imaging system 220, causing the medical radiation imaging system 220 to image the patient. For example, when a surgical mode is a real-time control mode, a user may depress the exposure switch 223, causing the exposure switch 223 to issue the first enable information, which causes the medical radiation imaging system 220 to scan the patient in real-time to acquire a real-time image of the patient. When the user releases the exposure switch 223, the exposure switch 223 stops issuing the first enable information, and the medical radiation imaging system 220 stops real-time scanning of the patient.

In some embodiments, the exposure switch 223 may be independently provided or integrated with the active control end 230. The provision of the exposure switch 223 allows the user to control the operating state of the medical radiation imaging system 220 at a suitable location, avoiding radiation exposure to the user from the medical radiation imaging system 220. At the same time, the provision of the exposure switch 223 also allows the user to conveniently and quickly control the activation and deactivation of the medical radiation imaging system 220, thereby preventing the medical radiation imaging system 220 from continuously exposing the patient and reducing unnecessary radiation exposure to the patient.

In some embodiments, the exposure switch 223 may be an exposure foot pedal. In some embodiments, when the surgical mode is the real-time control mode, the processor may be further configured to acquire the first enable information from the exposure switch 223, and based on the real-time control mode and the first enable information, scan the patient in real-time via the medical radiation imaging system 220 to acquire a real-time image of the patient.

In some embodiments, the master manipulator 232 is further provided with a control enable button 2324. The control enable button 2324 is configured to issue second enable information in response to a user operation. For example, when the user presses the control enable button 2324, the control enable button 2324 issues the second enable information; when the user releases the control enable button 2324, the control enable button 2324 stops issuing the second enable information. In some embodiments, the control enable button 2324 is configured to permit or prohibit the master manipulator 232 from controlling the surgical execution system 241 to perform a procedure. That is, the second enable information can activate and enable a control command of the master manipulator 232. In some embodiments, the control enable button 2324 may control whether the surgical execution system 241 is locked or not, i.e., the second enable information output by the control enable button 2324 can unlock the surgical execution system 241, allowing the surgical execution system 241 to move. In some embodiments, the control enable button 2324 may control whether the master manipulator 232 is locked or not, i.e., the second enable information output by the control enable button 2324 can unlock the master manipulator 232, allowing the master manipulator 232 to be operated. For example, in the real-time control mode, the second enable information allows a control command to take effect. In other words, in the real-time control mode, only when the user operates the control enable button 2324, the master manipulator 232 can control the surgical execution system 241 via the control command.

In some embodiments, the processor is further configured to, in response to both the first enable information and the second enable information being acquired, acquire a control command from the active control end 230. In other words, only when the processor acquires both the first enable information and the second enable information, the control command from the master manipulator 232 can take effect.

In some embodiments, the first enable information enables the activation of the second enable information. That is, after the user operates the exposure switch 223 to issue the first enable information, the first enable information causes the second enable information to take effect, thereby activating and enabling the control command of the master manipulator 232, allowing the master manipulator 232 to control the surgical execution system 241 to perform a procedure. This prevents the user from operating the master manipulator 232 without a view of the patient (e.g., medical images of the patient), thereby avoiding additional harm to the patient.

In some embodiments, due to a delay between the triggering of the first enable information and the output of the medical image of the patient by the medical radiation imaging system 220 scanning the patient, if the first enable information directly caused the second enable information to take effect, the user might operate the master manipulator 232 during the period after the first enable information is triggered but before the medical radiation imaging system 220 outputs the medical image of the patient, leading to blind puncturing and potentially causing additional harm to the patient. In some embodiments, the medical image of the patient output by the medical radiation imaging system 220 may serve as third enable information. The second enable information is activated and takes effect only when the first enable information and the third enable information act together. That is, after the user operates the exposure switch 223 to issue the first enable information, the medical radiation imaging system 220 scans the patient. After the medical radiation imaging system 220 outputs the medical image of the patient (i.e., the third enable information), the control command of the master manipulator 232 is activated and takes effect. In some embodiments, when the second enable information is not output, the first enable information may only control the exposure of the medical radiation imaging system 220, causing it to scan the patient and output the medical image. That is, the user may operate the exposure switch 223 alone to view the medical image of the patient.

In some embodiments, the first enable information indirectly activates and enables the control command of the master manipulator 232. Specifically, the user operates the exposure switch 223 to generate the first enable information. The first enable information enables the activation of the second enable information generated by the control enable button 2324. The second enable information can then activate and enable the control command generated by the master manipulator 232. That is, only when the user operates both the control enable button 2324 and the exposure switch 223 simultaneously, the exposure switch 223 generates the first enable information, which activates the second enable information generated by the control enable button 2324, which in turn activates and enables the control command generated by the master manipulator 232. At this point, the master manipulator 232 is permitted to control the surgical execution system 241 to perform a procedure. When the user does not operate the exposure switch 223 and/or the control enable button 2324, the master manipulator 232 is prohibited from controlling the surgical execution system 241 to perform a procedure.

In other embodiments, when the surgical mode is the real-time control mode, the second enable information may also enable the first enable information. In other words, in the real-time control mode, only when the user operates the control enable button 2324, the processor can, based on the first enable information, scan the patient in real-time via the medical radiation imaging system 220 to acquire a real-time image of the patient. In some embodiments, in the real-time control mode, when the user presses the control enable button 2324 to issue the second enable information, it indicates that the surgical procedure is still in progress. To improve operational precision, the second enable information enables the first enable information, thereby allowing the medical radiation imaging system 220 to scan and image the patient during the surgical procedure. When the user releases the control enable button 2324 and the control enable button 2324 stops issuing the second enable information, it indicates that the surgical procedure has stopped (e.g., completed or paused). To reduce radiation exposure to the patient, scanning and imaging via the medical radiation imaging system 220 should be avoided once the surgical procedure is stopped and the first enable information is deactivated.

In some embodiments, when the surgical mode is a breakpoint control mode, the end effector 2412 may include a clamping structure. The clamping structure clamps an execution instrument. When the surgical mode is the breakpoint control mode, the second enable signal issued by the control enable button 2324 may be used to control the clamping state of the clamping structure to the execution instrument. For more details on the clamping structure and the execution instrument, please refer to the subsequent description. In some embodiments, when the surgical mode is the breakpoint control mode, the processor is further configured to acquire the second enable information from the control enable button 2324 and control the clamping structure to adjust to a clamped state based on the second enable information. The second enable information enables a control command to take effect.

In some embodiments, in the breakpoint control mode, the processor is further configured to acquire a scan signal, scan the patient via the medical radiation imaging system 220 to obtain a surgical image; and based on the breakpoint control mode and the surgical image, control an execution instrument 24122 via the master manipulator 232 to perform a portion of the surgical procedure corresponding to the surgical image. The scan signal is a signal manually input by the user. For example, the user may input the scan signal via an operation interface, or via a control button on the master manipulator 232, or directly on the imaging host 221. In the breakpoint control mode, the processor may, based on the scan signal, control the medical radiation imaging system 220 via the imaging host 221 to scan the patient according to preset parameters to obtain a surgical image. The preset parameters may include scan time, scan area, etc. The preset parameters may be pre-input or historical. In some embodiments, in the breakpoint control mode, when the user presses the control enable button 2324 to issue the second enable information, it indicates that the surgical procedure is still in progress. To reduce radiation exposure to the patient, scanning and imaging the patient via the medical radiation imaging system 220 during the surgical procedure should be avoided, and the user should not input the scan signal at this time. When the user releases the control enable button 2324, it indicates that the current portion of the surgical procedure is paused or completed. The control enable button 2324 stops issuing the second enable information. At this time, the user may input the scan signal to scan the patient via the medical radiation imaging system 220 and generate a corresponding surgical image. Thus, in the breakpoint control mode, scanning and imaging across different portions of the surgical procedure are facilitated, allowing corresponding portions of the surgical procedure to be performed based on the obtained surgical images.

In some embodiments, the active control end 230 may further include a selection unit (not shown). The selection unit may be used to adjust the working mode of the master manipulator 232. When the master manipulator 232 is in different working modes, it can control the surgical execution system 241 to perform corresponding procedures. In some embodiments, the working modes of the master manipulator 232 may include an execution mode and a pose adjustment mode. When the master manipulator 232 is in the execution mode, the master manipulator 232 can control the end effector 2412 and the execution instrument to move for performing corresponding surgical procedures. For example, when the execution instrument of the end effector 2412 includes a puncture needle, the master manipulator 232 can control the puncture needle to move linearly along its extension direction to puncture the patient. When the master manipulator 232 is in the pose adjustment mode, it can control the surgical execution arm 2411 to move to adjust the pose of the surgical execution arm 2411 and the end effector 2412. In some embodiments, to improve operational precision, the master manipulator 232 cannot operate simultaneously in the execution mode and the pose adjustment mode, i.e., it cannot simultaneously control the movement of the surgical execution arm 2411 and the end effector 2412. In the execution mode, the master manipulator 232 can only control the movement of the end effector 2412, while the surgical execution arm 2411 is in a locked state to maintain the pose of the surgical execution arm 2411 and the end effector 2412, reducing operational error of the end effector 2412. In the pose adjustment mode, the master manipulator 232 can only control the movement of the surgical execution arm 2411, while the end effector 2412 is in a locked state.

In some embodiments, the pose adjustment mode may include a first pose adjustment mode and a second pose adjustment mode. When the master manipulator 232 is in the first pose adjustment mode, the master manipulator 232 can control the surgical execution arm 2411 to move within a transverse plane (i.e., horizontal plane) of the patient to adjust its pose. The first pose adjustment mode may also be referred to as an intra-slice pose adjustment mode. When the master manipulator 232 is in the second pose adjustment mode, the master manipulator 232 can control the surgical execution arm 2411 to move within a sagittal plane of the patient to adjust its pose. The second pose adjustment mode may also be referred to as an inter-slice pose adjustment mode. In some embodiments, when the selection unit selects the pose adjustment mode, it can select only one of the first pose adjustment mode or the second pose adjustment mode exclusively. In some embodiments, when the selection unit selects the pose adjustment mode, it may also select both the first pose adjustment mode and the second pose adjustment mode simultaneously.

In some embodiments, to reduce operational complexity, the working modes of the master manipulator 232 may further include a free mode. When the master manipulator 232 is in the free mode, the master manipulator 232 can control the movement of the surgical execution arm 2411 in a first direction and within a first plane while simultaneously controlling the movement of the end effector 2412.

In some embodiments, the selection unit may further include a speed control module (not shown). The speed control module may be used to control the movement speed of the surgical execution system 241 when the master manipulator 232 is in different working modes. For example, in the free mode, the movement speed of the surgical execution system 241 may be higher, allowing the user to quickly and freely control the movement and operation of the surgical execution system 241. In the pose adjustment mode, the movement speed of the surgical execution arm 2411 may be relatively slower, allowing the user to precisely adjust the position and posture of the surgical execution arm 2411. In the execution mode, the movement speed of the end effector 2412 may be relatively slower, allowing the user to precisely control the end effector 2412 to perform the corresponding surgical procedure. In some embodiments, in different working modes of the master manipulator 232, the corresponding movement speed of the surgical execution system 241 may be set manually or preset.

In some embodiments, the selection unit may include a selection button 2322 provided on the master manipulator 232, allowing the user to switch the working mode of the master manipulator 232 while operating the master manipulator 232. In some embodiments, the selection unit may also include an operation interface displayed on the execution display 233, allowing the user to select the working mode of the master manipulator 232 intuitively and conveniently. In some embodiments, the operation interface is configured to perform at least one of the following: displaying and processing relevant information and surgical information for a patient; displaying and entering a procedure plan; and displaying prompt information. In some embodiments, the operation interface may display the working mode of the master manipulator 232 and the pose of the surgical execution system 241 to improve the precision of the user's control and adjustment of the surgical execution system 241 via the master manipulator 232. In some embodiments, the operation interface may also display the speed selection for the surgical execution system 241, as set by the speed control module, to facilitate the user's control of the surgical execution system 241 via the master manipulator 232. In some embodiments, the operation interface may also be integrated into the execution host 231.

In some embodiments, the master manipulator 232 may be further provided with a quick-switch button 2323. The quick-switch button 2323 may be used to quickly switch the master manipulator 232 from the pose adjustment mode to the execution mode, quickly lock the pose of the surgical execution system 241, and control the end effector 2412 to perform a procedure, thereby improving efficiency and reducing surgical time.

In some embodiments, the operation end effector 240 may include a surgical execution system 241. The surgical execution system 241 may be used to perform a corresponding surgical procedure on a patient. In some embodiments, the surgical execution system 241 may include a surgical operation assembly and an auxiliary procedure assembly.

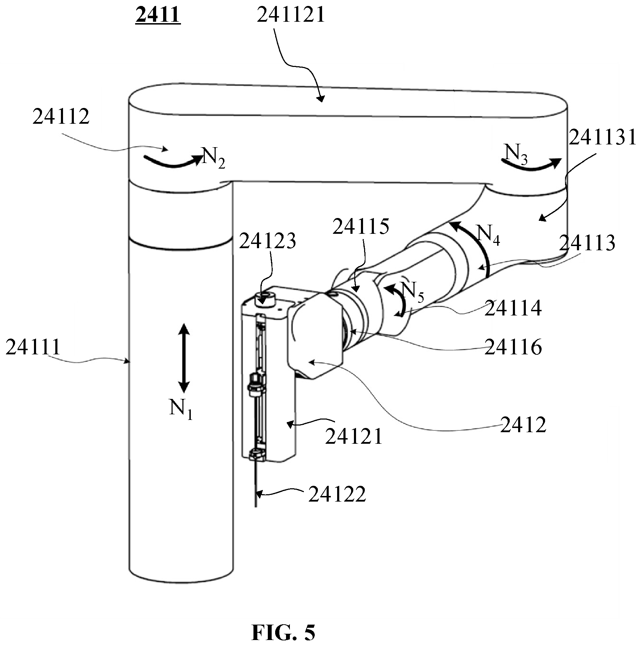

FIG. 5 is a structural schematic diagram illustrating a surgical execution arm according to some embodiments of the present disclosure.

Referring to FIG. 2 to FIG. 5, in some embodiments, the surgical operation assembly may include a surgical execution arm 2411, an end effector 2412, and an execution instrument 24122. The end effector 2412 may be disposed at a distal end of the surgical execution arm 2411, and the execution instrument 24122 is disposed at the end effector 2412. The surgical execution arm 2411 and the end effector 2412 cooperate to control the execution instrument 24122 to perform a corresponding surgical procedure. The surgical operation assembly is configured to at least receive and execute an input control command. In some embodiments, the control command may be issued by the active control end 230 (e.g., the master manipulator 232, the execution display 233, etc.). In some embodiments, when the surgical execution system 241 performs different surgical procedures, corresponding types of end effectors 2412, and execution instruments 24122 may be selected.

In some embodiments, the surgical execution arm 2411 may include a first articulated arm 24111, a second articulated arm 24112, a third articulated arm 24113, a fourth articulated arm 24114, and a fifth articulated arm 24115. One end of the first articulated arm 24111 is connected with a base of the surgical execution arm 2411 (e.g., the base 2415), and the other end of the first articulated arm 24111 is connected with one end of the second articulated arm 24112. The second articulated arm 24112 includes a first extension segment 241121 perpendicular to the axial direction of the first articulated arm 24111. The other end of the second articulated arm 24112 is connected with one end of the third articulated arm 24113. The two ends of the second articulated arm 24112 are respectively disposed at the two axial ends of the first extension segment 241121. The other end of the third articulated arm 24113 is connected with one end of the fourth articulated arm 24114. The third articulated arm 24113 includes a second extension segment 241131 perpendicular to the axial direction of the first extension segment 241121. The other end of the second articulated arm 24112 may be connected with the second extension segment 241131. The fourth articulated arm 24114 is perpendicular to the axial direction of the second extension segment 241131. The other end of the fourth articulated arm 24114 is connected with one end of the fifth articulated arm 24115, and the other end of the fifth articulated arm 24115 is connected with the end effector 2412. In some embodiments, the first articulated arm 24111 is provided with a first joint axis N₁, which has a translational degree of freedom along the axial direction of the first articulated arm 24111. A second joint axis N₂ is provided between the first articulated arm 24111 and the second articulated arm 24112, and has a rotational degree of freedom around the axial direction of the first articulated arm 24111. A third joint axis N₃ is provided between the second articulated arm 24112 and the third articulated arm 24113, and has a rotational degree of freedom around the axial direction of the second extension segment 241131. A fourth joint axis N₄ is provided between the third articulated arm 24113 and the fourth articulated arm 24114, and has a rotational degree of freedom around the axial direction of the fourth articulated arm 24114. A fifth joint axis N₅ is provided between the fourth articulated arm 24114 and the fifth articulated arm 24115, and has a rotational degree of freedom relative to the distal end of the fourth articulated arm 24114. By adjusting the first joint axis N₁, the second joint axis N₂, the third joint axis N₃, the fourth joint axis N₄, and the fifth joint axis N₅, the position and posture of the surgical execution arm 2411 may be adjusted. In some embodiments, each of the first joint axis N₁, the second joint axis N₂, the third joint axis N₃, the fourth joint axis N₄, and the fifth joint axis N₅ is provided with a servo controller (not shown), a dual-channel position encoder (not shown), a motor (not shown), and a brake (not shown). The servo controller may be used to control the movement of the joint, thereby controlling joint parameters (e.g., translation distance, rotation angle, etc.) of the corresponding joint. The dual-channel position encoder may be used to read the joint parameters of the corresponding joint. The motor may be used to provide power for the movement of the corresponding joint. The brake may lock the corresponding joint and is used to stop the movement of the corresponding joint in an emergency.

In some embodiments, the end effector 2412 may include an execution mount 24121. The execution mount 24121 is used to install the execution instrument 24122. The execution instrument 24122 may be an external component or an integral part of the end effector 2412. The execution mount 24121 is connected with the distal end of the surgical execution arm 2411 (i.e., the end of the fifth articulated arm 24115 away from the fourth articulated arm 24114), and the execution instrument 24122 is installed on the execution mount 24121. The execution mount 24121 may be used to determine the position of the execution instrument 24122.

In some embodiments, the execution mount 24121 may be detachably connected (e.g., via snap-fit, etc.) with the surgical execution arm 2411, allowing for the selection of different end effectors 2412 according to different surgical procedures. In some embodiments, the execution mount 24121 is connected with the surgical execution arm 2411 before spatial registration to establish a fourth coordinate system O₄ of the end effector 2412 during spatial registration. In some embodiments, the execution mount 24121 may be drivingly connected with the surgical execution arm 2411 (e.g., via a universal joint, etc.). The execution mount 24121 may move relative to the fifth articulated arm 24115 at the distal end of the surgical execution arm 2411, meaning the execution mount 24121 can perform pose adjustment independently, allowing the end effector 2412 to fine-tune its pose. It should be noted that during the pose adjustment of the end effector 2412, the surgical execution arm 2411 may remain stationary, and the position of the connection portion between the end effector 2412 and the surgical execution arm 2411 remains unchanged.

In some embodiments, the execution instrument 24122 is detachably fixed to the execution mount 24121. The detachable configuration of the execution instrument 24122 allows the end effector 2412 to select a corresponding type of execution instrument 24122 when the surgical execution system 241 performs different surgical procedures. At the same time, the detachable configuration of the execution instrument 24122 allows the end effector 2412 to install the execution instrument 24122 before the surgical procedure and remove the execution instrument 24122 after the surgical procedure is completed, preventing the execution instrument 24122 from causing harm to the user, patient, etc. In some embodiments, the execution instrument 24122 may include, but is not limited to, a puncture needle, an ablation needle, etc.

In some embodiments, the execution mount 24121 may be provided with a drive mechanism. The drive mechanism may be used to drive the execution instrument 24122 to perform a surgical procedure. The type of drive mechanism may match the execution instrument 24122. For example, when the execution instrument 24122 is a puncture-type instrument (e.g., a puncture needle), the drive mechanism may be a puncture mechanism (e.g., a linear motion mechanism, etc.).

In some embodiments, the end effector 2412 may include a clamping structure (not shown). The clamping structure may be disposed on the execution mount 24121, and the execution instrument 24122 may be installed on the execution mount 24121 via the clamping structure. The clamping structure may clamp the execution instrument 24122. In some embodiments, when the user presses the control enable button 2324, the clamping structure clamps and fixes the execution instrument; when the user releases the control enable button 2324, the clamping structure adjusts to a semi-released state, allowing the execution instrument 24122 a degree of freedom to move relative to the clamping structure while preventing the execution instrument 24122 from detaching from the clamping structure. For example, the clamping structure may include two arc-shaped clamping jaws. One end of each of the two clamping jaws is hinged together, and the other ends of the two clamping jaws serve as free ends that can open and close. When the clamping structure is in the clamped state, the size of the region defined between the two clamping jaws corresponds to the size of the execution instrument 24122 (i.e., the two clamping jaws directly contact and clamp the execution instrument 24122), thereby fixing the execution instrument 24122. When the clamping structure is in the semi-released state, the size of the region defined between the two clamping jaws is larger than the size of the execution instrument 24122, and the opening size between the free ends of the two clamping jaws is smaller than the size of the execution instrument 24122, allowing the execution instrument 24122 to move within the region defined between the two clamping jaws while preventing the execution instrument 24122 from detaching from the clamping structure through the gap between the free ends of the two clamping jaws. In some embodiments, the clamping structure further includes two limit blocks disposed along the axial direction of the execution instrument 24122. The two limit blocks are respectively disposed on opposite sides of the plane containing the two clamping jaws along the axial direction of the execution instrument 24122, thereby preventing the execution instrument 24122 from detaching from the clamping structure along its axial direction when the clamping structure is in the semi-released state.

In some embodiments, the execution mount 24121 is further provided with an instrument confirmation control (not shown). In some embodiments, the instrument confirmation control is configured to output a confirmation signal in response to completion of installation of the execution instrument 24122. The confirmation signal is used to confirm that the execution instrument 24122 on the execution mount 24121 has been installed, facilitating subsequent operations. In some embodiments, the processor is configured to, in response to completion of installation of the execution instrument 24122, control the instrument confirmation control to output the confirmation signal; and based on the confirmation signal, control the surgical execution arm 2411 to adjust to an operation pose.

In some embodiments, the execution mount 24121 may be provided with a manual control 24123. The manual control 24123 may be used to control the clamping structure to clamp or release the execution instrument 24122. That is, the manual control 24123 can cause the clamping structure to maintain clamping and fixation of the execution instrument, keeping the execution instrument 24122 in a clamped state to complete the installation of the execution instrument 24122; or cause the clamping structure to release the fixation of the execution instrument 24122, allowing the execution instrument 24122 to be detached or removed from the end effector 2412. The provision of the manual control 24123 allows the execution instrument 24122 to be released or removed from the end effector 2412 in emergency situations (e.g., power outage, loss of power), thereby enhancing safety. In some embodiments, the specific form of the manual control 24123 may include, but is not limited to, a button, a key, a knob, etc.

In some embodiments, the auxiliary procedure assembly includes an auxiliary indicator 244. The auxiliary indicator 244 is configured to indicate an auxiliary procedure. In some embodiments, the auxiliary indicator 244 may include a linear laser that indicates the auxiliary procedure via laser light. In some embodiments, the auxiliary indicator 244 may also include a projector to indicate the auxiliary procedure via projection. In some embodiments, the auxiliary indicator 244 may also include a display screen that indicates the auxiliary procedure by displaying a real-time image of the area containing the target location and marking the target location on the image. In some embodiments, the auxiliary indicator 244 may indicate an entry point for an auxiliary procedure. In some embodiments, the auxiliary indicator 244 may also indicate an entry path for an auxiliary procedure. For example, when the auxiliary indicator 244 is a linear laser, the laser illumination path can indicate the entry path. In other embodiments, when a surgical mode is a manual control mode, the auxiliary indicator 244 may indicate a surgical procedure, and indicate an entry point and/or an entry path for the surgical procedure, and the user can perform the surgical procedure according to the indication of the auxiliary indicator 244.

In some embodiments, the auxiliary indicator 244 may be disposed on the surgical execution arm 2411 or the end effector 2412. By adjusting the pose of the surgical execution arm 2411, the indication area of the auxiliary indicator 244 may be directly adjusted without the need for separate adjustment of the auxiliary indicator 244. Moreover, the surgical execution arm 2411 has a large range of motion, allowing the auxiliary indicator 244 to have a large indicate-able area.

In some embodiments, the auxiliary procedure assembly further includes a navigation support arm 2413. The auxiliary indicator 244 may also be disposed on the navigation support arm 2413. In some embodiments, the navigation support arm 2413 may be fixed to a base (e.g., the base 2415) of the surgical execution system 241. In other embodiments, the navigation support arm 2413 may be fixed to an external support platform or the medical radiation imaging system 220. The navigation support arm 2413 may support and install the auxiliary indicator 244.

In some embodiments, the navigation support arm 2413 has a stowed state and a deployed state. When the navigation support arm 2413 is in the stowed state, the auxiliary indicator 244 is not operational. When the navigation support arm 2413 is in the deployed state, the auxiliary indicator 244 can adjust its pose to indicate a procedure. That is, after the navigation support arm 2413 is deployed and fixed, the auxiliary indicator 244 can slide and rotate within a preset range (e.g., the navigation support arm 2413 is provided with a slider that can slide relative to the navigation support arm 2413, and the auxiliary indicator 244 may be hinged to the slider). In some embodiments, to prevent the navigation support arm 2413 from interfering with the auxiliary indicator 244, the auxiliary indicator 244 may be disposed on the bottom side of the distal end of the navigation support arm 2413 (as shown in FIG. 3B). In other embodiments, the auxiliary indicator 244 may also be disposed at other positions on the navigation support arm 2413 and may be connected by other means, and this specification does not impose excessive restrictions on this.

In some embodiments, the surgical execution system 241 may further include a touch display 2414. The touch display 2414 may be used to display a procedure plan (e.g., operation pose, etc.) of the surgical execution system 241, facilitating user control of the surgical execution system 241 and improving operational precision.

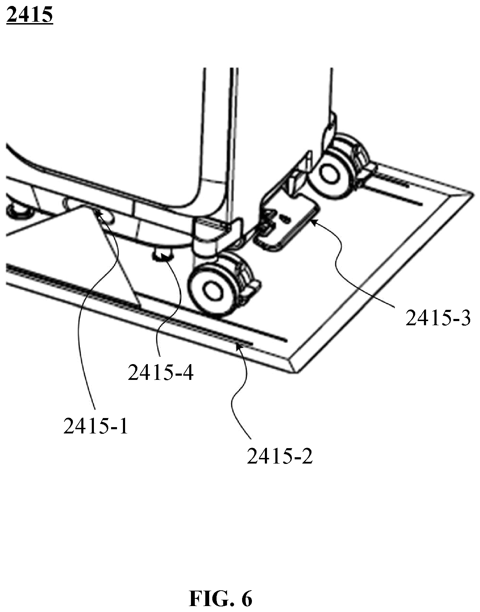

FIG. 6 is a partial structural schematic diagram illustrating a base according to some embodiments of the present disclosure.

Referring to FIG. 3B and FIG. 6, in some embodiments, the surgical execution system 241 may further include a base 2415. The surgical operation assembly and the auxiliary procedure assembly may be disposed on the base 2415, which can provide a platform for mounting and fixing them. In some embodiments, the base 2415 may be immovably fixed at a preset position (e.g., near the medical radiation imaging system 220) to facilitate the surgical procedure. In some embodiments, the base 2415 may be movable, capable of being moved to a target position (e.g., near the medical radiation imaging system 220) when needed for the surgical procedure, and moved away from the target position after use to avoid obstruction. For example, the base 2415 may be a fixed platform, a surgical cart, etc.

In some embodiments, the base 2415 may include a positioning indicator 2415-1, and the preset position may include a positioning marker 2415-2. The cooperation of the positioning indicator 2415-1 and the positioning marker 2415-2 indicates that the base 2415 is at the preset position. For example, the positioning indicator 2415-1 may include laser lights disposed on at least two adjacent sides around the perimeter of the base 2415, and the positioning marker 2415-2 may include floor markings corresponding to the laser lights. When the laser lights on at least two adjacent sides of the base 2415 project light bars onto the floor that coincide with the corresponding floor markings, it indicates that the base 2415 is at the preset position.

In some embodiments, the base 2415 may further include a locking mechanism 2415-3. The locking mechanism 2415-3 may be used to lock the position of the base 2415. Specifically, the base 2415 may include the locking mechanism 2415-3 and a support member 2415-4. When the locking mechanism 2415-3 is activated, the support member 2415-4 can support the base 2415, lifting the base 2415off the ground. For example, the locking mechanism 2415-3 may include a floor-brake foot pedal, and the support member 2415-4 may include floor-brake support legs. When the base 2415 is moved to a preset area, the user can depress the floor-brake foot pedal, causing the floor-brake support legs to lower or extend, supporting the base 2415 to lift the base (e.g., its casters) off the ground, thereby locking the position of the base 2415 at the preset position.

It should be noted that when the base 2415 is fixedly installed and cannot move, it may initially be placed at the preset position, and the positioning indicator 2415-1, positioning marker 2415-2, locking mechanism 2415-3, and support member 2415-4 may be omitted.

In some embodiments, the operation end effector 240 may further include a scanning table 242 (as shown in FIG. 3B). The scanning table 242 provides a lying space for a patient, allowing the patient to adjust their pose and facilitating subsequent surgical procedures. In some embodiments, the scanning table 242 may cooperate with the medical radiation imaging system 220. The scanning table 242 may enter or exit the medical radiation imaging system 220 via a movable support. When the scanning table 242 is moved out of the medical radiation imaging system 220, the patient can get on or off the scanning table 242. When the scanning table 242 is moved into the medical radiation imaging system 220, the medical radiation imaging system 220 can image the patient on the scanning table 242, forming a medical image of the patient. In some embodiments, the scanning table 242 may also be a structural module of the medical radiation imaging system 220.

In some embodiments, the operation end effector 240 may further include an input device. The input device may be used to input at least one of instructions and information to the surgical execution system 241. In some embodiments, the touch display 2414 may serve as the input device, allowing the user to input at least one of instructions and information to the surgical execution system 241 via the touch display 2414. In some embodiments, the execution display 233 may also serve as the input device.

In some embodiments, the surgical operation assembly may further include a motion enable switch 245. The motion enable switch 245 is configured to permit or prohibit movement of the surgical execution arm 2411. In some embodiments, when the user operates the motion enable switch 245, the surgical execution arm 2411 is permitted to move; when the user does not operate the motion enable switch 245, the surgical execution arm 2411 is prohibited from moving to prevent unintended displacement.

In some embodiments, the medical radiation imaging system 220 is used to scan and image a patient, forming one or more medical images of the patient. In some embodiments, the medical radiation imaging system 220 may include, but is not limited to, a CT imaging system, an MR (Magnetic Resonance) imaging system, an ultrasound imaging system, a DSA (Digital Subtraction Angiography) imaging system, etc. In some embodiments, the medical radiation imaging system 220 may be an external device or an internal component module of the interventional medical system 200.

In some embodiments, the medical radiation imaging system 220 is signally connected with an imaging host 221. The imaging host 221 is configured to control exposure of the medical radiation imaging system 220, acquire one or more medical images of the patient from the medical radiation imaging system 220, and transmit the one or more medical images to the execution host 231. In some embodiments, during a surgical procedure, the medical radiation imaging system 220 may scan the patient in real time, and the execution display 233 may display the real-time medical images of the patient. The user can perform a surgical procedure (e.g., puncture, etc.) based on the displayed real-time medical images, improving the precision of the surgical procedure and reducing the difficulty of the surgical procedure. In some embodiments, after the imaging host 221 transmits the one or more medical images of the patient to the execution host 231, the execution host 231 may archive and store the one or more medical images for subsequent retrieval. In some embodiments, the medical radiation imaging system 220 may further be provided with an image controller 222. The image controller 222 may adjust the generated one or more medical images of the patient. In some embodiments, the imaging host 221 and/or the image controller 222 may be provided independently of the medical radiation imaging system 220 or integrated with the active control end 230.

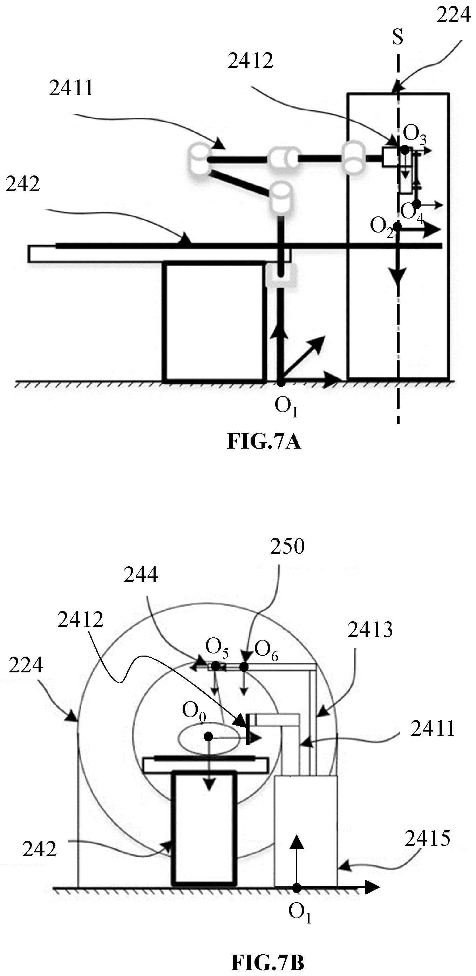

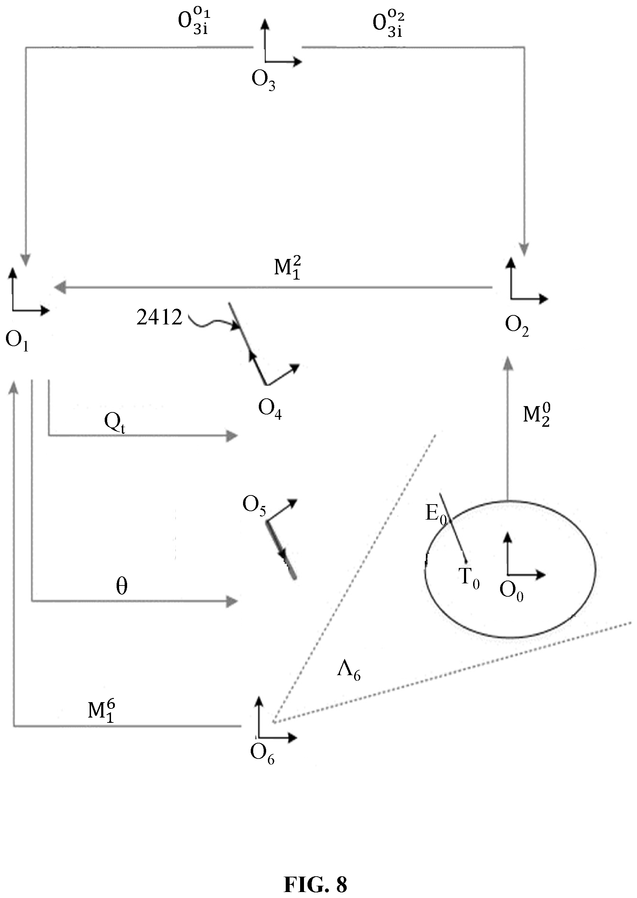

FIG. 7A is a schematic diagram illustrating reference coordinate systems of an operation end effector according to some embodiments of the present disclosure. FIG. 7B is another schematic diagram illustrating reference coordinate systems of an operation end effector according to some embodiments of the present disclosure. FIG. 8 is a schematic diagram illustrating transformations between reference coordinate systems according to some embodiments of the present disclosure. The reference coordinate systems of the operation end effector 240 are described below with reference to FIG. 7A, FIG. 7B, and FIG. 8.

As shown in FIG. 7A, FIG. 7B, and FIG. 8, in some embodiments, when a patient lies on the scanning table 242, a patient coordinate system O₀ is established based on the patient's position (e.g., the surgical area of the patient).

In some embodiments, a first coordinate system O₁ of the surgical execution arm 2411 may be established based on the position of the surgical execution arm 2411. In some embodiments, during a surgical procedure performed by the surgical execution system 241, the position of the connection portion between the surgical execution arm 2411 and the base 2415 remains relatively unchanged. The reference position for the first coordinate system O₁ may be the projection on the ground of the position of the connection portion between the surgical execution arm 2411 and the base 2415, as shown in FIG. 7A and FIG. 7B. In some embodiments, the reference position for the first coordinate system O₁ may be other positions relative to the base 2415, as long as the relative position between the surgical execution arm 2411 and the base 2415 may be clearly defined, such as the position of the connection portion between the surgical execution arm 2411 and the base 2415.

In some embodiments, a second coordinate system O₂ of the medical radiation imaging system 220 is established based on the position of the medical radiation imaging system 220. In some embodiments, the medical radiation imaging system 220 may include a gantry 224, and an imaging scanning plane S of the medical radiation imaging system 220 is located within the gantry 224. In some embodiments, the reference position for the second coordinate system O₂ may be located on the imaging scanning plane S. In some embodiments, the medical radiation imaging system 220 may also include the scanning table 242, and the second coordinate system O₂ may also be established based on the position of the scanning table 242.

In some embodiments, a third coordinate system O3 of a reference point may be established based on the reference point located near the distal end of the surgical execution arm 2411. The third coordinate system O3, in conjunction with the first coordinate system O₁, can accurately represent the position and posture of the surgical execution arm 2411. In some embodiments, the end effector 2412 is integrally provided with the reference point (e.g., a marker ball, etc.).

In some embodiments, the reference point may be disposed on the surgical execution arm 2411, such as at the distal end of the surgical execution arm 2411 (i.e., the connection position between the surgical execution arm 2411 and the end effector 2412). In some embodiments, the reference point may also be disposed on the execution mount 24121 of the end effector 2412. Correspondingly, the processor may be configured to perform spatial registration on the interventional medical system 200 after the execution mount 24121 is installed onto the surgical execution arm 2411. In some embodiments, the reference point may also be disposed on the execution instrument 24122. Correspondingly, the processor may be configured to perform spatial registration of the interventional medical system 200 after the execution mount 24121 is installed on the surgical execution arm 2411 and the execution instrument 24122 is installed on the execution mount 24121. In some embodiments, the reference point may also be disposed on a registration tool. The registration tool is used for registration during spatial registration of the interventional medical system 200. The registration tool is detachably connected with the execution mount 24121. The registration tool is installed onto the execution mount 24121 before spatial registration and is detached from the execution mount 24121 after spatial registration is completed. Correspondingly, the processor may be further configured to perform spatial registration of the interventional medical system 200 after the execution mount 24121 is installed on the surgical execution arm 2411 and the registration tool is installed on the execution mount 24121.

In some embodiments, the end of the end effector 2412 away from the surgical execution arm 2411 is the portion that performs the surgical procedure. Using the end of the end effector 2412 away from the surgical execution arm 2411as a reference, a fourth coordinate system O₄ of the end effector 2412 may be established. The fourth coordinate system O₄, in conjunction with the third coordinate system O3, can accurately represent the position and posture of the end effector 2412.

In some embodiments, a fifth coordinate system O₅ of the auxiliary procedure assembly may be established based on the position of the auxiliary indicator 244. It should be noted that when the auxiliary indicator 244 is disposed on the surgical execution arm 2411 or the end effector 2412, the fifth coordinate system O₅ may be established directly based on the position of the auxiliary indicator 244. When the auxiliary indicator 244 is disposed on the navigation support arm 2413, the fifth coordinate system O₅ is established based on the position of the auxiliary indicator 244 when the navigation support arm 2413 is in the deployed state.

In some embodiments, the interventional medical system 200 may further include an optical imaging system 250. The optical imaging system 250 may be used to perform optical imaging on the patient, forming a first optical image of the patient. The first optical image complements the medical image of the patient formed by the medical radiation imaging system 220, improving the positioning accuracy of various positions (e.g., in the reference coordinate systems) of the operation end effector 240 (e.g., the surgical execution system 241), thereby enhancing surgical precision and reducing difficulty. In some embodiments, the optical imaging system 250 may include a camera, a video camera, etc. In some embodiments, the optical imaging system 250 may be disposed at the distal end of the navigation support arm 2413 (i.e., the end of the navigation support arm 2413 away from the base 2415) to facilitate imaging of the patient's surgical area. In some embodiments, a sixth coordinate system O₆ of the optical imaging system 250 may be established based on the position of the optical imaging system 250 when the navigation support arm 2413 is in the deployed state.

The workflow of the interventional medical system 200 is described below with reference to the reference coordinate systems of the operation end effector 240.

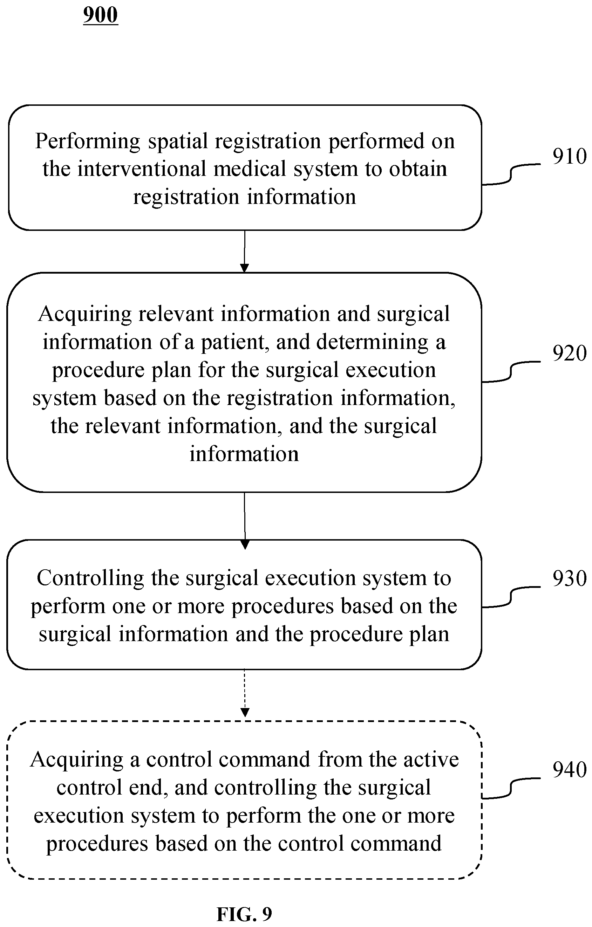

FIG. 9 is a schematic flowchart illustrating an exemplary workflow of an interventional medical system according to some embodiments of the present disclosure. In some embodiments, process 900 may be performed by the interventional medical system 200 or the processor 110. For example, process 900 may be implemented as a set of instructions (e.g., an application) stored in the active control end 230 (e.g., execution host 231) or processor 110 of the interventional medical system 200, or in a memory (e.g., memory 130) external to the active control end 230 or processor 110 and accessible by the active control end 230 or processor 110. The active control end 230 or processor 110 may execute the set of instructions, and when executing the set of instructions, may be configured to perform process 900. The steps of process 900 presented below are intended to be illustrative. In some embodiments, the process may be completed using one or more additional steps not described and/or one or more steps not discussed. Additionally, the order of the steps of process 900 shown in FIG. 9 and described below is not intended to be limiting.

In some embodiments, process 900 may include step 910, step 920, and step 930.

In 910, spatial registration is performed on the interventional medical system 200 to obtain registration information.

In some embodiments, the processor may perform spatial registration based on relevant position information of the interventional medical system 200. The relevant position information of the interventional medical system 200 may include position information of the surgical execution arm 2411, the medical radiation imaging system 220, the reference point, the end effector 2412, the auxiliary indicator 244, the optical imaging system 250, etc. Performing spatial registration on the interventional medical system 200 may include establishing corresponding coordinate systems based on the aforementioned position information. For example, the coordinate systems may include the first coordinate system O₁ of the surgical execution arm 2411, the second coordinate system O₂ of the medical radiation imaging system 220, the third coordinate system O3 of the reference point, the fourth coordinate system O₄ of the end effector 2412, the fifth coordinate system O₅ of the auxiliary procedure assembly (auxiliary indicator 244), the sixth coordinate system O₆ of the optical imaging system 250, etc.

In some embodiments, before starting the interventional medical system 200, the base 2415 needs to be moved to a preset position to facilitate a subsequent procedure on the patient. The preset position may be located near the medical radiation imaging system 220 to allow the procedure on the patient in conjunction with the medical radiation imaging system 220 . In some embodiments, the preset position may also be located near the scanning table 242.

In some embodiments, when the base 2415 is movable (e.g., includes casters), the user may move the base 2415 to the preset position before starting the interventional medical system 200. In some embodiments, when the base 2415 is not movable, i.e., the base 2415 is initially at the preset position, the position of the base 2415 may not need adjustment before starting the interventional medical system 200.

In some embodiments, the processor may be configured to start the interventional medical system 200 at least after the execution mount 24121 is installed onto the surgical execution arm 2411. After the execution mount 24121 is installed, the interventional medical system 200 may be started, and various structures of the interventional medical system 200 enter an operational state. At this time, the processor of the interventional medical system 200 may check the installation status of the execution mount 24121 to confirm whether the execution mount 24121 is properly installed. After confirming that the execution mount 24121 is installed, the position of the end effector 2412 may be confirmed via the execution mount 24121.

In some embodiments, the processor is further configured to, after the interventional medical system 200 satisfies a preset condition, perform spatial registration on the interventional medical system 200 to obtain the registration information.

In some embodiments, the preset condition may include one or more positional conditions of various components of the interventional medical system 200, one or more system conditions, etc. For example, the one or more positional conditions may include that, but are not limited to, the base 2415 is at the preset position and the locking mechanism 2415-3 is locked, the end effector 2412 (e.g., the execution mount 24121) is installed on the distal end of the surgical execution arm 2411, the navigation support arm 2413 is in the deployed state, etc. The one or more system conditions may include that, but are not limited to, all cables are connected, the interventional medical system 200 has started and completed self-check, the account is logged in, a surgical mode is selected, etc. When the interventional medical system 200 satisfies the preset condition, the processor may perform spatial registration on the interventional medical system 200 to obtain the registration information.

In some embodiments, the registration information may be a known parameter and may be obtained directly. For example, when the relative position between the medical radiation imaging system 220 and the surgical execution system 241 is fixed or unchanged, the registration information is a known parameter and may be obtained directly. Specifically, when the medical radiation imaging system 220 and the surgical execution system 241 are respectively fixedly installed (i.e., the base 2415 of the medical radiation imaging system 220 and the surgical execution system 241 are fixedly installed), the registration information is a fixed parameter. When at least one of the medical radiation imaging system 220 or the surgical execution system 241 has a degree of freedom to move, the registration information corresponding to the relative position between the medical radiation imaging system 220 and the surgical execution system 241 may be obtained based on that relative position. In some embodiments, the registration information may be obtained from the processor or input externally.

In some embodiments, the registration information may be an unknown parameter that needs to be reacquired. In some embodiments, the relevant position information of the interventional medical system 200 may be determined to perform spatial registration and obtain the registration information. In some embodiments, the relevant position information may include position information of the surgical execution arm 2411, the medical radiation imaging system 220, the reference point, the end effector 2412, the auxiliary indicator 244, the optical imaging system 250, etc. Performing spatial registration on the interventional medical system 200 may include establishing corresponding coordinate systems based on the aforementioned position information. For example, the coordinate systems may include the first coordinate system O₁ of the surgical execution arm 2411, the second coordinate system O₂ of the medical radiation imaging system 220, the third coordinate system O3 of the reference point, the fourth coordinate system O₄ of the end effector 2412, the fifth coordinate system O₅ of the auxiliary procedure assembly (auxiliary indicator 244), the sixth coordinate system O₆ of the optical imaging system 250, etc. The registration information may include the position information of these coordinate systems and the corresponding mapping relationships of these coordinate systems (e.g., the first mapping relationship between the first coordinate system O₁ and the second coordinate system O₂, the third mapping relationship between the fourth coordinate system O4 and the first coordinate system O₁, the fifth mapping relationship between the sixth coordinate system O₆ and the first coordinate system O₁, etc.).

In some embodiments, after the interventional medical system 200 is started, the operation interfaces of the touch display 2414 and the execution display 233 are opened. The operation interfaces display the registration information of the interventional medical system 200, i.e., the operation interfaces can display the relevant position information of the various structures (e.g., the first coordinate system O₁ of the surgical execution arm 2411, the second coordinate system O₂ of the medical radiation imaging system 220, the third coordinate system O3 of the reference point, the fourth coordinate system O4 of the end effector 2412, the fifth coordinate system O₅ of the auxiliary procedure assembly (auxiliary indicator 244), the sixth coordinate system O₆ of the optical imaging system 250) and display the corresponding mapping relationships (e.g., the first mapping relationship between the first coordinate system O₁ and the second coordinate system O₂, the third mapping relationship between the fourth coordinate system O4 and the first coordinate system O₁, the fifth mapping relationship between the sixth coordinate system O₆ and the first coordinate system O₁, etc.).

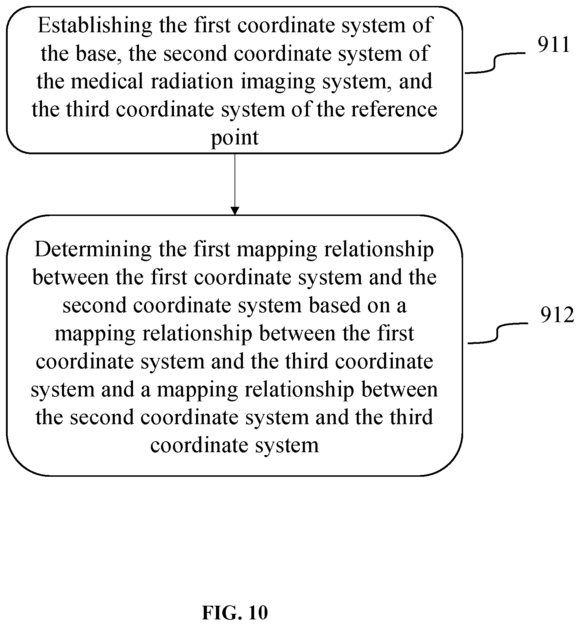

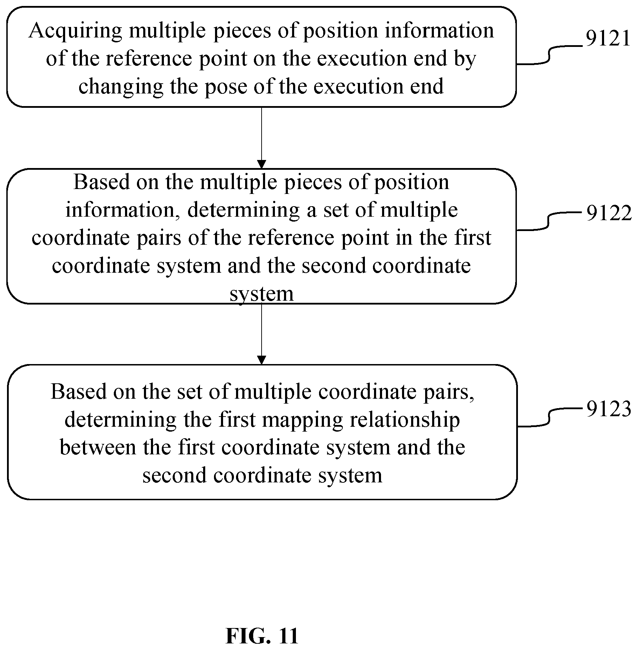

FIG. 10 and FIG. 11 are schematic flowcharts illustrating a process for determining a first mapping relationship according to some embodiments of the present disclosure. Referring to FIG. 10, in some embodiments, step 910 may include sub-steps 911 and 912.

In 911, the first coordinate system O₁ of the base 2415, the second coordinate system O₂ of the medical radiation imaging system 220, and the third coordinate system O3 of the reference point are established.

In some embodiments, for details regarding the first coordinate system O₁, the second coordinate system O₂, and the third coordinate system O3, please refer to FIG. 7A-FIG. 8 and their related descriptions, which will not be repeated here.

In 912, the first mapping relationship between the first coordinate system O₁ and the second coordinate system O₂ is determined based on a mapping relationship between the first coordinate system O₁ and the third coordinate system O3 and a mapping relationship between the second coordinate system O₂ and the third coordinate system O3.

In some embodiments, the reference point may serve as the origin of the third coordinate system O3. Based on the position information of the reference point, combined with coordinates of the reference point in the first coordinate system O₁ and the second coordinate system O₂, the mapping relationship between the first coordinate system O₁ and the third coordinate system O3 (i.e., ${O}_{3}^{{o}_{1}}$) and the mapping relationship between the second coordinate system O₂ and the third coordinate system O3 (i.e., ${O}_{3}^{{o}_{2}}$) may be determined, thereby determining the first mapping relationship between the first coordinate system O₁ and the second coordinate system O₂.

Referring to FIG. 11, in some embodiments, step 912 may include: sub-step 9121 including controlling the surgical execution arm 2411 to move from an initial pose into a scanning area of the medical radiation imaging system 220, and acquiring multiple pieces of position information of the reference point on the end effector 2412 by changing the pose of the end effector 2412; sub-step 9122 including, based on the multiple pieces of position information, determining a set of multiple coordinate pairs of the reference point in the first coordinate system O₁ and the second coordinate system O₂; and sub-step 9123 including, based on the set of multiple coordinate pairs Γ, determining the first mapping relationship between the first coordinate system O₁ and the second coordinate system O₂.

In some embodiments, the initial pose of the surgical execution arm 2411 may be the pose of the surgical execution arm 2411 at the start of spatial registration. In some embodiments, the initial pose of the surgical execution arm 2411 may be determined based on sensors on the surgical execution arm 2411. In some embodiments, when the surgical execution arm 2411 is in the initial pose, the end effector 2412 is installed on the surgical execution arm 2411, i.e., the initial pose of the surgical execution arm 2411 may correspond to the initial pose of the end effector 2412.

The surgical execution arm 2411 moves into the scanning area of the medical radiation imaging system 220, enabling the medical radiation imaging system 220 to scan and locate the reference point, obtaining the position information of the reference point. In some embodiments, each time the pose of the surgical execution arm 2411 changes, the medical radiation imaging system 220 scans and images the surgical execution arm 2411 once, thereby obtaining new position information of the reference point. In some embodiments, by changing the pose of the end effector 2412, the position information of the reference point changes accordingly, and the coordinates of the reference point in the first coordinate system O₁ and the second coordinate system O₂ also change correspondingly. In some embodiments, the position information of the reference point may be obtained by acquiring the pose of the surgical execution arm 2411 (e.g., obtaining position information from encoders at each joint of the surgical execution arm 2411 and performing forward kinematics calculations) or by scanning the reference point with the medical radiation imaging system 220. In some embodiments, each time the position and posture of the surgical execution arm 2411 change, the position information of the reference point is obtained once. By changing the position and posture of the surgical execution arm 2411, multiple (e.g., n, where n is not less than 3) pieces of position information of the reference point may be obtained.

In some embodiments, after the multiple pieces of position information of the reference point are acquired, the surgical execution arm 2411 may be moved back to the initial pose to facilitate positioning of the surgical execution arm 2411 and subsequent control operations.

In some embodiments, based on the position information of the reference point, the coordinate ${O}_{3}^{{o}_{1}}$ of the reference point in the first coordinate system O₁ (i.e., the mapping relationship between the third coordinate system O3 and the first coordinate system O₁) and the coordinate ${O}_{3}^{{o}_{2}}$ of the reference point in the second coordinate system O₂ (i.e., the mapping relationship between the third coordinate system O3 and the second coordinate system O₂) may be determined. In some embodiments, by obtaining position information from one or more encoders at each joint of the surgical execution arm 2411 and performing forward kinematics calculations (i.e., calculating the position and posture of the distal end of the surgical execution arm 2411 based on the angles of joints of the surgical execution arm 2411), the position information of the reference point may be determined, and simultaneously the coordinate ${O}_{3}^{{o}_{1}}$ in the first coordinate system O₁ may be obtained. In some embodiments, by scanning the position and posture of the surgical execution arm 2411 with the medical radiation imaging system 220, the position information of the reference point may also be determined, and simultaneously the coordinate ${O}_{3}^{{o}_{2}}$ in the second coordinate system O₂ may be obtained. For the same piece of position information of the reference point corresponding to the same position and posture of the surgical execution arm 2411, its corresponding coordinate ${O}_{3}^{{o}_{1}}$ in the first coordinate system O₁ and coordinate ${O}_{3}^{{o}_{2}}$ in the second coordinate system O₂ form a coordinate pair. By changing the position and posture of the surgical execution arm 2411 n times, a set of n (e.g., n≥3) coordinate pairs $Γ = \left\{\left({O}_{3 i}^{{o}_{1}}, {O}_{3 i}^{{o}_{2}}\right)\right\}$ of the reference point in the first and second coordinate systems may be obtained, where i=1...n.

In some embodiments, during the process of changing the position and posture of the surgical execution arm 2411 and determining the set of coordinate pairs Γ, the images scanned by the medical radiation imaging system 220 may be transmitted to the imaging host 221 for display. Further, the imaging host 221 may transmit the scanned images to the execution host 231 for archiving, facilitating subsequent determination of planning information, reducing manual import of the patient's scanned images (medical images), and simplifying the surgical workflow. In some embodiments, the medical radiation imaging system 220 scans once each time the position and posture of the surgical execution arm 2411 change. In some embodiments, during multiple changes of the position and posture of the surgical execution arm 2411, the medical radiation imaging system 220 may scan continuously.

In some embodiments, the first mapping relationship between the first coordinate system O₁ and the second coordinate system O₂ may be determined using an ICP (Iterative Closest Point) algorithm. Through the ICP algorithm, a transformation matrix ${M}_{1}^{2}$ between the first coordinate system O₁ and the second coordinate system O₂ may be directly calculated, i.e., the first mapping relationship is ${M}_{1}^{2} = ICP \left(Γ\right)$. In some embodiments, the provision of the multiple coordinate pairs (e.g., at least 3) can reduce errors and improve the calculation accuracy of the first mapping relationship, thereby enhancing surgical precision.

In some embodiments, step 910 may further include acquiring a patient coordinate system O₀ of the patient, and determining a second mapping relationship between the patient coordinate system O₀ and the second coordinate system O₂.

In some embodiments, during a pre-operative scanning phase, the patient lies on the scanning table 242, and the patient coordinate system O₀ is established via the medical radiation imaging system 220. Simultaneously, the second mapping relationship ${M}_{2}^{0}$ between the patient coordinate system O₀ and the second coordinate system O₂ is determined. For more details regarding the patient coordinate system O₀ and the second coordinate system O₂, please refer to FIG. 7A, FIG. 7B and their related descriptions, which will not be repeated here. In some embodiments, the second mapping relationship between the patient coordinate system O₀ and the second coordinate system O₂ refers to the corresponding coordinate transformation relationship between the patient coordinate system O₀ and the second coordinate system O₂, representing the relative positional relationship between the patient and the medical radiation imaging system 220.

It should be noted that for different patient positions, the patient's pose may change, and the corresponding second mapping relationship may also differ. In some embodiments, taking the patient's position during the surgical procedure as HFS (Head First-Supine) as an example, the corresponding standard second mapping relationship ${M}_{2}^{0}$ is an identity matrix.

In some embodiments, step 910 may further include establishing the fourth coordinate system O₄ of the end effector 2412 (or execution mount 24121), and determining a third mapping relationship between the fourth coordinate system O₄ and the first coordinate system O₁. For more details regarding the fourth coordinate system O₄ and the first coordinate system O₁, please refer to FIG. 7A, FIG. 7B and their related descriptions, which will not be repeated here. In some embodiments, the third mapping relationship between the fourth coordinate system O₄ and the first coordinate system O₁ refers to the corresponding coordinate transformation relationship between them. Through the third mapping relationship, the relative positional relationship between the end effector 2412 and the surgical execution system 241 may be represented, facilitating the determination of the position and posture of the end effector 2412, improving the positioning accuracy of the operation pose of the end effector 2412, thereby enhancing surgical precision and reducing difficulty. The operation pose of the end effector 2412 is the pose when it performs a procedure.

In some embodiments, the third mapping relationship between the fourth coordinate system O₄ and the first coordinate system O₁ may be directly determined by the execution host 231. In some embodiments, the third mapping relationship between the fourth coordinate system O₄ and the first coordinate system O₁ may be obtained by acquiring position information from encoders at each joint axis of the surgical execution arm 2411 and using forward kinematics calculations to determine the pose information of the connecting end of the end effector 2412 (i.e., the end near the surgical execution arm 2411, the reference point). Then, combined with the offset between the third coordinate system O3 and the fourth coordinate system O₄, the pose information of the free end of the end effector 2412 (i.e., the end away from the surgical execution arm 2411) may be determined, and simultaneously the coordinates of the free end of the end effector 2412 in the first coordinate system O₁ may be obtained, thus obtaining the third mapping relationship between the fourth coordinate system O₄ and the first coordinate system O₁. In some embodiments, the offset between the third coordinate system O3 and the fourth coordinate system O₄ is the offset from the connecting end to the free end of the end effector 2412.

In some embodiments, the processor is further configured to establish the fifth coordinate system O₅ of the auxiliary indicator 244, and determine a fourth mapping relationship between the fifth coordinate system O₅ and the first coordinate system O₁. The fourth mapping relationship between the fifth coordinate system O₅ and the first coordinate system O₁ refers to the corresponding coordinate transformation relationship between them. Through the fourth mapping relationship, the relative positional relationship between the auxiliary procedure assembly and the surgical execution arm 2411 may be represented, facilitating the determination of the pose of the auxiliary procedure assembly and improving the precision when the auxiliary procedure assembly performs an auxiliary procedure. In some embodiments, the fourth mapping relationship between the fifth coordinate system O₅ and the first coordinate system O₁ may be determined based on design parameters of the surgical execution system 241 (e.g., parameters of a mechanical link between the auxiliary indicator 244 and the surgical execution arm 2411), which may be obtained by consulting documentation or through measurement.

In some embodiments, the processor is further configured to establish the sixth coordinate system O₆ of the optical imaging system 250, and determine a fifth mapping relationship between the sixth coordinate system O₆ and the first coordinate system O₁.

**[**For more details regarding the sixth coordinate system O₆ and the first coordinate system O₁, please refer to FIG. 7A, FIG. 7B and their related descriptions, which will not be repeated here. In some embodiments, the fifth mapping relationship ${M}_{1}^{6}$ between the sixth coordinate system O₆ and the first coordinate system O₁ refers to the corresponding coordinate transformation relationship between them. Through the fifth mapping relationship ${M}_{1}^{6}$, the relative positional relationship between the optical imaging system 250 and the surgical execution system 241 may be represented, facilitating the determination of the pose of the optical imaging system 250 and improving the precision and quality of imaging of the optical imaging system.

In some embodiments, the fifth mapping relationship the optical imaging system 250 between the sixth coordinate system O₆ and the first coordinate system O₁ may be established through "hand-eye" calibration. For example, the surgical execution arm 2411 may serve as a "hand-eye" calibration target. For a pose S₀ of the surgical execution arm 2411, a point cloud model C₁ in the first coordinate system O₁ may be obtained from a design model. When the surgical execution arm 2411 is controlled to move to the pose S₀, the optical imaging system 250 captures an image of the surgical execution arm 2411 to obtain a point cloud C₂. For the point clouds C₁ and C₂, the transformation matrix (fifth mapping relationship) ${M}_{1}^{6}$ between the sixth coordinate system O₆ and the first coordinate system O₁ may be further calculated using the ICP algorithm, i.e., ${M}_{1}^{6} = ICP \left(C1,C2\right)$. The optical imaging system 250 is installed on the navigation support arm 2413. After the interventional medical system 200 is started, the navigation support arm 2413 is adjusted from the stowed state to the deployed state to enable the optical imaging system 250 to work.

In some embodiments, after the interventional medical system 200 is started, the operation interfaces of the touch display 2414 and/or the execution display 233 may display the state of the navigation support arm 2413. When the navigation support arm 2413 is in the stowed state, the operation interfaces of the touch display 2414 and/or the execution display 233 may display prompt information to remind the user to deploy the navigation support arm 2413.

In some embodiments, after the interventional medical system 200 is started and remains powered on, since the base 2415 is locked in the preset position and the position of the medical radiation imaging system 220 remains essentially unchanged, the relative position between the medical radiation imaging system 220 and the base 2415 remains unchanged. That is, the first mapping relationship between the first coordinate system O₁ and the second coordinate system O₂ remains unchanged. Since the initial pose of the surgical execution arm 2411 is known, when the surgical execution arm 2411 is in the initial pose, the relative positions of the surgical execution arm 2411, the reference point, the end effector 2412, and the base 2415 remain unchanged. That is, the third mapping relationship between the fourth coordinate system O₄ and the first coordinate system O₁ remains unchanged. Since the deployed state of the navigation support arm 2413 is known, when the navigation support arm 2413 is in the pose corresponding to the deployed state, the relative position of the optical imaging system 250 installed on the navigation support arm 2413 and the base 2415 remains unchanged. That is, the fifth mapping relationship between the sixth coordinate system O₆ and the first coordinate system O₁ remains unchanged. When the auxiliary indicator 244 is disposed on the surgical execution arm 2411, since the initial pose of the surgical execution arm 2411 is known, when the surgical execution arm 2411 is in the initial pose, the relative position of the auxiliary indicator 244 and the base 2415 remains unchanged. That is, the fourth mapping relationship between the fifth coordinate system O₅ and the first coordinate system O₁ remains unchanged. When the auxiliary indicator 244 is disposed on the navigation support arm 2413, since the deployed state of the navigation support arm 2413 is known, when the navigation support arm 2413 is in the pose corresponding to the deployed state, the relative position of the auxiliary indicator 244 installed on the navigation support arm 2413 and the base 2415 remains unchanged. That is, the fourth mapping relationship between the fifth coordinate system O₅ and the first coordinate system O₁ remains unchanged.

Therefore, when performing multiple surgeries with the interventional medical system 200, as long as the interventional medical system 200 remains powered on and the base 2415 is not moved, it is only necessary to confirm the first mapping relationship between the first coordinate system O₁ and the second coordinate system O₂, the third mapping relationship between the fourth coordinate system O₄ and the first coordinate system O₁, the fourth mapping relationship between the fifth coordinate system O₅ and the first coordinate system O₁, and the fifth mapping relationship between the sixth coordinate system O₆ and the first coordinate system O₁ once at system startup. There is no need to reconfirm them for each surgery. That is, when performing multiple surgeries with the interventional medical system 200, as long as the interventional medical system 200 remains powered on and the base 2415 is not moved, only one spatial registration is needed at startup to obtain registration information. Subsequent surgeries can directly use this registration information without repeating spatial registration, reducing surgical steps, decreasing complexity, and improving efficiency.

In 920, relevant information and surgical information of a patient is acquired, and a procedure plan for the surgical execution system 241 is determined based on the registration information, the relevant information, and the surgical information.

In some embodiments, the relevant information of the patient may include, but is not limited to, one or more medical images, one or more optical images, or the like, of the patient. The surgical information may include, but is not limited to, the type of surgery (e.g., puncture biopsy, fiducial marker placement, guidewire implantation, seed implantation, catheter drainage, ablation therapy, etc.), the surgical area (e.g., chest, abdomen, etc.), the surgical mode (e.g., different control modes), etc. The relevant information and surgical information of the patient may be obtained directly from the processor or manually input by a user (e.g., medical staff). The procedure plan refers to the planned surgical procedure, such as the operation pose of the surgical execution system 241, an executable path, etc. In some embodiments, based on the registration information, the relevant information, and the surgical information, the execution host 231 may determine the procedure plan for the surgical execution system 241. For example, based on the first medical image and an optical image, a contour model of the patient is determined, which includes the patient's lesion area, thereby determining an initial planned pose for the surgical execution system 241 and verifying the initial planned pose. As another example, based on the contour model and the registration information, a mapping model of the patient in a coordinate system (e.g., the first coordinate system O₁ based on the surgical execution arm 2411) may be determined, and the initial planned pose is verified based on the mapping model. In some embodiments, the procedure plan may also be manually input by a user (e.g., medical staff) via the execution display 233 based on the registration information, the relevant information of the patient, and the surgical information.

In some embodiments, step 920 may include acquiring a medical image of the patient from the medical radiation imaging system 220 and controlling the optical imaging system 250 to acquire a first optical image of the patient.

The medical image of the patient may be obtained by scanning the patient with the medical radiation imaging system 220. The medical radiation imaging system 220 may transmit the scanned medical image to the imaging host 221. The imaging host 221 may display the medical image of the patient and transmit the medical image to the execution host 231 for archiving, facilitating observation, improving surgical precision, and reducing difficulty. In some embodiments, the optical imaging system 250 performs contour point cloud acquisition on the patient to obtain the first optical image of the patient.

In some embodiments, after the interventional medical system 200 completes spatial registration, the patient may lie on the scanning table 242, and the optical imaging system 250 captures the patient to obtain the first optical image. The patient lies on the scanning table 242 and enters the scanning area of the medical radiation imaging system 220, which scans the patient to obtain the medical image. During imaging by the optical imaging system 250 and the medical radiation imaging system 220, the patient's position on the scanning table 242 is the same and remains unchanged to enhance imaging accuracy and facilitate the combination of the two images. In some embodiments, both the medical image and the first optical image include the patient's surgical area to facilitate subsequent surgical procedures.

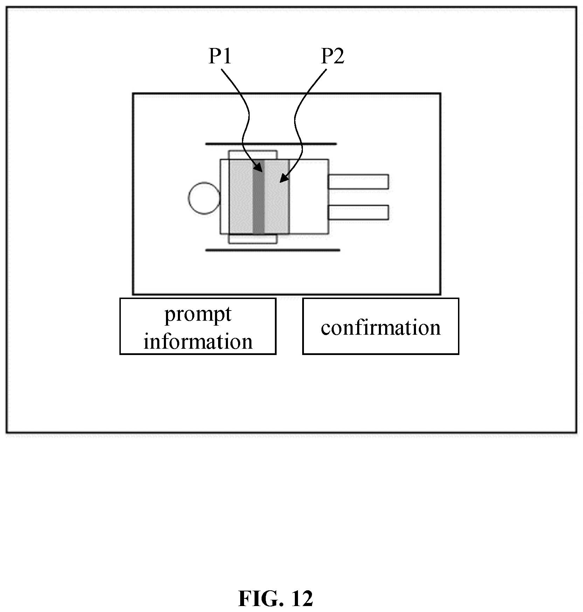

FIG. 12 is a schematic diagram illustrating an operation interface for an optical imaging system to image a patient according to some embodiments of the present disclosure.

Referring to FIG. 12, in some embodiments, based on the patient's initial diagnostic image, a lesion area P1 of the patient may be determined. The initial diagnostic image refers to the scan image from the patient's first examination during the current episode, such as a contrast-enhanced CT image or an MR image.

During the process of the optical imaging system 250 capturing the first optical image of the patient, after the patient lies on the scanning table 242, the user may control the capturing by the optical imaging system 250 on the operation interface displayed on the touch display 2414 or the execution display 233. In some embodiments, the operation interface may display a capture field of view P2 of the optical imaging system 250. The operation interface may display relevant prompt information, such as "Please move the patient's lesion area P1 to the center of the capture field of view P2". The user may control the movement of the scanning table 242 based on the displayed image and the prompt information, thereby adjusting the patient's position within the capture field of view of the optical imaging system 250. In some embodiments, the operation interface may further include a confirmation option. When a condition is satisfied (e.g., the patient's lesion area P1 is moved to the center of the capture field of view P2), the user may select the confirmation option, thereby controlling the optical imaging system 250 to capture the patient and obtain the first optical image. The first optical image is the image displayed on the operation interface when the user selects the confirmation option.

During the process of the medical radiation imaging system 220 scanning the patient to obtain the medical image, after the patient lies on the scanning table 242 and enters the scanning area of the medical radiation imaging system 220, the user may control the scanning of the medical radiation imaging system 220 on the operation interface. In some embodiments, the operation interface may display a scan field of view of the medical radiation imaging system 220. The operation interface may display relevant prompt information, such as "Please move the patient's lesion area P1 into the scan field of view". The user can control the movement of the scanning table 242 based on the displayed image and the prompt information, thereby adjusting the patient's position within the scan field of view of the medical radiation imaging system 220. In some embodiments, the operation interface may further include a confirmation option. When a condition is satisfied (e.g., the patient's lesion area P1 is moved into the scan field of view), the user may select the confirmation option, thereby causing the medical radiation imaging system 220 to scan the patient and obtain the medical image. The medical image is the image displayed on the operation interface when the user selects the confirmation option.

In some embodiments, the medical radiation imaging system 220 may transmit the scanned medical image to the imaging host 221. The imaging host 221 may display the medical image and transmit the medical image to the execution host 231 for archiving, facilitating observation, improving surgical precision, and reducing difficulty. In some embodiments, the optical imaging system 250 may transmit the patient's first optical image to the execution host 231 for processing, facilitating subsequent surgical procedures.

In some embodiments, during the pre-operative scanning phase, the patient lies on the scanning table 242, and the patient coordinate system O₀ is established via the medical radiation imaging system 220. Simultaneously, the second mapping relationship ${M}_{2}^{0}$ between the patient coordinate system O₀ and the second coordinate system O₂ is determined.

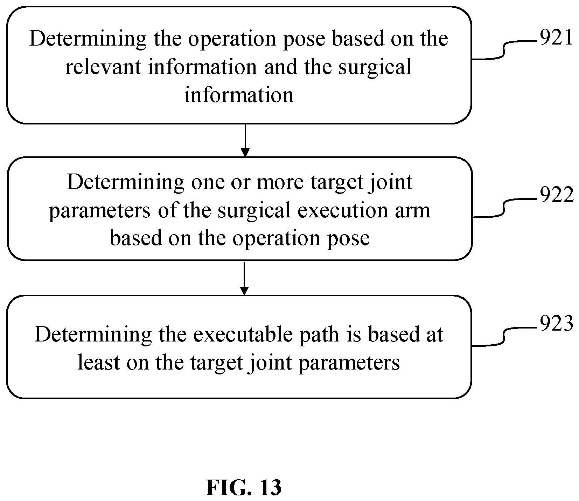

FIG. 13 is a schematic flowchart illustrating a process for determining target joint parameters according to some embodiments of the present disclosure.

In some embodiments, step 920 may include sub-steps 921, 922, and 923.

In 921, the operation pose is determined based on the relevant information and the surgical information.

In some embodiments, based on the patient's relevant information (e.g., medical image, target point, entry point, etc.) and the surgical information (e.g., surgical area, etc.), an operation area for the end effector 2412 may be determined, thereby determining an operation pose for the end effector 2412. The operation pose is a pose under a theoretical condition that allows the end effector 2412 to operate on the patient within the operation area.

In 922, one or more target joint parameters of the surgical execution arm 2411 are determined based on the operation pose. In some embodiments, the one or more target joint parameters may refer to one or more target parameters for each joint of the surgical execution arm 2411, such as a target angle. In some embodiments, based on the operation pose of the surgical execution arm 2411, the execution host 231 may determine the poses of the components of the surgical execution arm 2411, thereby determining the target joint parameters, enabling the surgical execution system 241 to move to the planned pose for subsequent surgical procedures.

In some embodiments, based on the relevant information, the coordinates of the target point in the patient coordinate system O₀ may be determined as T (Xₜ, Yₜ, Zₜ), and the coordinates of the entry point in the patient coordinate system O₀ as E (Xₑ, Yₑ, Zₑ). The corresponding coordinate of the target point T in the first coordinate system O₁ is T', and the corresponding coordinate of the entry point E in the first coordinate system O₁ is E'. For more details on the target point and the entry point, please refer to the subsequent related description.

In some embodiments, based on the third mapping relationship between the fourth coordinate system O₄ and the first coordinate system O₁, a second calculation function for the target joint parameters may be determined. Based on the third mapping relationship, the coordinates of the free end of the end effector 2412 (i.e., the end away from the surgical execution arm 2411) in the first coordinate system O₁ may be determined, thereby determining the position and posture corresponding to the operation pose of the end effector 2412. Using the position and posture of the end effector 2412, an inverse kinematics calculation is performed to determine the second calculation function IK₁ corresponding to the first coordinate system O₁ for the target joint parameters. In some embodiments, based on the coordinate E' of the entry point in the first coordinate system O₁, the coordinate T' of the target point in the first coordinate system O₁, and the second calculation function IK₁, an inverse kinematics calculation is performed to determine the target joint parameters Qₜ=IK₁(E', T').

In 923, the executable path is determined based at least on the one or more target joint parameters.

In some embodiments, the executable path of the surgical execution system 241 (or surgical execution arm 2411) refers to the movement path during its adjustment to the operation pose. In some embodiments, after determining the target joint parameters and combining the target joint parameters with the initial pose of the surgical operation assembly (surgical execution arm 2411 and end effector 2412), the theoretical change in the pose of the surgical operation assembly may be determined, thereby defining the executable path for the surgical execution arm 2411. Since the surgical execution system 241 needs to move to the operation pose, the joints of the surgical execution arm 2411 need to be adjusted to preset angles. To avoid collision with the patient during movement and adjustment of the surgical execution system 241, the executable path for the surgical execution system 241 needs to be planned and calculated.

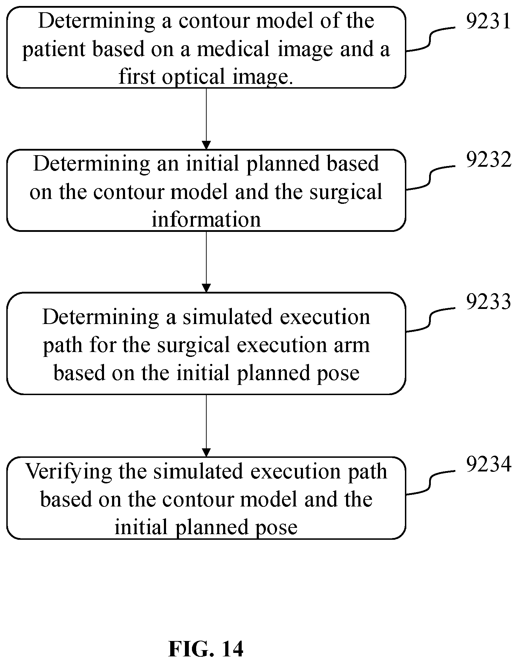

FIG. 14 is a schematic flowchart illustrating a process for determining an executable path according to some embodiments of the present disclosure.

In some embodiments, step 920 (or step 923) may include sub-steps 9231, 9232, 9233, and 9234.

In 9231, a contour model of the patient is determined based on a medical image and a first optical image.

In some embodiments, both the medical image and the first optical image include the patient's lesion area. Combining the medical image with the first optical image clearly displays the lesion area and its surrounding body tissues, thereby enabling determination of the patient's contour model.

In some embodiments, the first optical image includes a first image covering the patient's lesion area, and the medical image covers the patient's lesion area. The aforementioned step 921 may include replacing the first image in the first optical image with the medical image to determine the patient's contour model. In some embodiments, the optical imaging system 250 performs contour point cloud acquisition on the patient to obtain the first optical image, and the acquired data is denoted as Λ₆. By replacing the data corresponding to the first image in the first optical image Λ₆ with the data from the medical image, the patient's contour model ${Λ}_{6}^{′}$ may be obtained.

In some embodiments, based on the relevant mapping relationships from the registration information, a mapping model of the patient's contour model in a corresponding coordinate system may be determined. For example, based on the contour model ${Λ}_{6}^{′}$ and the fifth mapping relationship ${M}_{1}^{6}$, the patient's contour model ${Λ}_{6}^{′}$ in the sixth coordinate system O₆ may be transformed into a mapping model in the first coordinate system O₁. The patient's mapping model is ${Λ}_{1} = {M}_{1}^{6} {Λ}_{6}^{′}$. For details on the fifth mapping relationship ${M}_{1}^{6}$, please refer to the previous description related to coordinate systems, which will not be repeated here.

In the contour model obtained by replacement and fusion, an area image corresponding to the patient's lesion area is scanned by the medical radiation imaging system 220, offering higher accuracy and clarity, which is more conducive to subsequent surgical procedures. In the contour model, an area image not corresponding to the patient's lesion area is captured by the optical imaging system 250, which is easier and quicker to obtain. In the contour model, the area image corresponding to the patient's lesion area is centrally located, providing better visualization for the user. In some embodiments, step 921 may be performed by the execution host 231, and the obtained contour model may be displayed on the execution display 233.

In 9232, an initial planned pose is determined based on the contour model and the surgical information.

In some embodiments, based on the patient's contour model and the surgical information, the operation area for the end effector 2412 may be determined, thereby determining the initial planned pose for the surgical execution arm 2411 and the end effector 2412. The initial planned pose is a pose under the theoretical condition that allows the end effector 2412 to operate on the patient within the operation area.

In some embodiments, based on the contour model, the patient's lesion location may be determined. Combined with the surgical information, the operation area where the end effector 2412 will operate on an area of the patient's body corresponding to the lesion may be determined. Different surgical information may lead to different operation areas for the end effector 2412. For example, when the surgical type in the surgical information is a puncture biopsy, the end effector 2412 may operate from above the patient's lesion location. As another example, when the surgical type is catheter drainage, the end effector 2412 may operate from the inferolateral side of the patient's lesion location. Different operation areas for the end effector 2412 may result in different initial planned poses and correspondingly different simulated execution paths. As another example, when the surgical area differs, the operation area for the end effector 2412 may differ, leading to different corresponding initial planned poses and simulated execution paths. For example, when the surgical area is on the side of the patient facing the base 2415, the surgical area is close to the base with fewer obstacles between , the surgical area and the base, so the simulated execution path may be simpler. When the surgical area is on the side of the patient away from the base 2415, there are more obstacles between the surgical area and the base, so the simulated execution path may be more complex and winding.

In some embodiments, step 9232 may include determining an entry point and a target point based on the contour model and the surgical information, and determining the initial planned pose based on the entry point and the target point.

In some embodiments, based on the contour model, the patient's lesion area may be determined, thereby determining the target point, and subsequently determining the entry point corresponding to the target point.

In some embodiments, based on the entry point and the target point, an initial planned pose may be determined. For example, in the patient coordinate system O₀, the coordinates T (xₜ, yₜ, zₜ) of the target point on the patient and the coordinates E (xₑ, yₑ, zₑ) of the entry point may be determined. Based on the second mapping relationship ${M}_{2}^{0}$ between the patient coordinate system O₀ and the second coordinate system O₂, and the first mapping relationship ${M}_{1}^{2}$ between the first coordinate system O₁ and the second coordinate system O₂, the coordinates T' (xₜ', yₜ', zₜ') of the target point in the first coordinate system O₁ and the coordinates E' (xₑ', yₑ', zₑ') of the entry point in the first coordinate system O₁ may be determined. Subsequently, a pose vector η'=(xₜ'-x_{e'}, yₜ'-yₑ', zₜ'-zₑ') corresponding to the initial planned pose of the end effector 2412 at the entry point may be determined.

In 9233, a simulated execution path for the surgical execution arm 2411 is determined based on the initial planned pose.

In some embodiments, after determining the initial planned pose, combined with the initial pose of the surgical operation assembly (the surgical execution arm 2411 and the end effector 2412), the theoretical change in the pose of the surgical operation assembly may be determined, thereby determining the simulated execution path for the surgical execution arm 2411. That is, via the simulated execution path, the surgical execution arm 2411 can theoretically drive the end effector 2412 to reach the initial planned pose.

In some embodiments, whether the surgical execution arm 2411 can theoretically drive the end effector 2412 to reach the initial planned pose via the simulated execution path needs to be verified.

In 9234, the simulated execution path is verified based on the contour model and the initial planned pose.

In some embodiments, based on the patient's contour model (or the mapping model) and the initial planned pose, the position and posture of the end effector 2412 within the operation area may be determined, thereby verifying whether the surgical execution arm 2411 can reach the position in the operation area unobstructed via the simulated execution path.

When the surgical execution arm 2411 can theoretically drive the end effector 2412 to reach the initial planned pose via the simulated execution path without interfering or colliding with the patient along the simulated execution path, it indicates that the initial planned pose and the simulated execution path have passed verification. The initial planned pose of the end effector 2412 then corresponds to the operation pose, and the simulated execution path corresponds to the executable path.

The operation pose may refer to the operation position of the end effector 2412 and the corresponding operation posture when the surgical execution arm 2411 performs the procedure. In some embodiments, the operation pose may include an operation position and an operation posture, where the operation posture may be determined based on the operation position. In some embodiments, the operation position may be a position determined using the patient coordinate system O₀ as a reference. Based on the mapping relationship between the patient coordinate system O₀ and the first coordinate system O₁, the corresponding position of the operation position in the first coordinate system O₁ may be determined. In some embodiments, the operation position may include the entry point E located on the patient's body surface. The entry point E corresponds to the target point T located in the patient's lesion area. Both the target point T and the entry point E are determined with reference to the patient's body, and the operation posture may correspondingly include the operation posture of the end effector 2412 at the entry point E.

The target point refers to a location on the patient's body corresponding to the lesion area. In some embodiments, the target point may be directly determined from the patient's medical image. In some embodiments, the target point may be a position selected or input by a user (e.g., medical staff) on the operation interface of the execution display 233 or the touch display 2414. The entry point refers to the location on the patient's body where the procedure is actually performed. For example, the target point may be inside the patient's body, while the entry point may be on the patient's body surface. The end effector 2412 performs the procedure at the entry point, ultimately achieving the surgical effect on the target point. In some embodiments, the distance between the entry point and the corresponding target point should not be too large to avoid an ineffective surgical procedure on the lesion. In some embodiments, the distance between the entry point and the corresponding target point may not exceed 5 cm, i.e., the target point may be located within a circular area with a radius of 5 cm centered on the entry point. In some embodiments, the specific distance between the entry point and the corresponding target point may be determined based on the actual situation.

In some embodiments, the initial planned pose may include an initial planned position and an initial planned posture. The initial planned position includes an entry point on the patient's body surface that corresponds to the target point in the lesion area. When verification passes, the initial planned position is determined as the operation position, and the initial planned posture is determined as the operation posture.

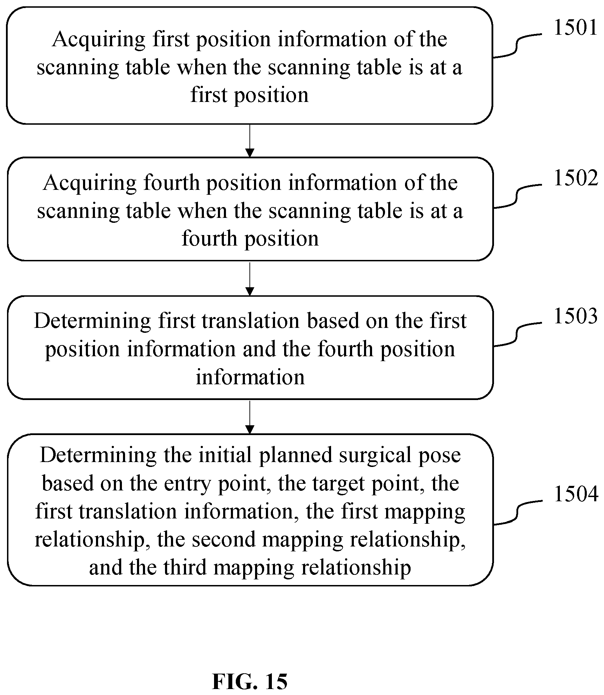

FIG. 15 is a schematic flowchart illustrating a process for determining an initial planned surgical pose according to some embodiments of the present disclosure.

In some embodiments, the scanning table 242 has one or more translational degrees of freedom to move relative to the imaging area of the medical radiation imaging system 220. The imaging area refers to the medical scanning area of the medical radiation imaging system 220. In some embodiments, the aforementioned translational degrees of freedom may include a horizontal movement degree of freedom and a vertical movement degree of freedom. The horizontal movement degree of freedom may refer to the ability of the scanning table 242 to move horizontally towards or away from the imaging area of the medical radiation imaging system 220, allowing the scanning table 242 to enter and exit the imaging area. The vertical movement degree of freedom may refer to the ability of the scanning table 242 to move vertically within the imaging area of the medical radiation imaging system 220 to adjust its position. In some embodiments, the relative height between the scanning table 242 and the medical radiation imaging system 220 is fixed, meaning that when the scanning table 242 translates vertically (e.g., in its height direction), the medical radiation imaging system 220 (e.g., the gantry 224) translates synchronously, so the corresponding vertical movement degree of freedom may be ignored, and only the horizontal movement degree of freedom of the scanning table 242 is considered. In some embodiments, the relative height between the scanning table 242 and the medical radiation imaging system 220 is variable, and the scanning table 242 can move vertically relative to the medical radiation imaging system 220, requiring consideration of both its horizontal and vertical movement degrees of freedom. When the scanning table 242 moves, the relative position between the patient coordinate system O₀ and the second coordinate system O₂ changes.

In some embodiments, the procedure comprises a surgical procedure. The operation pose of the surgical execution arm 2411 includes a surgical operation pose of the surgical execution arm 2411. The surgical operation pose includes a surgical operation position and a surgical operation posture. The initial planned pose includes an initial planned surgical pose, which includes an initial planned surgical position and an initial planned surgical posture. The simulated execution path includes a simulated surgical execution path, and the executable path includes a surgical executable path. When verification passes in step 9234, the initial planned surgical pose is determined as the surgical operation pose, the operation position includes the surgical operation position, and the operation posture includes the surgical operation posture.

The aforementioned sub-step of step 9232, "determining the initial planned pose based on the entry point and the target point," may include sub-steps 1501, 1502, 1503, and 1504.

In 1501, first position information of the scanning table 242 when the scanning table 242 is at a first position is acquired.

In some embodiments, when the patient's scanning position remains unchanged, because the patient lies on the scanning table 242 and the scanning table 242 has translational degrees of freedom relative to the imaging area of the medical radiation imaging system 220, the scanning table 242 can translate horizontally perpendicular to the imaging scanning plane S to enter or exit the medical radiation imaging system 220. In some embodiments, the scanning table 242 may also translate vertically along its height direction to adjust the patient's position within the imaging area. Therefore, the relative position change between the patient coordinate system O₀ and the second coordinate system O₂ may involve translation in the direction of the scanning table's horizontal movement, and may also involve translation in the direction of the scanning table's vertical movement.

In some embodiments, the first position may refer to the initial position of the scanning table 242, for example, the position when the interventional medical system 200 performs spatial registration. In some embodiments, the first position may be located outside the imaging area of the medical radiation imaging system 220 to facilitate the patient getting on and off the scanning table 242. In some embodiments, when the surgical execution system 241 performs spatial registration, the first position information of the scanning table 242 at the first position may be acquired simultaneously. Based on the first position information, the patient coordinate system O₀ is determined. The first position information refers to the position coordinates of the scanning table 242 in a relevant reference coordinate system when the scanning table 24 is at the first position. In some embodiments, the first position information is the coordinate position (0, Y_{cr}, Z_{cr}) of the scanning table 242 in the second coordinate system O₂.

In 1502, fourth position information of the scanning table 242 when the scanning table 242 is at a fourth position is acquired.

In some embodiments, the fourth position is the position of the scanning table 242 when the patient undergoes the surgical procedure. In some embodiments, the fourth position is determined based on the target point. In some embodiments, the fourth position is the position of the scanning table 242 when the target point T is located within the imaging area of the medical radiation imaging system 220 (e.g., in a real-time control mode or a breakpoint control mode), allowing the surgical execution system 241 to perform the surgical procedure on the patient's lesion guided by relevant images. In some embodiments, the fourth position may be the position of the scanning table 242 when the target point T is located outside the imaging area of the medical radiation imaging system 220 (e.g., in a manual control mode), allowing the user (medical staff) to perform the surgical procedure on the patient. In some embodiments, the fourth position information refers to the position coordinates of the scanning table 242 in a relevant reference coordinate system when the scanning table 242 is at the fourth position. In some embodiments, when the surgical mode is a real-time control mode or a breakpoint control mode, the target point T corresponding to the fourth position information of the scanning table 242 is located at the collimation center line of the imaging scanning plane S. In some embodiments, when the surgical mode is a manual control mode, the target point T corresponding to the fourth position information of the scanning table 242 is located outside the imaging area of the medical radiation imaging system 220. In some embodiments, the scanning table 242 moves horizontally perpendicular to the imaging scanning plane S, and the fourth position information is the coordinate position (0, Yₜ, Zₜ) of the scanning table 242 in the second coordinate system O₂.

In 1503, first translation information is determined based on the first position information and the fourth position information.

In some embodiments, based on the first position information and the fourth position information, the position change of the scanning table 242 within the second coordinate system may be determined, i.e., the first translation information of the scanning table 242 is (0, Y_{cr}-Yₜ, Z_{cr}-Zₜ). When the relative height between the scanning table 242 and the medical radiation imaging system 220 is fixed, the value of Y_{cr}-Yₜ is 0.

In 1504, the initial planned surgical pose is determined based on the entry point, the target point, the first translation information, the first mapping relationship, the second mapping relationship, and the third mapping relationship.

In some embodiments, based on the entry point E, the target point T, the first mapping relationship, the second mapping relationship, the third mapping relationship, and the first translation information, the coordinates of the target point T for the initial planned surgical pose in the second coordinate system O₂ may be determined as $Transl \left(0, {Y}_{cr}-{Y}_{t}, {Z}_{cr}-{Z}_{t}\right) { M}_{2}^{0} {T}_{0}$, and the coordinates of the entry point E for the initial planned surgical pose in the second coordinate system O₂ as $Transl \left(0, {Y}_{cr}-{Y}_{t}, {Z}_{cr}-{Z}_{t}\right) { M}_{2}^{0} {E}_{0}$. Combined with the first mapping relationship, the coordinates of the target point T for the initial planned surgical pose in the first coordinate system O₁ may be determined as $T ′ = {M}_{1}^{2} Transl \left(0, {Y}_{cr}-{Y}_{t}, {Z}_{cr}-{Z}_{t}\right) { M}_{2}^{0} T$, and the coordinates of the entry point E for the initial planned surgical pose in the first coordinate system O₁ as $E ′ = {M}_{1}^{2} Transl \left(0, {Y}_{cr}-{Y}_{t}, {Z}_{cr}-{Z}_{t}\right) { M}_{2}^{0} E$. For details on the first mapping relationship, the second mapping relationship, and the third mapping relationship, please refer to the previous relevant descriptions, which will not be repeated here. Based on the coordinates T' of the target point and E' of the entry point, the initial planned surgical position of the surgical execution arm 2411 may be determined, thereby determining the initial planned surgical posture of the surgical execution arm 2411 when the surgical execution arm 2411 is located at the entry point.

In some embodiments, step 923 may include determining an initial positioning point based on the entry point and the initial planned surgical pose, when verification passes, the initial positioning point is determined as a navigation positioning point; and determining the simulated surgical execution path based on the navigation positioning point and the initial planned surgical pose.

In some embodiments, the navigation positioning point refers to a safe position for the surgical operation assembly before performing the surgical procedure. In some embodiments, the navigation positioning point may be determined based on the entry point. For example, the navigation positioning point may be at a preset safe distance from the entry point, such that when the end effector 2412 of the surgical operation assembly is at the navigation positioning point, the end effector 2412 (e.g., the execution instrument 24122) is at a safe distance from the entry point, preventing it from harming the patient's skin before the surgical procedure. It should be noted that in this specification, the surgical operation assembly (or the surgical execution arm 2411) being at or moving to the navigation positioning point means that the tip of the end effector 2412 of the surgical operation assembly (or surgical execution arm 2411) (e.g., the tip of the execution instrument 24122) is at or moving to the navigation positioning point.

In some embodiments, if the safe distance between the initial positioning point (the navigation positioning point) and the entry point is Dₛ, then the coordinates of the initial positioning point (the navigation positioning point) are Eₛ = E' - η'Dₛ. The posture of the end effector 2412 when the end effector 2412 is located at the entry point is consistent with the posture when the end effector 2412 is located at the navigation positioning point (the initial positioning point), both corresponding to the initial planned surgical posture (the surgical operation posture).

In some embodiments, based on the navigation positioning point (the initial positioning point) Eₛ and the pose vector η' of the initial planned surgical posture (the surgical operation posture) when the end effector 2412 is at the entry point, the initial planned surgical posture of the surgical execution arm 2411 at the navigation positioning point may be determined. Then, combining the initial planned surgical position (the navigation positioning point), the initial planned surgical posture of the surgical execution arm 2411 at the navigation positioning point, and the initial pose of the surgical operation assembly (the surgical execution arm 2411 and the end effector 2412), an inverse kinematics solution may be performed to obtain spatial position commands for each joint of the surgical execution arm 2411, thereby obtaining the simulated surgical execution path from the initial pose to the navigation positioning point. When verification passes, the simulated surgical execution path is determined as the surgical executable path, and the initial positioning point is determined as the navigation positioning point.

In some embodiments, during the determination of the procedure plan, the medical radiation imaging system 220 may transmit the patient's medical image to the execution host 231. The execution host 231 may perform segmentation, fusion, and registration on the patient's initial diagnostic image and the medical image, fusing the segmented blood vessels and organs from the initial diagnostic image with the medical image. The result is transmitted to the execution display 233 for display, providing the user with finely visualized tissue information of the patient to assist in surgical planning.

In some embodiments, the left area of the operation interface on the touch display 2414 or the execution display 233 may serve as an auxiliary image interface, displaying a three-dimensional image, a coronal plane image, a sagittal plane image, etc., of the patient. The central area of the operation interface on the execution display 233 may serve as a main image interface, displaying a transverse plane (horizontal plane) image of the patient. Through multi-perspective image display, the user is provided with detailed visualizations of the tissue information of the patient to assist in surgical planning. The three-dimensional image, the coronal plane image, the sagittal plane image, and the transverse plane (horizontal plane) image of the patient can all be obtained from the fused image of the initial diagnostic image and the medical image.

In some embodiments, the right area of the operation interface may serve as a parameter area, displaying surgical information and the procedure plan. For example, the right area of the operation interface may display the surgical type, the surgical area, the surgical mode, the operation area of the execution instrument 24122, the operation pose of the execution instrument 24122, etc.

In some embodiments, based on actual needs, the user may perform operations such as zooming in, zooming out, flipping, rotating, overlaying, etc., relevant images on the operation interface, and may also modify data such as the surgical type, the surgical area, the surgical mode, the operation area of the execution instrument 24122, the operation pose of the execution instrument 24122, etc.

In some embodiments, the relevant images may also be processed on the operation interface to simulate and display the operation path of the end effector 2412 at the operation pose.

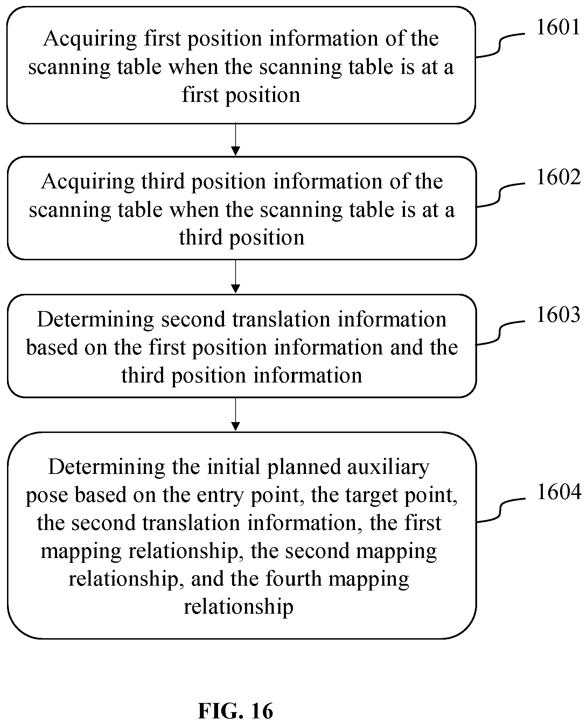

FIG. 16 is a schematic flowchart illustrating a process for determining an initial planned auxiliary pose according to some embodiments of the present disclosure.

In some embodiments, the one or more procedures include one or more auxiliary procedures. The auxiliary indicator 244 is disposed on the surgical execution arm 2411 or the end effector 2412. The operation pose of the surgical execution arm 2411 includes an auxiliary operation pose of the surgical execution arm 2411. The auxiliary operation pose includes an auxiliary operation position and an auxiliary operation posture. The initial planned pose includes an initial planned auxiliary pose, which includes an initial planned auxiliary position and an initial planned auxiliary posture. The simulated execution path includes a simulated auxiliary execution path, and the executable path includes an auxiliary executable path. When verification passes in step 9234, the initial planned auxiliary pose is determined as the auxiliary operation pose, the operation position includes the auxiliary operation position, and the operation posture includes the auxiliary operation posture.

The aforementioned sub-step of step 9232, "determining the initial planned pose based on the entry point and the target point," may include sub-steps 1601, 1602, 1603, and 1604.

In 1601, first position information of the scanning table 242 when the scanning table 242 is at a first position is acquired.

In some embodiments, the first position information is the coordinate position (0, Y_{cr}, Z_{cr}) of the scanning table 242 in the second coordinate system O₂. The content of step 1601 may refer to step 1501 and will not be repeated here.

In 1602, third position information of the scanning table 242 when the scanning table 242 is at a third position is acquired.

In some embodiments, the third position is the position of the scanning table 242 when the patient undergoes the auxiliary procedure. In some embodiments, because the auxiliary procedure has a certain effective area, the location of the auxiliary procedure point may be near the entry point. For example, the auxiliary procedure point may be located within a circular area with a preset radius centered on the entry point. The specific distance (i.e., the aforementioned preset radius) between the auxiliary procedure point and the entry point may be determined based on the actual situation. In some embodiments, the auxiliary procedure point may coincide with the corresponding entry point. In some embodiments, the third position is within an indication area of the auxiliary indicator 244. In some embodiments, the third position is the position of the scanning table 242 when the entry point E is located within the indication area of the auxiliary indicator 244. In some embodiments, the third position information refers to the position coordinate of the scanning table 242 in a relevant reference coordinate system when it is at the third position. In some embodiments, the entry point E corresponding to the third position information of the scanning table 242 is located at a position corresponding to a zero position of the auxiliary indicator 244, so that the entry point E and its surrounding area may fall within the indication area of the auxiliary indicator 244 as much as possible for indication. The zero position of the auxiliary indicator 244 may refer to the initial position of the auxiliary indicator 244.

In some embodiments, when the auxiliary indicator 244 is disposed on the surgical execution arm 2411 or the end effector 2412, during the indication of the auxiliary procedure by the auxiliary indicator 244, the position of the auxiliary indicator 244 is located outside the imaging area of the medical radiation imaging system 220.

For example, in the fifth coordinate system O₅, the origin is at the position of the auxiliary indicator 244 at its zero position, and the Z-direction is the horizontal direction for entering or exiting the gantry 224, perpendicular to the imaging scanning plane S. Then, the Z-coordinate of the entry point in the fifth coordinate system O₅ corresponding to the third position is 0.

In some embodiments, the scanning table 242 moves horizontally perpendicular to the imaging scanning plane S, and the third position information is the coordinate position (0, Y'ₜ, Z'ₜ) of the scanning table 242 in the second coordinate system O₂.

In 1603, second translation information is determined based on the first position information and the third position information.

In some embodiments, based on the first position information and the third position information, the position change of the scanning table 242 within the second coordinate system may be determined, i.e., the second translation information of the scanning table 242 is (0, Y_{cr}-Y'ₜ, Z_{cr}-Z'ₜ). When the relative height between the scanning table 242 and the medical radiation imaging system 220 is fixed, the value of Y_{cr}-Y'ₜ is 0.

In 1604, the initial planned auxiliary pose is determined based on the entry point, the target point, the second translation information, the first mapping relationship, the second mapping relationship, and the fourth mapping relationship.

Similar to step 1504, based on the entry point E, the target point T, the first mapping relationship, the second mapping relationship, the fourth mapping relationship, and the second translation information, the coordinates of the target point T for the initial planned auxiliary pose in the second coordinate system O₂ and the coordinates of the entry point E for the initial planned auxiliary pose in the second coordinate system O₂ may be determined. Combined with the first mapping relationship, the coordinate T" of the target point T and the coordinate E" of the entry point E for the initial planned auxiliary pose in the first coordinate system O₁ may be determined. Based on the coordinates T" and E", the initial planned auxiliary position of the surgical execution arm 2411 may be determined, thereby determining the initial planned auxiliary posture of the surgical execution arm 2411 when located at the entry point, and consequently the initial planned auxiliary pose of the surgical execution arm 2411.

It should be noted that the principle for determining the target joint parameters corresponding to the initial planned surgical pose (the surgical operation pose) when the surgical execution arm 2411 performs a surgical procedure is the same as the principle for determining the target joint parameters corresponding to the initial planned auxiliary pose (the auxiliary operation pose) when the surgical execution arm 2411 performs auxiliary indication.

In some embodiments, the procedure comprises an auxiliary procedure, and the auxiliary indicator 244 is disposed on the navigation support arm 2413. The step "determining a procedure plan for the surgical execution system based on the registration information, the relevant information, and the surgical information" in step 920 further includes determining an auxiliary indication pose of the navigation support arm 2413 based on the surgical operation pose of the surgical execution arm 2411. Specifically, determining an auxiliary indication pose of the navigation support arm 2413 based on the surgical operation pose of the surgical execution arm 2411 may include the following steps including determining the third position of the scanning table 242 based on the surgical operation pose of the surgical execution arm 2411, acquiring third position information of the scanning table 242 at the third position; determining third translation information based on the fourth position information and the third position information; and determining the auxiliary indication pose based on the fourth mapping relationship and the third translation information.

In some embodiments, the patient undergoes the surgical procedure at the fourth position via the entry point and the auxiliary procedure at the third position via the entry point. The auxiliary indicator 244 may indicate the entry point for the auxiliary procedure. In some embodiments, the entry point E corresponding to the third position information of the scanning table 242 is located at the position corresponding to the zero position of the auxiliary indicator 244. The zero position of the auxiliary indicator 244 may refer to the initial position of the auxiliary indicator 244. It should be noted that the positions of the auxiliary indicator 244 mentioned above are all its positions when the navigation support arm 2413 is in the deployed state.

**[**For example, in the fifth coordinate system O₅, the origin is at the position of the auxiliary indicator 244 at its zero position, and the Z-direction is the horizontal direction for entering or exiting the gantry 224, perpendicular to the imaging scanning plane S. Then, the Z-coordinate of the entry point in the fifth coordinate system O₅ corresponding to the third position is 0.

In some embodiments, the scanning table 242 enters or exits the medical radiation imaging system 220 by moving horizontally perpendicular to the imaging scanning plane S. Therefore, based on the fourth position information and the third position information, the position change of the scanning table 242 within the second coordinate system may be determined, thereby determining the third translation information of the scanning table 242.

In some embodiments, based on the third translation information, the position change of the scanning table 242 within the first coordinate system O₁ may be determined. Combined with the fourth mapping relationship between the fifth coordinate system O₅ and the first coordinate system O₁, the coordinates of the entry point E at the third position in the first coordinate system O₁ may be determined, thereby determining the position and posture of the auxiliary indicator 244, and consequently determining the auxiliary indication pose of the navigation support arm 2413.

In some embodiments, using the position and posture of the navigation support arm 2413 (or the auxiliary indicator 244), an inverse kinematics calculation is performed to determine a first calculation function IK₅ corresponding to the first coordinate system O₁ for auxiliary procedure control parameters of the navigation support arm 2413. In some embodiments, based on the position and posture of the auxiliary indicator 244 and the first calculation function IK₅, an inverse kinematics calculation is performed to determine the auxiliary procedure control parameters θ = IK₅(E, O₅). By determining the auxiliary procedure control parameters θ, the auxiliary indication pose of the auxiliary indicator 244 may be determined, allowing it to indicate the specific location of the entry point E for the auxiliary procedure (e.g., local anesthesia) on the patient's body according to the auxiliary indication pose, facilitating subsequent surgical execution.

In 930, the surgical execution system 241 is controlled to perform one or more procedures based on the surgical information and the procedure plan.

In some embodiments, based on the surgical information and the procedure plan, the operation pose and the executable path for the end effector 2412 may be determined. The surgical execution system 241 is controlled to adjust the end effector 2412 to the operation pose along the executable path and perform a corresponding procedure at the corresponding entry point.

In some embodiments, based on the initial planned surgical pose and the second mapping relationship between the patient coordinate system O₀ and the second coordinate system O₂, the surgical operation pose for the end effector 2412 to perform a surgical procedure may be determined.

In some embodiments, the one or more procedures received by the patient may include a surgical procedure and an auxiliary procedure. Step 930 may include, based on the surgical information and the procedure plan, controlling the auxiliary indicator 244 to indicate the auxiliary procedure, and after the auxiliary procedure is completed, controlling the surgical operation assembly to perform the surgical procedure.

Before performing the surgical procedure on the patient, the auxiliary procedure may be performed first to enhance safety and avoid causing additional harm to the patient. For example, the surgical procedure may correspond to the surgical type in the surgical information, and the auxiliary procedure may include local anesthesia, etc.

In some embodiments, before performing the surgical procedure on the patient, the patient needs to be moved out of the imaging area to perform the auxiliary procedure (e.g., anesthesia). That is, the scanning table 242 may be controlled to move to the third position. After the scanning table 242 is moved to the third position, the auxiliary indicator 244 provides guidance according to the auxiliary operation pose of the surgical execution arm 2411 or the auxiliary procedure control parameters θ of the navigation support arm 2413, assisting the user in performing the auxiliary procedure (e.g., local anesthesia) on the patient's surgical area (e.g., the entry point E).

In some embodiments, step 930 may include controlling the surgical operation assembly to move to the navigation positioning point, and after the surgical operation assembly moves to the navigation positioning point, controlling the surgical operation assembly to perform the surgical procedure based on the surgical mode. For more details on "controlling the surgical operation assembly to perform the surgical procedure based on the surgical mode," please refer to the subsequent description related to "surgical mode."

In some embodiments, the step "controlling the surgical execution system 241 to perform one or more procedures based on the surgical information and the procedure plan" in step 930 may include, in response to completion of spatial registration, controlling the scanning table 242 to move to a second position, in response to completion of scanning the patient by the medical radiation imaging system 220, controlling the scanning table 242 to move to a fourth position, in response to the scanning table 242 moving to the fourth position, controlling the surgical execution arm 2411 to adjust to a surgical operation pose; and in response to the surgical execution arm 2411 adjusting to the surgical operation pose, controlling the end effector 2412 to perform the surgical procedure. The second position is the position of the scanning table 242 when the medical radiation imaging system 220 scans the patient.

In some embodiments, when the scanning table 242 is at the second position, the medical radiation imaging system 220 scans the patient, forming a medical image of the patient. Based on the medical image, the positions of the target point T and the entry point E in the patient coordinate system O₀ may be determined, thereby determining the fourth position of the scanning table 242 (e.g., the position when the scanning table 242 is moved so that the target point T is at the collimation center line of the imaging scanning plane S of the medical radiation imaging system 220) and the third position (e.g., the position when the scanning table 242 is moved so that the entry point E is at the zero position of the auxiliary indicator 244). In some embodiments, the second position and the fourth position may be the same or different.

In some embodiments, the aforementioned "in response to the scanning table 242 moving to the fourth position, controlling the surgical execution arm 2411 to adjust to a surgical operation pose" includes (1) in response to the scanning table 242 moving to the fourth position, controlling the surgical execution arm 2411 to adjust to an installation pose, (2) in response to the surgical execution arm 2411 adjusting to the installation pose, controlling the execution mount 24121 to install the execution instrument 24122, (3) in response to completion of installation of the execution instrument 24122, controlling the surgical execution arm 2411 to adjust to the surgical operation pose. The installation pose is any feasible pose for the surgical execution arm 2411 that avoids the patient and does not interfere with them.

In some embodiments, before performing the surgical procedure on the patient, an auxiliary procedure needs to be performed first. The aforementioned "in response to completion of scanning the patient on the scanning table 242 at the first position by the medical radiation imaging system 220, controlling the scanning table 242 to move to a fourth position" may include in response to completion of scanning the patient by the medical radiation imaging system 220, controlling the scanning table 242 to move to a third position, in response to the scanning table 242 moving to the third position, controlling the auxiliary indicator 244 to provide an indication for the auxiliary procedure, and in response to completion of the auxiliary procedure, controlling the scanning table 242 to move to the fourth position.

In some embodiments, the auxiliary indicator 244 is disposed on the surgical execution arm 2411 or the end effector 2412, and the procedure plan includes an auxiliary operation pose of the surgical execution arm 2411. The aforementioned "in response to the scanning table 242 moving to the third position, controlling the auxiliary indicator 244 to provide an indication for the auxiliary procedure" includes in response to the scanning table 242 moving to the third position, controlling the surgical execution arm 2411 to adjust to the auxiliary operation pose, and in response to the surgical execution arm 2411 adjusting to the auxiliary operation pose, controlling the auxiliary indicator 244 to provide the indication for the auxiliary procedure.

In some embodiments, the auxiliary indicator 244 is disposed on the navigation support arm 2413, and the procedure plan includes an auxiliary indication pose of the navigation support arm 2413. The aforementioned "in response to the scanning table 242 moving to the third position, controlling the auxiliary indicator 244 to provide an indication for the auxiliary procedure" includes in response to the scanning table 242 moving to the third position, controlling the navigation support arm 2413 to adjust to the auxiliary indication pose, and in response to the navigation support arm 2413 adjusting to the auxiliary indication pose, controlling the auxiliary indicator 244 to provide the indication for the auxiliary procedure.

In some embodiments, after part or all of the surgical procedure is completed on the patient, the surgery may be paused or stopped. To facilitate the patient getting on and off the scanning table 242, the scanning table 242 may be moved to a fifth position, which may be located outside the imaging area of the medical radiation imaging system 220. That is, the step "controlling the surgical execution system 241 to perform one or more procedures based on the surgical information and the procedure plan" in step 930 may further include, in response to completion of at least a portion of the surgical procedure, controlling the scanning table 242 to move to the fifth position. The fifth position may be the same as or different from the third position.

In some embodiments, process 900 may further include step 940.

In 940, a control command is acquired from the active control end 230, and the surgical execution system 241 is controlled to perform the one or more procedures based on the control command.

In some embodiments, the control command may include a command generated by the master manipulator 232 and transmitted to the surgical execution system 241. In some embodiments, the control command may include a command generated by the operation end effector 240 (e.g., the touch display 2414, etc.). The control command may include an instruction for controlling the surgical execution system 241, such as a movement instruction for the scanning table 242.

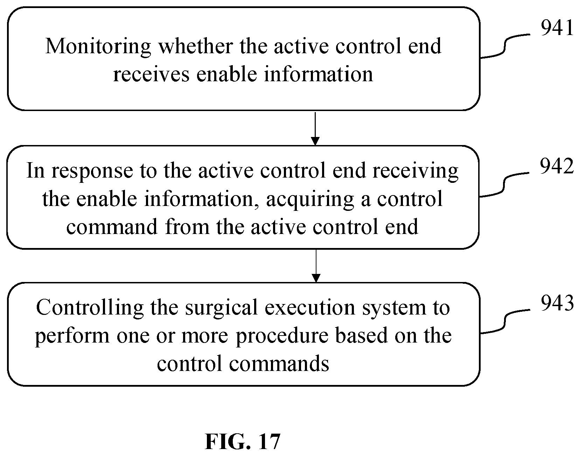

FIG. 17 is a schematic flowchart illustrating a process for controlling a surgical execution system based on a control command according to some embodiments of the present disclosure. As shown in FIG. 17, in some embodiments, step 940 may include sub-steps 941, 942, and 943.

In 941, whether the active control end 230 receives enable information is monitored.

In some embodiments, the enable information may activate and enable a control command. In some embodiments, the enable information includes first enable information sent by the exposure switch 223 and second enable information sent by the control enable button 2324. In some embodiments, the first enable information enables the activation of the second enable information. That is, after the user operates the exposure switch 223 to issue the first enable information, the first enable information causes the second enable information to take effect, thereby activating and enabling the control command of the master manipulator 232, allowing the master manipulator 232 to control the surgical execution system 241 to perform a procedure. This prevents the user from operating the master manipulator 232 without a view of the patient (e.g., medical images), avoiding additional harm. For more details on the enable information, please refer to the previous description related to the master manipulator 232, which will not be repeated here.

In 942, in response to the active control end 230 receiving the enable information, a control command is acquired from the active control end 230.

In some embodiments, in response to the active control end 230 receiving the enable information, the control command is acquired from the active control end 230. In some embodiments, step 942 may include, in response to both the first enable information and the second enable information being acquired, acquiring the control command from the active control end 230. In some embodiments, the first enable information indirectly activates and enables the control command of the master manipulator 232. Specifically, the user operates the exposure switch 223 to generate the first enable information. The first enable information activates the second enable information generated by the control enable button 2324. Then, in response to the second enable information, the control command from the active control end 230 is acquired.

In 943, the surgical execution system 241 is controlled to perform a procedure based on the control command.

In some embodiments, based on the acquired control command, the surgical execution system 241 is controlled to perform the corresponding procedure. In some embodiments, before controlling the surgical execution system 241 based on the control command, the medical radiation imaging system 220 may be controlled to expose first based on the first enable information, causing the medical radiation imaging system 220 to image the patient. This prevents the user from controlling the surgical execution system 241 via the master manipulator 232 without a view of the patient (e.g., medical images), avoiding additional harm.

In some embodiments, the interventional medical system 200 may further include a surgical field camera 260 (as shown in FIG. 3B). The surgical field camera 260 may be disposed on the gantry 224 or a support platform near the gantry 224. The surgical field camera 260 is used to acquire a second optical image of the patient and transmit the second optical image to the execution display 233 for display. When the surgical execution system 241 performs a procedure, the execution display 233 may present the second optical image in real-time, allowing the user to monitor the patient in real-time and promptly stop the relevant procedure if an anomaly is observed, further enhancing surgical safety.

At this point, the step "controlling the surgical execution system 241 to perform one or more procedures based on the surgical information and the procedure plan" in step 900 may further include controlling the surgical field camera 260 to acquire the second optical image of the patient. The second optical image is presented when the surgical execution system 241 performs the procedure.

In some embodiments, the surgical field camera 260 may acquire the second optical image of the patient in real-time and transmit the second optical image to the execution host 231. The second optical image is presented synchronously when the surgical execution system 241 performs the procedure, allowing the user to monitor the patient in real-time and promptly stop relevant procedure if an anomaly is observed, further enhancing surgical safety.

It should be noted that the above description of process 900 is for illustration and explanation only and does not limit the scope of application of the present disclosure. For those skilled in the art, various modifications and changes may be made to process 900 under the guidance of the present disclosure. However, these modifications and changes are still within the scope of the present disclosure.

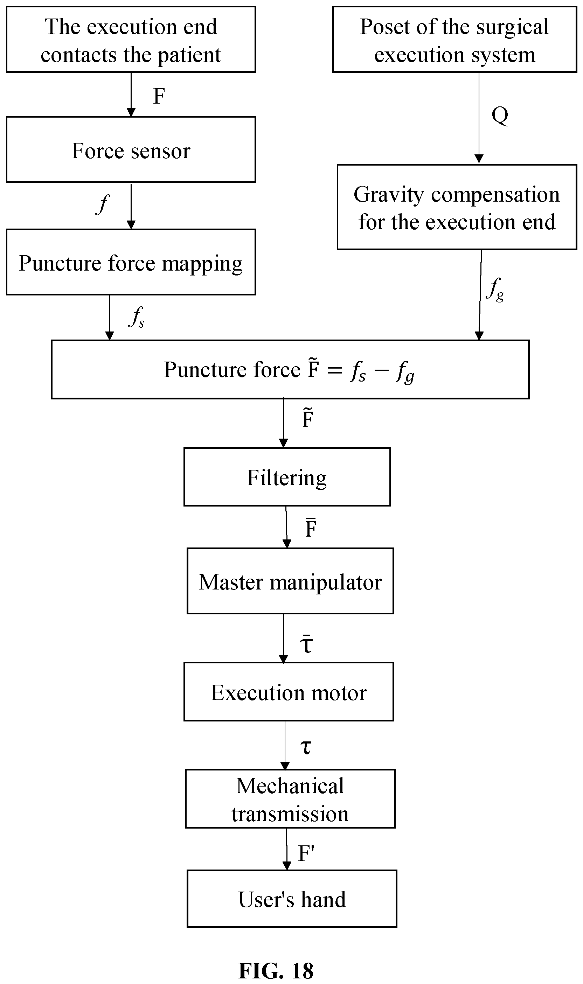

FIG. 18 is a schematic diagram illustrating master-slave control feedback according to some embodiments of the present disclosure.

Referring to FIG. 5 and FIG. 18, in some embodiments, the surgical execution arm 2411 further includes a force sensor 24116. The end effector 2412 is connected with the other end of the fifth articulated arm 24115 via the force sensor 24116. The force sensor 24116 may be used to measure a reaction force F generated by contact between the end effector 2412 and the patient, facilitating force feedback from the master manipulator 232 to the user. As shown in FIG. 18, in some embodiments, during the control of the surgical execution system 241 of the operation end effector 240 by the master manipulator 232 of the active control end 230, the end effector 2412 contacts the patient, generating a reaction force F acting on the end effector 2412 and the surgical execution arm 2411. The surgical execution system 241 calculates a gravity compensation value f_{g} for the end effector 2412 in the puncture direction based on the pose of the end effector 2412. Based on real-time detection of the reaction force F by the force sensor 24116 integrated at the distal end of the surgical execution arm 2411, a force mapping calculation is performed to obtain a puncture force fs. The true puncture force F̃ = fₛ - f_{g}. After filtering, the filtered F̃ is transmitted to the master manipulator 232. The master manipulator 232 controls an execution motor on the end effector 2412 to operate in a torque mode τ̅, outputting a feedback torque τ, which is then transmitted mechanically as a force F' to the user's hand. This achieves real-time force feedback during master-slave control, enhancing the user's realistic operating experience, thereby improving operational precision and reducing difficulty.

In some embodiments, when the patient has multiple lesions or a large lesion area, multiple surgical procedures may be required. That is, the surgical operation pose may include multiple target points or multiple entry points. When the scanning table 242 is at the second position, the target points corresponding to the multiple entry points are all located within the imaging area of the medical radiation imaging system 220, meaning all the target points may be displayed on the patient's medical image. In this case, each entry point may correspond to a fourth position. In some embodiments, when the surgical mode includes a navigation control mode or a breakpoint control mode, the target point corresponding to each entry point is respectively located at the collimation center line of the imaging scanning plane S of the medical radiation imaging system 220, and each entry point may respectively correspond to a fourth position. In some embodiments, when the surgical mode includes a manual control mode, the target points corresponding to the multiple entry points are all located outside the scanning area of the medical radiation imaging system 220, and the fourth positions corresponding to the multiple entry points may be the same position. Each entry point may respectively correspond to a surgical operation pose of the surgical operation assembly (the surgical execution arm 2411 and the end effector 2412). In some embodiments, when the target points corresponding to the multiple entry points are all located at the collimation center line of the imaging scanning plane S of the medical radiation imaging system 220, the fourth positions corresponding to the multiple entry points may be the same, and the corresponding surgical operation poses of the surgical operation assembly may be the same.

In some embodiments, when the target planned pose includes multiple entry points, one or more auxiliary procedures may be required. In some embodiments, the distribution area of the multiple entry points has a center point (e.g., the geometric center of a polygon formed by sequentially connecting the outermost entry points). In some embodiments, the location of the center point may be selected by a user (e.g., medical staff).

In some embodiments, when the auxiliary indicator 244 is disposed on the navigation support arm 2413, the center point may be located at the position corresponding to the zero position of the auxiliary indicator 244, corresponding to the scanning table 242 being at the third position. That is, the multiple entry points may correspond to a single third position. After the scanning table 242 is moved to the third position, the auxiliary indicator 244 may adjust its position and posture to indicate the locations of the multiple entry points, respectively, facilitating the auxiliary procedure at each of the multiple entry points. In some embodiments, each entry point may correspond to a pose of the auxiliary indicator 244, i.e., each entry point may correspond to an auxiliary procedure control parameter θ of the navigation support arm 2413. When the auxiliary indicator 244 needs to indicate a target entry point, the target pose of the auxiliary indicator 244 (or the target auxiliary indication pose of the navigation support arm 2413) may be determined based on the target entry point, and the auxiliary indicator 244 is adjusted to the target pose to indicate the target entry point. In some embodiments, the pose of the auxiliary indicator 244 corresponding to any entry point may be determined based on the relative position of that entry point to the center point and the second translation information. By setting the center point at the position corresponding to the zero position of the auxiliary indicator 244, it is possible to ensure that all multiple entry points are located within the indication area of the auxiliary indicator 244 as much as possible.

In some embodiments, when the auxiliary indicator 244 is disposed on the surgical execution arm 2411 or the end effector 2412, the center point is located within the indication area of the auxiliary indicator 244, corresponding to the scanning table 242 being at the third position. That is, the multiple entry points may correspond to a single third position. After the scanning table 242 is moved to this third position, the auxiliary indicator 244 may adjust its position and posture to indicate the locations of the multiple entry points, respectively, facilitating the auxiliary procedure for each of the multiple entry points. In some embodiments, each entry point may correspond to a pose of the auxiliary indicator 244, i.e., each entry point corresponds to an auxiliary operation pose of the surgical execution arm 2411. When the auxiliary indicator 244 needs to indicate a target entry point, the target pose of the auxiliary indicator 244 (the target auxiliary operation pose of the surgical execution arm 2411) may be determined based on the target entry point, and the auxiliary indicator 244 is adjusted to the target pose to indicate the target entry point.

In some embodiments, because the auxiliary procedure has a certain effective area, for the multiple entry points, the auxiliary procedure may be performed only once at the aforementioned center point. In some embodiments, to ensure the smooth progress of the surgery, the auxiliary procedure may be performed at or near each of the multiple entry points.

In some embodiments, when performing multiple auxiliary procedures, the scanning table 242 may be moved to the third position, and after performing the auxiliary procedures for all entry points, the scanning table 242 is then moved sequentially through the multiple fourth positions to perform surgical procedures on the corresponding entry points. Specifically, after moving the scanning table 242 to the third position, the pose of the auxiliary indicator 244 is adjusted to indicate a corresponding entry point, and the auxiliary procedure is performed for that entry point. After completing the auxiliary procedure for that entry point, the pose of the auxiliary indicator 244 is adjusted to indicate the next entry point, and the auxiliary procedure is performed for the next entry point. This process is repeated until auxiliary procedures for all entry points are completed. Then, the scanning table 242 is moved to a fourth position corresponding to a certain entry point, and the surgical procedure is performed for the certain entry point. After completing the surgical procedure for the certain entry point, the scanning table 242 is moved to a fourth position corresponding to the next entry point, and the surgical procedure is performed for the next entry point. This process is repeated until surgical procedures for all entry points are completed.

In some embodiments, when performing multiple auxiliary procedures, the scanning table 242 may be moved to the third position. A certain entry point is selected, and based on the location of the certain entry point, the auxiliary indicator 244 is adjusted to a corresponding pose to indicate the certain entry point. After performing one auxiliary procedure for the certain entry point, the scanning table 242 is moved to a fourth position corresponding to the certain entry point to perform the surgical procedure. After completing the surgical procedure for the certain entry point, the scanning table 242 is moved back to the third position, and based on the location of the next target entry point, the auxiliary indicator 244 is adjusted to a corresponding pose to indicate the next target entry point. After performing one auxiliary procedure for the next target entry point, the scanning table 242 is moved to a fourth position corresponding to the next target entry point to perform the surgical procedure. This process is repeated until surgical procedures for all entry points are completed.

In some embodiments, each entry point corresponds to a target point, and one target point may correspond to one or more entry points. For example, multiple target points may include a first target point, a second target point, ..., an nth target point, and multiple entry points may include a first entry point, a second entry point, ..., an mth entry point, where m ≥ n. For example, the first entry point may correspond to the first target point, the second entry point may correspond to the second target point, ..., the (n-1)th entry point may correspond to the (n-1)th target point, and the nth through mth entry points may all correspond to the nth target point. In some embodiments, the number of fourth positions may be n. Each entry point may correspond to a fourth position, and the fourth positions corresponding to different entry points may be the same or different. For example, the first entry point corresponds to the fourth position-1, the second entry point corresponds to the fourth position-2, ..., and the nth through mth entry points all correspond to the fourth position-n. The multiple entry points correspond to a single third position, and each entry point corresponds to a pose of the auxiliary indicator 244. For example, the first entry point corresponds to a first pose of the auxiliary indicator 244, the second entry point corresponds to a second pose of the auxiliary indicator 244, ..., and the mth entry point corresponds to an mth pose of the auxiliary indicator 244. In some embodiments, along the direction of a perpendicular line perpendicular to the imaging scanning plane S of the medical radiation imaging system 220, when the projections of multiple target points onto the perpendicular line overlap, it may be considered that these multiple target points are located on the same scanning layer of the medical radiation imaging system 220. In this case, the fourth positions corresponding to the multiple entry points associated with these multiple target points are the same, and the multiple fourth positions corresponding to these multiple target points are in the same position. In some embodiments, along the direction of the perpendicular line perpendicular to the imaging scanning plane S of the medical radiation imaging system 220, when the projections of multiple target points onto the perpendicular line are offset, it may be considered that these multiple target points are located on different scanning layers of the medical radiation imaging system 220. In this case, the fourth positions corresponding to the multiple entry points associated with these target points are different, and the multiple fourth positions corresponding to these target points are different positions. For example, after the scanning table 242 is moved to the third position, the auxiliary indicator 244 is adjusted to the first pose to indicate the first entry point, and the user may perform an auxiliary procedure (e.g., local anesthesia) on the first entry point according to the indication. After completing the auxiliary procedure, the scanning table 242 may be moved to the fourth position-1 corresponding to the first target point associated with the first entry point to perform the surgical procedure (e.g., puncture) on the first entry point. This process is repeated for other entry points until surgical procedures for all entry points are completed.

In some embodiments, because the auxiliary procedure has a certain effective area, multiple entry points may also undergo only one auxiliary procedure. For example, for the multiple entry points, an auxiliary procedure may be performed only once at the center point of the distribution of the multiple entry points. Based on the location of the center point, a single corresponding pose of the auxiliary indicator 244 may be determined. The scanning table 242 is moved to the third position, the auxiliary indicator 244 is adjusted to the single corresponding pose of the auxiliary indicator 244 to indicate the center point, and the auxiliary procedure is performed on the center point according to the indication. After the auxiliary procedure is completed, the scanning table 242 is moved to the fourth position corresponding to the target point associated with a certain entry point, and the surgical procedure is performed for the certain entry point. After completing the surgical procedure for the certain entry point, the scanning table 242 is moved to a fourth position corresponding to the next entry point, and the surgical procedure is performed for the next entry point. This process is repeated until surgical procedures for all entry points are completed.

In some embodiments, the processor is configured to: (1) in response to a start signal, perform spatial registration on the interventional medical system 200; (2) in response to completion of the spatial registration, control the scanning table 242 to move into the imaging area of the medical radiation imaging system 220 to a second position, the second position being the position of the scanning table 242 when the medical radiation imaging system 220 scans a patient; (3) in response to completion of scanning the patient by the medical radiation imaging system 220, control the scanning table 242 to move to an execution position, the patient undergoing a procedure at the execution position. In some embodiments, the processor may be further configured to: (4) in response to completion of all procedures, control the scanning table 242 to move to a fifth position, the fifth position being outside the imaging area of the medical radiation imaging system 220.

In some embodiments, the aforementioned procedures may include an auxiliary procedure, and the execution position includes a third position. In this case, step (3) may include: in response to acquiring a signal indicating completion of scanning the patient by the medical radiation imaging system 220, controlling the scanning table 242 to move to the third position, the third position being the position of the scanning table 242 when the interventional medical system 200 performs the auxiliary procedure on the patient, and the third position being outside the imaging area of the medical radiation imaging system 220; and in response to the scanning table 242 being at the third position, controlling the auxiliary indicator 244 to indicate the auxiliary procedure. Correspondingly, step (4) may include: in response to completion of all auxiliary procedures, controlling the scanning table 242 to move to the fifth position, the fifth position being outside the imaging area of the medical radiation imaging system 220. In some embodiments, the fifth position may be the same as or different from the third position. When the fifth position is the same as the third position, the scanning table 242 may remain stationary after all auxiliary procedures are completed, and step (4) may be omitted. When the fifth position is different from the third position, step (4) may be executed after all auxiliary procedures are completed, moving the scanning table 242 from the third position to the fifth position.

In some embodiments, the aforementioned procedures may include one or more surgical procedures, and the execution position includes one or more fourth positions. In this case, step (3) may include: in response to acquiring a signal indicating completion of scanning the patient by the medical radiation imaging system 220, controlling the scanning table 242 to move to the one or more fourth positions, the fourth positions being positions of the scanning table 242 when the patient undergoes the surgical procedures; and in response to the scanning table 242 moving to the one or more fourth positions, controlling the end effector 2412 to perform the surgical procedures. Correspondingly, step (4) may include: in response to completion of all surgical procedures, controlling the scanning table 242 to move to the fifth position.

In some embodiments, the aforementioned procedures includes one or more auxiliary procedures and one or more surgical procedures, and the execution position includes a third position and one or more fourth positions. In this case, step (3) may include: in response to acquiring a signal indicating completion of scanning the patient by the medical radiation imaging system 220, controlling the scanning table 242 to move to the third position; in response to the scanning table 242 moving to the third position, controlling the auxiliary indicator 244 to indicate the auxiliary procedure; and in response to completion of the auxiliary procedure, controlling the scanning table 242 to move to a fourth position. Correspondingly, step (4) may include: in response to completion of all surgical procedures, controlling the scanning table 242 to move to the fifth position.

In some embodiments, after completing a portion of the surgical procedures, it may be necessary to move the scanning table 242 out of the medical radiation imaging system 220 for an intermediate procedure (e.g., checking the patient, operating the medical radiation imaging system 220, etc.) or for an intermission, before resuming the surgical procedures. In some embodiments, when the aforementioned procedures at least includes one or more surgical procedures and the execution position at least includes one or more fourth positions, the processor is further configured to: in response to completion of a portion of the one or more surgical procedures when the scanning table 242 is at one of the fourth positions, and the one or more surgical procedure not being fully completed, control the scanning table 242 to move to a fifth position; and in response to a continue signal, control the scanning table 242 to move to another fourth position, and control the end effector 2412 to continue performing the one or more surgical procedures. The continue signal may be input by the user or output by the processor after a preset condition is met (e.g., the intermediate procedure is completed, the intermission is over, or the scanning table 242 has been at the fifth position for a preset duration).

In some embodiments, the processor may be further configured to: after the scanning table 242 is moved to one of the fourth positions, in response to completion of a portion of the surgical procedure when the scanning table 242 is at that fourth position, and the surgical procedure not being fully completed, control the scanning table 242 to move to the third position.

In some embodiments, the processor may be further configured to: after the scanning table 242 is moved to one of the fourth positions, in response to completion of a portion of the one or more surgical procedures when the scanning table 242 is at that fourth position, and the one or more surgical procedures not being fully completed, control the scanning table 242 to move to another fourth position.

In some embodiments, the processor may be further configured to: in response to a start signal, control the surgical execution arm 2411 to install the execution mount 24121; and in response to completion of installation of the execution mount 24121, perform spatial registration on the interventional medical system 200.

In some embodiments, the processor may be further configured to: (1) in response to the scanning table 242 moving to one of the fourth positions, control the surgical execution arm 2411 to adjust to an installation pose, wherein the installation pose is a feasible pose for the surgical execution arm 2411 to avoid the patient; (2) in response to the surgical execution arm 2411 adjusting to the installation pose, control the execution mount 24121 to install an execution instrument 24122; (3) in response to completion of installation of the execution instrument 24122, control the surgical execution arm 2411 to adjust to a surgical operation pose; (4) in response to the surgical execution arm 2411 adjusting to the surgical operation pose, control the execution instrument 24122 to perform the surgical procedure.

In some embodiments, the aforementioned step (3) may include: in response to completion of installation of the execution instrument 24122, controlling the surgical execution arm 2411 to adjust to an initial pose; and in response to the surgical execution arm 2411 adjusting to the initial pose, controlling the surgical execution arm 2411 to adjust to the surgical operation pose. In some embodiments, the aforementioned step (4) may include: in response to the surgical execution arm 2411 adjusting to the surgical operation pose, controlling the execution mount 24121 to adjust a pose of the execution instrument 24122; and in response to completion of adjustment of the pose of the execution instrument 24122, controlling the execution instrument 24122 to perform the surgical procedure.

In some embodiments, the instrument confirmation control of the end effector 2412 is configured to output a confirmation signal in response to completion of installation of the execution instrument 24122. The confirmation signal is used to confirm that the execution instrument 24122 on the execution mount 24121 has been installed, facilitating subsequent procedures. The aforementioned step (3) may include: in response to completion of installation of the execution instrument 24122, controlling the instrument confirmation control to output the confirmation signal; and controlling the surgical execution arm 2411 to adjust to the surgical operation pose based on the confirmation signal.

In some embodiments, the processor may be further configured to: in response to completion of all of the one or more surgical procedures, control the surgical execution arm 2411 to adjust to an end pose. The end pose is the same as or different from the initial pose.

In some embodiments, the surgical execution arm 2411 is configured to: (1) install the execution mount 24121 before spatial registration; (2) after the scanning table 242 is moved to one of the fourth positions and before performing the surgical procedure, adjust to an installation pose and install the execution instrument 24122 onto the execution mount 24121, wherein the installation pose is any feasible pose where the surgical execution arm 2411 does not interfere with the patient; (3) after the installation of the execution instrument 24122 is completed, adjust to the surgical operation pose in preparation for the surgical procedure.

In some embodiments, the surgical execution arm 2411 may be further configured to: after the installation of the execution mount 24121 is completed and before spatial registration, adjust the surgical execution arm 2411 so that at least a part of the surgical execution arm 2411 is within the scanning area of the medical radiation imaging system 220, facilitating more precise determination of the pose of the surgical execution arm 2411 later. In some embodiments, the pose of the surgical execution arm 2411 during spatial registration (e.g., the pose when at least part of it is within the scanning area) is its initial pose.

In some embodiments, the surgical execution arm 2411 is adjusted to the installation pose after the auxiliary procedure is completed and before the surgical procedure starts. In some embodiments, during the adjustment to the installation pose, the surgical execution arm 2411 may first be adjusted to the initial pose for positioning, and then to the installation pose. In some embodiments, after the installation of the execution instrument 24122 is completed, during the adjustment from the installation pose to the surgical operation pose of the surgical execution arm 2411, the surgical execution arm 2411 may first be adjusted from the installation pose to the initial pose, and then from the initial pose to the surgical operation pose. This ensures that the surgical execution arm 2411 moves along a preset path (e.g., the executable path), preventing interference with the external environment or the user during movement. In some embodiments, during the movement of the surgical execution arm 2411, the motion enable switch 245 is depressed. In some embodiments, the surgical execution arm 2411 may be controlled to move and adjust automatically based on a relevant command from the processor (e.g., processor 110). Alternatively, the surgical execution arm 2411 may be may be moved and adjusted manually by dragging. It should be noted that the movement mode of the surgical execution arm 2411 (automatic or manual dragging) may be switched. For example, after releasing the motion enable switch 245, the movement mode is switched, and then the motion enable switch 245 is depressed again to control the movement of the surgical execution arm 2411.

In some embodiments, the surgical execution arm 2411 may be further configured to: (4) after adjusting to the surgical operation pose, fine-tune the pose of the execution mount 24121 to adjust the pose of the execution instrument 24122, facilitating a subsequent surgical procedure; (5) control the execution instrument 24122 to perform the subsequent surgical procedure. In some embodiments, the surgical execution arm 2411 may be further configured to: (6) after all surgical procedures are completed, adjust to an end pose, which may be the same as or different from the initial pose.

In some embodiments, the adjustment of the surgical execution arm 2411 and the performance of the surgical procedure by the execution instrument 24122 may also be controlled separately by the master manipulator 232.

In some embodiments, step 930 may further include: controlling the surgical operation assembly to move to the navigation positioning point; and after the surgical operation assembly moves to the navigation positioning point, controlling the surgical operation assembly to perform the surgical procedure based on the surgical mode.

In some embodiments, after the surgical execution arm 2411 reaches the navigation positioning point and adjusts to the surgical operation pose, the user can control the end effector 2412 to perform the surgical procedure on the patient. Different surgical modes correspond to different procedure steps.

In some embodiments, the surgical mode in the surgical information may include a real-time control mode or a breakpoint control mode. The real-time control mode is a real-time visualization control mode, and the breakpoint control mode may be an intermittent control mode. Step 930 may further include, based on the surgical information and the procedure plan, determining an execution instrument 24122, and installing the execution instrument 24122 onto the end effector 2412, and after the installation of the execution instrument 24122 is completed, controlling the surgical execution arm 2411 to drive the end effector 2412 to move to the navigation positioning point.

In some embodiments, based on the surgical information and the procedure plan, the type of execution instrument 24122 may be determined. After determining the execution instrument 24122, the execution instrument 24122 may be installed on the end effector 2412. After the installation of the execution instrument 24122 is completed, the surgical operation assembly may be controlled to move to the navigation positioning point. At the navigation positioning point, the tip of the execution instrument 24122 is at the navigation positioning point. After the installation of the execution instrument 24122 is completed, the user may subsequently control the movement of the surgical operation assembly and the performance of the surgical procedure directly via the active control end 230, without needing to enter the radiology room to install the execution instrument 24122.

In some embodiments, when the surgical mode in the surgical information includes a real-time control mode or a breakpoint control mode, the sub-step "controlling the surgical operation assembly to perform the surgical procedure based on the surgical mode" in step 930 may further include, based on the real-time control mode or the breakpoint control mode, controlling the execution instrument 24122 to perform the surgical procedure via the active control end 230. That is, in the real-time control mode or the breakpoint control mode, the user may remotely control the execution instrument 24122 to perform the surgical procedure via the active control end 230, without needing to enter the radiology room and be near the patient.

In some embodiments, when the surgical mode in the surgical information includes a real-time control mode, the sub-step "controlling the surgical operation assembly to perform the surgical procedure based on the surgical mode" in step 930 may further include, based on the real-time control mode and a real-time image, controlling the execution instrument 24122 to perform the surgical procedure via the master manipulator 232. The real-time image may be obtained by the user operating the exposure switch 223 to cause the medical radiation imaging system 220 to scan the patient in real-time. For details on the exposure switch 223 and the real-time image, please refer to the previous description of the exposure switch 223, which will not be repeated here.

When the surgical mode is the real-time control mode, the operation interface of the execution display 233 may display the real-time image of the patient scanned by the medical radiation imaging system 220. Based on this real-time image, the user may control the surgical execution arm 2411 and the end effector 2412 to perform the surgical procedure via the master manipulator 232, improving surgical precision and reducing difficulty.

When the surgical mode is the breakpoint control mode, the sub-step "controlling the surgical operation assembly to perform the surgical procedure based on the surgical mode" in step 930 may further include, based on the breakpoint control mode and a surgical image, controlling the execution instrument 24122 via the master manipulator 232 to perform a portion of the surgical procedure corresponding to the surgical image. In some embodiments, when the surgical mode in the surgical information includes the breakpoint control mode, the sub-step "controlling the surgical operation assembly to perform the surgical procedure based on the surgical mode" in step 930 may further include, based on the breakpoint control mode, scanning the patient via the medical radiation imaging system 220 to obtain a first surgical image; based on the breakpoint control mode and the first surgical image, controlling the execution instrument 24122 to perform a portion of the surgical procedure corresponding to the first surgical image; after the portion of the surgical procedure is completed, continuing to scan the patient via the medical radiation imaging system 220 to obtain a second surgical image; based on the breakpoint control mode and the second surgical image, controlling the execution instrument 24122 to perform a next portion of the surgical procedure corresponding to the second surgical image; and repeating these steps until the surgical procedure is completed. The portion of the surgical procedure corresponding to the first surgical image refers to the part performed based on that image. In the breakpoint control mode, before executing a certain portion of the surgical procedure, the patient is scanned using the medical radiation imaging system 220 to obtain a surgical image (e.g., the first surgical image, the second surgical image, etc.), and then the certain portion of the surgical procedure is performed based on the surgical image.

When the surgical mode is the breakpoint control mode, the medical radiation imaging system 220 scans the patient who has reached the fourth position to obtain a first surgical image, which may be displayed on the operation interface. Based on this first surgical image, the user may control the execution instrument 24122 of the surgical execution arm 2411, and the end effector 2412 via the master manipulator 232 to perform a portion of the surgical procedure. When the portion of the surgical procedure is completed, the medical radiation imaging system 220 scans the patient again to obtain the second surgical image, which is displayed. Based on the second surgical image, the user controls the execution instrument 24122 via the master manipulator 232 to perform the next portion of the surgical procedure. This process is repeated until the current surgical procedure is completed. In some embodiments, based on the second surgical image, the next portion of the surgical procedure may be adjusted (e.g., the adjusted progress, the operation posture) to improve precision.

For example, when the surgical procedure is a puncture, the execution instrument 24122 may be a puncture needle. Based on the first surgical image, the user may control the puncture needle to penetrate the entry point and enter the patient's body to a certain depth, but the puncture needle may not yet reach the target point corresponding to the lesion at the target depth. After puncturing to the certain depth, the user may control the surgical execution arm 2411 and the end effector 2412 to stop the procedure and scan the patient via the medical radiation imaging system 220 to obtain a second surgical image. Based on the second surgical image, the user may control the puncture needle to continue puncturing to the next depth. After reaching the next depth, the user stops the surgical procedure and obtains a third surgical image via scanning. This process is repeated until the puncture needle reaches the target depth and the target point corresponding to the lesion, at which point the current surgical procedure is considered complete. In some embodiments, based on the second surgical image, if the puncture depth from the previous portion was too deep, the next portion of the procedure may involve withdrawing the needle to reduce the depth, aligning with the preset progress and improving precision. In some embodiments, based on the second surgical image, if the puncture depth from the previous portion of the surgical procedure was too shallow, the next portion of the surgical procedure may involve advancing the needle to increase the depth. In some embodiments, if the operation pose from the previous portion of the surgical procedure deviated (e.g., puncture direction error), the next portion of the surgical procedure may involve adjusting the operation pose of the puncture needle.

In some embodiments, during a surgical procedure on a patient, to improve precision, the patient may need to hold their breath to maintain a contour consistent with the image corresponding to the procedure plan. However, when the surgical mode is the breakpoint control mode, multiple scans are required during the surgical procedure to obtain corresponding surgical images, making it difficult for the patient to hold their breath for a long time. When the user releases the control enable button 2324, the control enable button 2324 stops issuing the second enable information, causing the clamping structure to enter a semi-released state. The semi-released state of the clamping structure allows the execution instrument 24122 a degree of freedom to move relative to the clamping structure while preventing the execution instrument 24122 from detaching. At the same time, because the second enable information enables the control command of the master manipulator 232, releasing the control enable button 2324 means the control command is no longer enabled, the surgical procedure pauses, and the user may input a scan signal to obtain a surgical image via the medical radiation imaging system 220. Thus, during a surgical procedure in the breakpoint control mode, when the user pauses the surgical procedure, releases the control enable button 2324, and inputs a scan signal (e.g., preparing to scan the patient), the patient can breathe. At this time, the execution instrument 24122 moves on the patient's body surface as the contour changes with breathing, while the position of the clamping structure remains unchanged. After the scan is completed and the surgical image is obtained, the user presses the control enable button 2324, which issues the second enable information, enabling the control command of the master manipulator 232 again, and the surgical procedure continues. At this point, the patient may resume holding their breath to keep the contour consistent with the image corresponding to the procedure plan, thereby maintaining procedural precision. In some embodiments, when the patient's contour matches the image, the user may press the control enable button 2324 to adjust the clamping structure to the clamped state, clamping the execution instrument 24122 to continue the procedure.

In some embodiments, when the surgical mode includes both the breakpoint control mode and the real-time control mode, the processor is further configured to switch the surgical mode between the breakpoint control mode and the real-time control mode. Switching surgical modes can enhance surgical flexibility and efficiency. In some embodiments, if the user activates the breakpoint control mode and the surgical process is slow or the patient's condition is complex, switching to the real-time control mode enables real-time scanning and viewing of medical images, improving efficiency. In some embodiments, if the patient has already received a significant radiation dose, switching from the real-time control mode to the breakpoint control mode can reduce further radiation exposure.

In some embodiments, the processor may be configured to switch the surgical mode from the breakpoint control mode to the real-time control mode. The sub-step "controlling the surgical operation assembly to perform the surgical procedure based on the surgical mode" in step 930 may further include, based on the breakpoint control mode, scanning the patient via the medical radiation imaging system 220 to obtain a first surgical image; based on the breakpoint control mode and the first surgical image, controlling the execution instrument 24122 to perform a portion of the surgical procedure corresponding to the first surgical image; after the portion of the surgical procedure is completed, controlling the processor to switch from the breakpoint control mode to the real-time control mode; based on the real-time control mode, scanning the patient in real-time via the medical radiation imaging system 220 to acquire a real-time image of the patient; and based on the real-time control mode and the real-time image, controlling the execution instrument 24122 to continue performing the surgical procedure.

In some embodiments, the processor may be further configured to switch the surgical mode from the real-time control mode to the breakpoint control mode. The sub-step "controlling the surgical operation assembly to perform the surgical procedure based on the surgical mode" in step 930 may further include: based on the real-time control mode, scanning the patient in real-time via the medical radiation imaging system 220 to acquire a real-time image of the patient; based on the real-time control mode and the real-time image, controlling the execution instrument 24122 to perform a portion of the surgical procedure; after that portion of the surgical procedure is completed, controlling the processor to switch from the real-time control mode to the breakpoint control mode; based on the breakpoint control mode, scanning the patient via the medical radiation imaging system 220 to obtain a first surgical image; based on the breakpoint control mode and the first surgical image, controlling the execution instrument 24122 to perform a portion of the surgical procedure corresponding to the first surgical image among the remaining portion of the surgical procedure; after that portion of the surgical procedure is completed, continuing to scan the patient via the medical radiation imaging system 220 to obtain a second surgical image; and based on the breakpoint control mode and the second surgical image, controlling the execution instrument 24122 to perform a next portion of the surgical procedure corresponding to the second surgical image.

It should be noted that the above two examples are merely illustrative of the principle of switching between real-time and breakpoint control modes and are not limiting. In actual scenarios, the surgical mode is not limited to these two examples. For instance, the surgical mode could switch from the real-time control mode to the breakpoint control mode and then back to the real-time control mode again. Alternatively, the surgical mode could switch from the breakpoint control mode to the real-time control mode, then back to the breakpoint control mode, and then back to the real-time control mode again, etc.

In some embodiments, the surgical mode in the surgical information may include a manual control mode. The manual control mode is a mode where the user manually controls the end effector to perform the surgical procedure. In some embodiments, the manual control mode may also be referred to as a navigation positioning mode. In some embodiments, the end effector 2412 used in the real-time control mode or the breakpoint control mode may not include a clamping structure, while the end effector 2412 used in the manual control mode may include a clamping structure and may also be referred to as a positioning end. Step 930 may include, based on the surgical information and the procedure plan, determining an execution instrument 24122, controlling the surgical execution arm 2411 to drive the end effector 2412 to move to the navigation positioning point, and installing the execution instrument 24122 onto the end effector 2412.

In some embodiments, based on the surgical information and the procedure plan, the type of the execution instrument 24122 may be determined. In the manual control mode, the user needs to enter the radiology room to directly control the end effector 2412 and the execution instrument 24122. In some embodiments, the user may first control the surgical execution arm 2411 via the active control end 230 to drive the end effector 2412 to move to the navigation positioning point, avoiding precision loss from manually moving the surgical operation assembly. In some embodiments, the user may also operate the motion enable switch 245 to manually control the surgical execution arm 2411 to drive the end effector 2412 to the navigation positioning point. After the end effector 2412 is moved to the navigation positioning point, the execution instrument 24122 may be installed onto the end effector 2412 for subsequent manual control by the user.

In some embodiments, when the surgical mode includes the manual control mode, the processor may be further configured to, based on the manual control mode, manually control the surgical execution system 241 to perform the surgical procedure.

When the surgical mode is the manual control mode, since the surgical execution system 241 is at the surgical operation pose, the user may directly and manually control the end effector 2412 to perform the procedure based on its current pose. In some embodiments, the user can manually operate the execution mount 24121, and the execution instrument 24122 is driven by the drive mechanism to perform the surgical procedure. For example, when the surgical procedure is a puncture, the execution instrument 24122 may be a puncture needle, and the user may manually operate the execution mount 24121 to drive the puncture needle to a preset depth, completing the surgical procedure.

It should be noted that when the surgical mode is the real-time control mode or the breakpoint control mode, the surgical procedure performed by the surgical execution system 241 (the surgical execution arm 2411 and the end effector 2412) requires the imaging of the patient by the medical radiation imaging system 220. That is, when the scanning table 242 is at the fourth position, the patient's target point may be located within the scanning area of the medical radiation imaging system 220. When the surgical mode is the manual control mode, during the surgical procedure performed by the surgical execution system 241, the imaging of the patient by the medical radiation imaging system 220 is not required. That is, when the scanning table 242 is at the fourth position, the patient's target point may be located outside the scanning area, reducing obstruction for the surgical execution system 241 and making the adjustment of the surgical execution system 241 more flexible.

In some embodiments, when multiple surgical procedures are required, each surgical procedure corresponds to a surgical operation pose, i.e., each surgical procedure corresponds to a navigation positioning point. In some embodiments, when the execution instrument 24122 does not need to be changed, after completing the current surgical procedure, the user may control the surgical execution system 241 to directly adjust to the surgical operation pose corresponding to the next surgical procedure.

In some embodiments, when the execution instrument 24122 needs to be changed, and the execution instrument 24122 must remain indwelling in the patient, after completing the current surgical procedure, the user may control the surgical execution arm 2411 to release the execution instrument 24122 via the release button 2321 on the master manipulator 232. After release, the user may control the surgical execution arm 2411 to adjust to the installation pose to install a new execution instrument 24122 corresponding to the next surgical procedure. After installation, the user may control the surgical execution arm 2411 to adjust to the navigation positioning point, and the surgical operation pose corresponding to the next surgical procedure. The execution instrument 24122 left indwelling in the patient may be removed by the user after all surgical procedures are completed, based on the actual situation (e.g., after diagnosis of the patient's condition).

In some embodiments, when the execution instrument 24122 needs to be changed but does not need to remain indwelling in the patient, after completing the current surgical procedure, the user may control the surgical execution arm 2411 to adjust to the installation pose to change the execution instrument 24122. After replacement, the user may control the surgical execution arm 2411 to adjust to the navigation positioning point and the surgical operation pose corresponding to the next surgical procedure.

When multiple surgical procedures are required and the surgical mode is the real-time control mode or the breakpoint control mode, the sub-step "controlling the surgical operation assembly to perform the surgical procedure based on the surgical mode" in step 930 may further include installing an execution instrument 24122 corresponding to the current surgical procedure onto the end effector 2412; controlling the surgical execution arm 2411 to drive the end effector 2412 to move to the navigation positioning point corresponding to the current surgical procedure; controlling the execution instrument 24122 to perform the current surgical procedure based on the real-time control mode or the breakpoint control mode; in response to completion of the current surgical procedure, controlling the surgical execution arm 2411 to move to an installation pose; in response to the surgical execution arm 2411 moving to the installation pose, installing an execution instrument 24122 corresponding to the next surgical procedure onto the end effector 2412; in response to completion of installation of the execution instrument 24122 for the next surgical procedure, controlling the surgical execution arm 2411 to drive the end effector 2412 to move to the navigation positioning point corresponding to the next surgical procedure; controlling the execution instrument 24122 to perform the next surgical procedure based on the real-time control mode or the breakpoint control mode; and repeating these steps until all the multiple surgical procedures are completed.

In some embodiments, when multiple surgical procedures are required and the surgical mode is the real-time control mode or the breakpoint control mode, each of the multiple surgical procedures corresponds to a fourth position, and the multiple fourth positions corresponding to the multiple surgical procedures are within the scanning area of the medical radiation imaging system 220 (e.g., the corresponding target points are at the collimation center line of the imaging scanning plane S). The sub-step "controlling the surgical operation assembly to perform the surgical procedure based on the surgical mode" in step 930 may further include: based on the real-time control mode or the breakpoint control mode, controlling the scanning table 242 to move to a fourth position corresponding to a first surgical procedure. If the fourth position corresponding to the current surgical procedure is the same as the fourth position corresponding to the next surgical procedure, no adjustment of the scanning table's position is needed after the current surgical procedure and before the next surgical procedure. If the fourth position corresponding to the current surgical procedure is different from the fourth position corresponding to the next surgical procedure, the sub-step "controlling the surgical operation assembly to perform the surgical procedure based on the surgical mode" may further include, in response to completion of the current surgical procedure, controlling the scanning table 242 to move to the other fourth position corresponding to the next surgical procedure. In some embodiments, after the current surgical procedure is completed, the surgical execution arm 2411 may first be controlled to move to the installation pose before moving the scanning table 242 to the next fourth position to avoid interference or collision between the patient and the surgical execution arm 2411. Of course, in other embodiments, the surgical execution arm 2411 and the patient could move simultaneously, or the patient could move first followed by the surgical execution arm 2411. In some embodiments, when the fourth positions are the same but the entry points differ for two surgical procedures, the angle of the gantry 224 of the medical radiation imaging system 220 may need to be adjusted after the first surgical procedure and before the second surgical procedure to obtain clear images for guiding the subsequent surgical procedure, improving precision.

When multiple surgical procedures are required, and the surgical mode is the real-time control mode or the breakpoint control mode, the multiple surgical procedures may collectively correspond to the same fourth position, but with different entry points. In some embodiments, multiple entry points may not all be within the scan field of view of the medical radiation imaging system 220 simultaneously. To improve precision, the fourth positions for the multiple procedures may be different, or some of the fourth positions for the multiple procedures may be the same and some of the fourth positions for the multiple procedures may be different. In some embodiments, each surgical procedure corresponds to a fourth position of the scanning table 242. At the fourth position, the target point corresponding to the entry point of the current surgical procedure is located at the collimation center line of the imaging scanning plane S, and the entry point is within the scanning area of the medical radiation imaging system 220. In some embodiments, after completing the current surgical procedure, the scanning table 242 may be moved to the fourth position for the next surgical procedure. After adjusting the position of the scanning table 242, the surgical execution arm 2411 may be controlled to adjust to the corresponding navigation positioning point and the surgical operation pose for the next surgical procedure.

When multiple surgical procedures are required and the surgical mode is the manual control mode, the multiple surgical procedures may collectively correspond to a single fourth position, which is located outside the scanning area of the medical radiation imaging system 220. In this case, the fourth position may be the same as or different from the third position. The sub-step "controlling the surgical operation assembly to perform the surgical procedure based on the surgical mode" in step 930 may further include: moving the scanning table 242 to the corresponding fourth position; controlling the surgical execution arm 2411 to drive the end effector 2412 to move to the navigation positioning point corresponding to the current surgical procedure; installing an execution instrument 24122 corresponding to the current surgical procedure onto the end effector 2412; based on the manual control mode, controlling the surgical execution arm 2411 to drive the end effector 2412 to perform the current surgical procedure; in response to completion of the current surgical procedure, controlling the surgical execution arm 2411 to drive the end effector 2412 to move to the navigation positioning point corresponding to the next surgical procedure; installing an execution instrument 24122 corresponding to the next surgical procedure onto the end effector 2412; based on the manual control mode, controlling the execution instrument 24122 to perform the next surgical procedure; and repeating these steps until all the multiple surgical procedures are completed. In some embodiments, when the fourth positions for the multiple surgical procedures are all different, or some of the fourth positions for the multiple surgical procedures are the same and some of the fourth positions for the multiple surgical procedures are different, the handling of same or different fourth positions between consecutive surgical procedures may be referred to in the previous description and will not be repeated here.

Since the manual control mode does not require imaging via the medical radiation imaging system 220 during the surgical procedure, the patient's position may remain unchanged after the initial positioning during multiple surgical procedures, requiring only replacement of the execution instrument 24122.

In some embodiments, when multiple surgical procedures are required and the surgical mode is the manual control mode, the sub-step "controlling the surgical operation assembly to perform the surgical procedure based on the surgical mode" in step 930 may further include: (1) after all or a portion of the current surgical procedure is completed, controlling the surgical execution arm 2411 to move to an installation pose; (2) moving the patient to a second position, and acquiring a new medical image of the patient via the medical radiation imaging system 220; (3) determining a new procedure plan for the remaining surgical procedures based on the new medical image and the procedure plan for the remaining surgical procedures; and (4) completing the remaining surgical procedures based on the new procedure plan. In some embodiments, when the surgical execution arm 2411 does not interfere with the patient and the scanning table 242, the aforementioned step (1) may be omitted.

In some embodiments, when the surgical mode is the manual control mode, the user may scan and image the patient using the medical radiation imaging system 220 during the surgical procedure to adjust the subsequent procedure plan, improving surgical precision.

In some embodiments, when the current surgical procedure in step (1) is fully completed, step (4) may include: based on the new procedure plan, manually controlling the execution instrument 24122 to perform the remaining surgical procedures. In other embodiments, when the current surgical procedure in step (1) is partially completed, step (4) may include: based on the new procedure plan, manually completing the uncompleted portion of the current surgical procedure; and after the current procedure is completed, manually controlling the execution instrument 24122 to perform the remaining surgical procedures.

Specifically, after completing a portion of the multiple surgical procedures (e.g., after fully completing the current surgical procedure, or after partially completing the current surgical procedure), the user may adjust the surgical execution arm 2411 to the installation pose to prevent the surgical execution arm 2411 from interfering with the movement of the patient and the scanning table 242. The scanning table 242 is then moved to a scanning position, bringing the patient into the scanning area of the medical radiation imaging system 220 for scanning to obtain a new medical image. By comparing the new medical image with the original medical image, a new procedure plan (e.g., entry point, target point, surgical operation pose, etc.) for the remaining surgical procedures may be determined based on the original procedure plan for the remaining surgical procedures. After determining the new procedure plan, the remaining surgical procedures may be completed based on the new procedure plan. For example, the scanning table 242 is moved to the fourth position, and the surgical execution arm 2411 is adjusted to the corresponding navigation positioning point and the operation pose for the next surgical procedure. In some embodiments, if a portion of the current surgical procedure is completed and a remaining portion of the current surgical procedure needs to be continued when the medical radiation imaging system 220 performs a scan, the user may directly move the scanning table 242 back to the fourth position for the uncompleted portion of the current surgical procedure and adjust the surgical execution arm 2411 with the same execution instrument 24122 still attached to the corresponding surgical operation pose to continue perform the uncompleted portion of the current surgical procedure. In some embodiments, if a portion of the current surgical procedure is completed and a remaining portion of the current surgical procedure needs to be continued when the medical radiation imaging system 220 performs a scan, the user may manually operate the execution instrument 24122 based on the new procedure plan to complete the uncompleted portion of the current surgical procedure. In some embodiments, after the current surgical procedure is completed, the user may manually control the execution instrument 24122 to perform the remaining surgical procedures based on the manual control mode and the new procedure plan. In some embodiments, if the current surgical procedure is fully completed when the medical radiation imaging system 220 performs a scan, the user can manually control the execution instrument 24122 to perform the remaining surgical procedures based on the new procedure plan.

In some embodiments, after all the multiple surgical procedures are completed, the user may control the surgical execution arm 2411 to adjust to the initial pose.

In some embodiments, the operation interface is configured to achieve at least one of the following: displaying and processing relevant information and surgical information of a patient; displaying and inputting a procedure plan; and displaying prompt information. In some embodiments, the operation interface may display the working mode of the master manipulator 232 and the pose of the surgical execution system 241 to improve the precision of the user's control and adjustment of the surgical execution system 241 via the master manipulator 232.

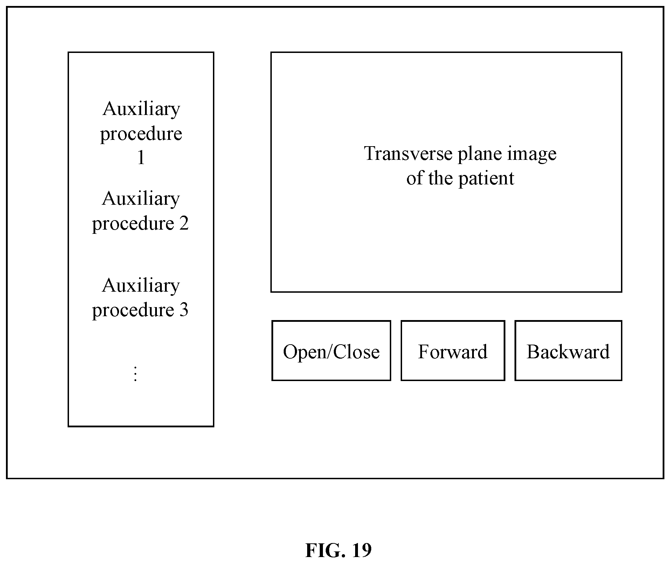

FIG. 19 is a schematic diagram illustrating an operation interface for an auxiliary procedure according to some embodiments of the present disclosure.

In some embodiments, after step 920 is completed and the procedure plan is determined, when the auxiliary procedure is to be performed manually by a user (e.g., medical staff), the user may approach the operation end effector 240 to perform the auxiliary procedure. At this time, the touch display 2414 of the surgical execution system 241 may display an operation interface to guide the user in performing the auxiliary procedure.

In some embodiments, after the procedure plan is determined, when the auxiliary procedure is to be performed by the user controlling the surgical execution arm 2411, the user may be near the active control end 230 and control the surgical execution arm 2411 to perform the auxiliary procedure. At this time, the execution display 233 may display an operation interface to guide the user.

As shown in FIG. 19, in some embodiments, the left area of the operation interface for the auxiliary procedure may include an auxiliary procedure list that displays the auxiliary procedures to be performed. In some embodiments, when the patient has multiple lesions or a large lesion area, multiple surgical procedures may be required, meaning the initial planned surgical procedure may include multiple target points or multiple entry points and may require one or more auxiliary procedures.

In some embodiments, when the scanning table 242 is at the third position, the patient undergoes the auxiliary procedure. In some embodiments, the multiple entry points may correspond to a single third position. After the scanning table 242 is moved to the single third position, the auxiliary indicator 244 may adjust its position and posture to indicate the locations of the multiple entry points, thereby facilitating auxiliary procedures at each entry point. In some embodiments, because the auxiliary procedure has a certain effective area, for the multiple entry points, the auxiliary procedure may be performed only once at the center point of the distribution of the multiple entry points(e.g., the geometric center of a polygon formed by sequentially connecting the outermost entry points). In some embodiments, to ensure the smooth progress of the surgery, for the multiple entry points, an auxiliary procedure may be performed respectively at or near each entry point.

In some embodiments, the auxiliary procedure list may display each entry point and the auxiliary procedure corresponding to each entry point, allowing the user to track the progress of auxiliary procedures via the list.

In some embodiments, the operation interface may further include a transverse plane (horizontal plane) image of the patient. Each entry point may correspond to a transverse plane image of the patient. The transverse plane image of the patient may be obtained from the fused image of the initial diagnostic image and the medical image. In some embodiments, relevant images may also be processed on the operation interface to simulate and display the operation path for the auxiliary procedure.

In some embodiments, the operation interface may further include control options, such as open/close options for controlling the display of relevant images, or forward/backward options for switching the currently displayed entry point and a relevant image corresponding to the currently displayed entry point. When the user switches an entry point using the forward/backward options, the auxiliary indicator 244 may be correspondingly controlled to indicate the relevant entry point, allowing the auxiliary procedure to be performed for that entry point. When all auxiliary procedures are completed, the operation interface may issue a prompt to remind the user to proceed to the next operation (e.g., surgical preparation, surgical procedure, etc.).

In some embodiments, when the scanning table 242 is at a fourth position, the patient undergoes the surgical procedure. The data for the fourth position may be automatically acquired by the execution host 231 or manually input by the user via the operation interface. In some embodiments, when the scanning table 242 is at the fourth position, the target point corresponding to the entry point may be located at the collimation center line of the imaging scanning plane S of the medical radiation imaging system 220 or outside the scanning area of the medical radiation imaging system 220. In some embodiments, when the patient has multiple lesions or a large lesion area, multiple surgical procedures may be required, meaning the initial planned surgical pose may include multiple target points or multiple entry points. Each entry point undergoes one surgical procedure and has a corresponding target point, and each entry point corresponds to a fourth position.

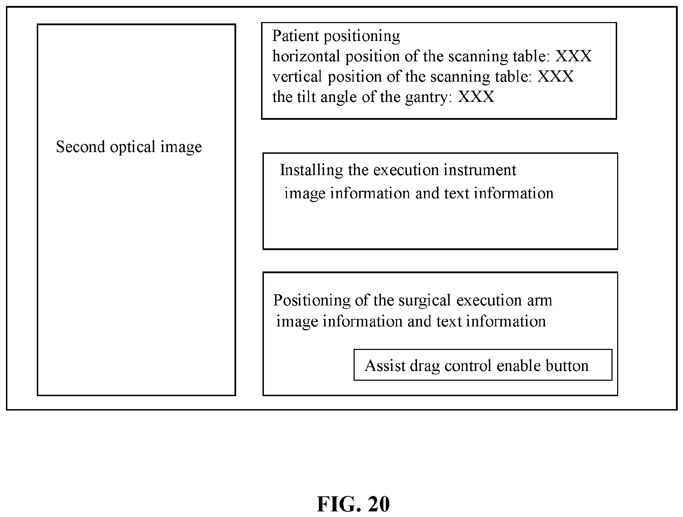

FIG. 20 is a schematic diagram illustrating an operation interface for surgical preparation according to some embodiments of the present disclosure.

In some embodiments, during the execution of step 930, when the scanning table 242 is moved to one of the fourth positions, the operation interface on the touch display 2414 or the execution display 233 may display prompt information to remind the user to perform surgical preparation. In some embodiments, surgical preparation may include patient positioning, installing the execution instrument 24122, etc.

Referring to FIG. 20, in some embodiments, the operation interface may display the field of view of the surgical field camera 260 in real-time, allowing the user to monitor the patient in real-time and promptly stop relevant procedures if an anomaly is observed, further enhancing surgical safety.

In some embodiments, the operation interface may display information related to patient positioning, such as the horizontal position of the scanning table 242 within the plane perpendicular to the height direction of the gantry 224, the vertical position of the scanning table 242 relative to the gantry's height direction, and the tilt angle of the gantry 224.

In some embodiments, the operation interface may display information related to installing the execution instrument 24122, such as image information and text information. The image information may include a real-time image of the surgical execution arm 2411, and the text information may include pose information of the surgical execution arm 2411. The image information may be obtained at least via the medical radiation imaging system 220, the optical imaging system 250, or the surgical field camera 260, and the text information may be determined based on the image information. In some embodiments, the text information may also include information about the execution instrument 24122, such as the type and model of the execution instrument 24122.

When the scanning table 242 is moved to the fourth position, the user may, based on the text information on the operation interface, unlock the surgical execution arm 2411, operate the motion enable switch 245, and control the surgical execution arm 2411 to move from its initial pose to the installation pose. The text information may be preset or input in real-time via the operation interface. In some embodiments, the text information corresponding to installing the execution instrument 24122 may include information about the installation pose, which is any feasible pose where the surgical execution arm 2411 does not interfere with the patient. In some embodiments, the installation pose may be the same as or different from the initial pose. After the surgical execution arm 2411 is moved to the installation pose, the user may install, remove, or replace the execution instrument 24122 based on the relevant information, surgical information (e.g., surgical mode), and the actual situation. For example, when the surgical mode is the real-time control mode or the breakpoint control mode, the execution instrument 24122 may be installed onto the surgical execution arm 2411 (or execution mount 24121) before the surgical operation assembly moves to the navigation positioning point. When the surgical mode is the manual control mode, the execution instrument 24122 may be installed onto the execution mount 24121 at the distal end of the surgical execution arm 2411 after the surgical operation assembly moves to the navigation positioning point, allowing the user to manually control the execution instrument 24122 via the manual control 24123 on the execution mount 24121. For details, please refer to the subsequent description of different surgical modes.

In some embodiments, the operation interface may also display information related to the positioning of the surgical execution arm 2411, such as image information and text information. The image information may include a real-time image of the surgical execution arm 2411, and the text information may include pose information of the surgical execution arm 2411. The image information may be obtained at least via the medical radiation imaging system 220, the optical imaging system 250, or the surgical field camera 260, and the text information may be determined based on the image information. In some embodiments, the text information may also include information about the surgical operation pose.

After the installation of the execution instrument 24122 is completed, the user may, based on the corresponding text information on the operation interface, operate the motion enable switch 245 to control the surgical execution arm 2411 to adjust to the surgical operation pose. The text information may be preset or input in real-time via the operation interface.

In some embodiments, the surgical execution arm 2411 may first be adjusted from the installation pose to the initial pose, and then from the initial pose to the surgical operation pose. In some embodiments, if the execution instrument 24122 of the surgical execution arm 2411 does not require installation, removal, replacement, etc., the user may directly control the surgical execution arm 2411 to move to the navigation positioning point and adjust to the surgical operation pose when the scanning table 242 is moved to the fourth position.

During the adjustment of the surgical execution arm 2411, via the field of view of the surgical field camera 260 on the operation interface, visual information of the surgical execution arm 2411 and the patient may be displayed in real-time. If an unexpected situation occurs (e.g., the patient coughs uncontrollably, the surgical execution arm 2411 and the end effector 2412 collide with or compress the patient), the user may promptly release the motion enable switch 245, stopping the movement of the surgical execution arm 2411 to prevent or minimize injury to the patient.

In some embodiments, the operation interface may further include an assist drag control enable button 2324. The assist drag control enable button 2324 has the same function as the motion enable switch 245, which permits or prohibits movement of the surgical execution arm 2411. During the pose adjustment of the surgical execution arm 2411, the user may need to manually assist in dragging the surgical execution arm 241. At this time, the user may release the motion enable switch 245 and press the assist drag control enable button 2324 on the operation interface to permit the movement of the surgical execution arm 241, allowing the user to manually drag the surgical execution arm 241 for adjustment.

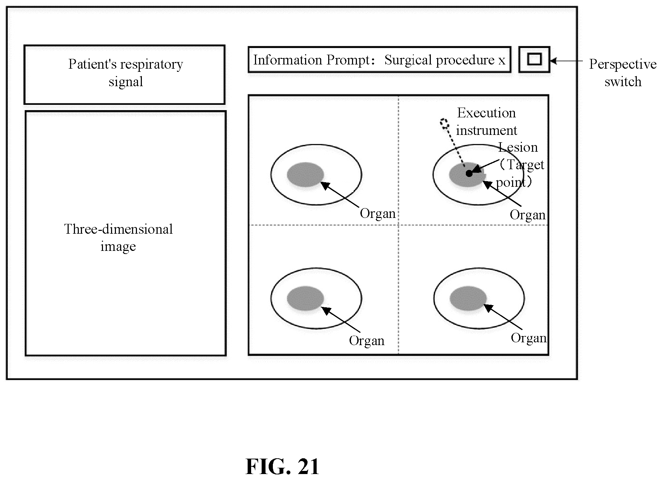

FIG. 21 is a schematic diagram illustrating an operation interface for a manual control mode according to some embodiments of the present disclosure.

As shown in FIG. 21, when performing the procedure on a patient based on the manual control mode, the operation interface may display a three-dimensional image of the patient. This three-dimensional image may be obtained from the fused image of the initial diagnostic image and the medical image, used to monitor and indicate the status of the surgical execution system 241 and the patient, enhancing surgical safety.

In some embodiments, the medical image on the operation interface may also display the initial planned surgical pose of the execution instrument 24122 as a reference. In some embodiments, the three-dimensional image of the patient may display an image of the execution instrument 24122 to show the progress and status of the surgical procedure.

In some embodiments, the operation interface may also display images of the surgical area from different perspectives, facilitating clearer observation of the target point of the patient's lesion. In some embodiments, the operation interface may be provided with a perspective switch button for switching between different perspectives of the images of the surgical area. In some embodiments, the user may also perform operations such as zooming in or rotating the image of the surgical area from a certain perspective via the operation interface.

In some embodiments, the execution host 231 may also process the images of the surgical area to simulate and display the surgical executable path of the end effector 2412 (or execution instrument 24122) at the current surgical operation pose.

In some embodiments, the operation interface may also display the patient's respiratory signal to monitor vital signs and enhance surgical safety.

In some embodiments, the operation interface may also include one or more information prompts. The one or more information prompts may include information related to the surgical procedure. For example, when multiple surgical procedures are required, the one or more information prompts may include the number of the current surgical procedure. As another example, the one or more information prompts may include the current surgical mode.

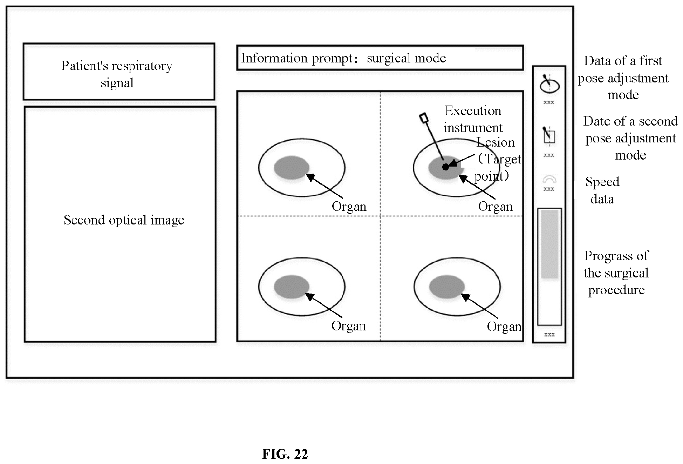

FIG. 22 is a schematic diagram illustrating an operation interface when a master manipulator controls a surgical execution system according to some embodiments of the present disclosure.

Referring to FIG. 22, in some embodiments, when the user controls the surgical execution system via the master manipulator 232, the operation interface may display the field of view of the surgical field camera 260, allowing the user to monitor the patient in real-time and promptly stop relevant procedures if an anomaly is observed, further enhancing surgical safety.

In some embodiments, the operation interface may also display the patient's respiratory signal to monitor vital signs and enhance surgical safety.

In some embodiments, the operation interface may also include one or more information prompts. The one or more information prompts may include information related to the surgical procedure. For example, when multiple surgical procedures are required, the one or more information prompts may include the number of the current surgical procedure. As another example, the one or more information prompts may include the current surgical mode.

In some embodiments, the operation interface may also display patient images from different perspectives, facilitating clearer observation of the target point of a patient's lesion. When the surgical mode is the real-time control mode, the patient images from different perspectives may be obtained based on the real-time images of the patient scanned by the medical radiation imaging system 220. When the surgical mode is the breakpoint control mode, the patient images may be obtained based on different surgical images (e.g., first surgical image, second surgical image, etc.) scanned at different times. In some embodiments, the user may also perform operations such as zooming in or rotating the patient image from a certain perspective via the operation interface.

In some embodiments, the operation interface may also include an information display content. In some embodiments, the information display content may include data of the pose adjustment mode of the master manipulator 232, such as the adjustment angle for the first pose adjustment mode and the adjustment angle for the second pose adjustment mode. In some embodiments, the information display content may include speed control data of the master manipulator 232, such as the movement speed of the master manipulator 232 controlling the surgical execution system 241, the operating speed of the execution instrument 24122, etc.

In some embodiments, the operation interface may also include a surgical procedure progress display, allowing the user to clearly grasp the progress of the current surgical procedure. In some embodiments, when the surgical mode is the real-time control mode, the surgical procedure progress display may show the operating progress of the execution instrument 24122 in real-time. When the surgical mode is the breakpoint control mode, the surgical procedure progress display may show the progress of the execution instrument 24122 each time the medical radiation imaging system scans.

In some embodiments, when all surgical procedures are completed, the operation interface may issue a prompt to remind the user to adjust the navigation support arm 2413 to stow the navigation support arm 2413. After confirming that the navigation support arm 2413 is stowed, the surgery ends, and the scanning table 242 and patient may be moved out of the medical radiation imaging system 220.

In some embodiments, when the interventional medical system 200 performs procedures on multiple patients, for the first patient, the following steps may be performed in sequence: (1) starting the interventional medical system 200; (2) obtaining registration information; (3) acquiring relevant information and surgical information of the patient; (4) determining a procedure plan based on the registration information, the relevant information, and the surgical information; (5) controlling the surgical execution system to perform the procedure based on the registration information, the surgical information, and the procedure plan. For each subsequent patient, only steps (3), (4), and (5) need to be repeated, and steps (1) and (2) do not need to be repeated; there is no need to perform spatial registration again or obtain new registration information. The registration information obtained in step 910 may be used directly, reducing surgical steps, decreasing complexity, and improving efficiency.

The following describes the control process of the processor using an example where the procedure includes an auxiliary procedure and a surgical procedure, and the execution position includes a third position and a fourth position. It should be noted that the cases where the procedure includes only an auxiliary procedure and the execution position includes only the third position, and where the procedure includes only a surgical procedure and the execution position includes only a fourth position, can both be derived by referring to the case where the procedure includes both and the execution position includes both, and therefore will not be repeated.

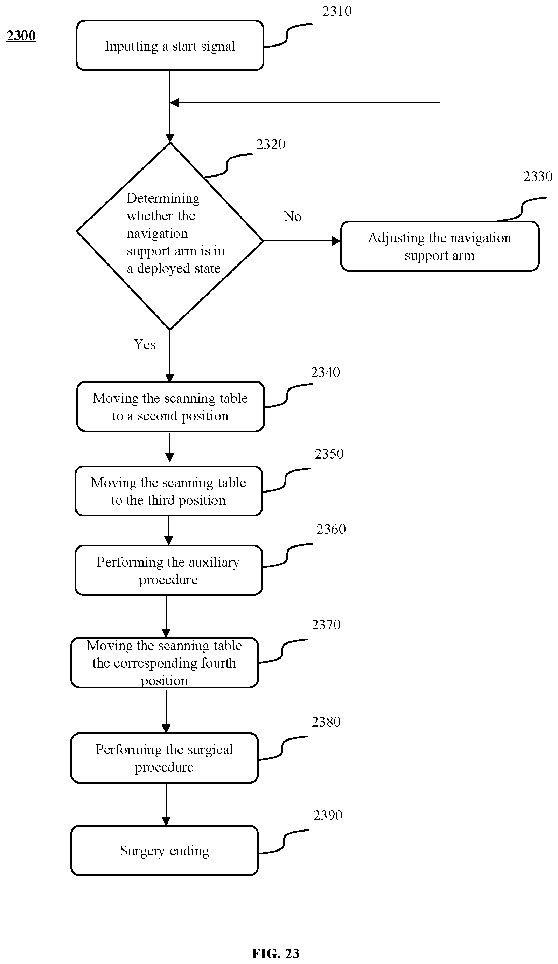

FIG. 23 is an exemplary flowchart illustrating a user controlling an interventional medical system to perform a procedure according to some embodiments of the present disclosure. As shown in FIG. 23, in some embodiments, process 2300 may include steps 2310, 2320, 2330, 2340, 2350, 2360, 2370, 2380, and 2390.

In 2310, a start signal is input.

In some embodiments, the user may input the start signal via an operation interface. In response to the start signal, the interventional medical system 200 enters an operational state, acquires position information of the interventional medical system 200, and performs spatial registration. At this time, the scanning table 242 is at a first position. In some embodiments, the user may input the start signal via touch control, button control, key control, etc. In some embodiments, the operation interface may be provided on the execution host 231 or an input device of the operation end effector 240 (e.g., the touch display 2414), etc.

In 2320, whether the navigation support arm 2413 is in a deployed state is determined.

In some embodiments, step 2320 may be performed by a processor (e.g., the first processor disposed in the active control end 230, the second processor disposed in the operation end effector 240, etc.). After receiving the start signal input by the user, the relevant processor may verify and determine whether the navigation support arm 2413 is in the deployed state. In some embodiments, the state of the navigation support arm 2413 may be determined based on the real-time image or real-time joint parameters, etc., of the navigation support arm 2413. If the navigation support arm 2413 is not in the deployed state, steps 2330 and 2320 are repeated until the navigation support arm 2413 is in the deployed state. If the navigation support arm 2413 is in the deployed state, step 2340 is performed.

In 2330, the navigation support arm 2413 is adjusted.

When it is determined that the navigation support arm 2413 is not in the deployed state, the navigation support arm 2413 needs to be adjusted to the deployed state to avoid obstructing the patient and affecting subsequent imaging quality (e.g., the first optical image, etc.) and subsequent steps (e.g., the auxiliary procedure, etc.). In some embodiments, when it is determined that the navigation support arm 2413 is not in the deployed state, the corresponding processor may issue a prompt signal to remind the user to adjust the navigation support arm 2413. In some embodiments, the prompt signal may be output by the operation interface. In some embodiments, the prompt signal may include, but is not limited to, a text signal, a light signal, a sound signal, a vibration signals, etc.

In 2340, the scanning table 242 is moved to a second position.

In some embodiments, in response to completion of spatial registration, a processor (e.g., the first processor in the active control end 230, the second processor in the operation end effector 240, etc.) may output a first signal. The first signal may be used to control the scanning table 242 to move into the imaging area of the medical radiation imaging system 220 to a second position, so that the medical radiation imaging system 220 can scan the patient to form a medical image. In some embodiments, when the patient's lesion moves into the imaging area of the medical radiation imaging system 220, the scanning table 242 is considered to have moved to the second position. That is, when the scanning table 242 is at the second position, the patient's lesion is displayed in the medical image.

In some embodiments, in response to acquiring a signal indicating that the medical radiation imaging system 220 has completed scanning the patient, a processor (e.g., the first processor in the active control end 230, the second processor in the operation end effector 240, etc.) may output a second signal. The second signal may be used to control the scanning table 242 to move to an execution position, where the patient undergoes a procedure. In some embodiments, the procedure may include an auxiliary procedure and a surgical procedure, and the execution position includes a third position and a fourth position. The third position is the position of the scanning table 242 when the interventional medical system 200 performs the auxiliary procedure on the patient, and the fourth position is an available position of the scanning table 242 when the interventional medical system 200 performs the surgical procedure on the patient. In some embodiments, the interventional medical system 200 may determine a procedure plan and operation information for the surgical execution system 241 based on the patient's medical image, thereby determining the third position and the fourth position of the scanning table 242. When the scanning table 242 is moved to the third position, the patient undergoes the auxiliary procedure. When the scanning table 242 is moved to the fourth position, the patient undergoes the surgical procedure.

In 2350, the scanning table 242 is moved to the third position.

In some embodiments, in response to acquiring the signal indicating that the medical radiation imaging system 220 has completed scanning the patient, a processor (e.g., the first processor in the active control end 230, the second processor in the operation end effector 240, etc.) may output the second signal. The second signal may be used to control the scanning table 242 to move from the second position to the third position. In some embodiments, based on the procedure plan and the operation information of the surgical execution system 241, the target point and the entry point may be determined, thereby determining the third position and the fourth position. The third position is located outside the scanning area of the medical radiation imaging system 220. When the surgical mode includes a real-time control mode or a breakpoint control mode, the fourth position is located within the scanning area of the medical radiation imaging system 220 (e.g., the position of the scanning table 242 when the corresponding target point is at the collimation center line of the imaging scanning plane S). When the surgical mode includes a manual control mode, the fourth position is located outside the scanning area, and the third position and the fourth position may be the same or different.

In some embodiments, to facilitate subsequent surgical procedures, the auxiliary procedure may be performed first on the patient. In some embodiments, the scanning table 242 is moved from the second position to the third position to perform the auxiliary procedure on the entry point at the third position. In some embodiments, an auxiliary procedure point may be located within a circular area with a preset radius centered on the entry point. In some embodiments, the auxiliary procedure point may coincide with the entry point.

In 2360, the auxiliary procedure is performed.

When the scanning table 242 and the patient are moved to the third position, the auxiliary procedure may be performed on the entry point of the patient. In some embodiments, after the scanning table 242 and the patient are moved to the third position, the auxiliary procedure assembly (e.g., auxiliary indicator 244) may adjust its position and posture (e.g., via the navigation support arm 2413 or the surgical execution arm 2411) so that the auxiliary indicator 244 points to the target entry point (i.e., the entry point for the auxiliary procedure and the entry point for the surgical procedure), thereby indicating the location of the target entry point. In some embodiments, the user may confirm to turn on the auxiliary indicator 244 via the operation interface before the auxiliary procedure starts or confirm to turn the auxiliary indicator 244 off after the auxiliary procedure is completed. The relevant processor turns the auxiliary indicator 244 on or off according to the user's input, so that the auxiliary indicator 244 indicates the target entry point on the patient's body during the auxiliary procedure, facilitating its execution.

In 2370, the scanning table 242 is moved to the corresponding fourth position.

In some embodiments, in response to acquiring a signal indicating completion of the auxiliary procedure, a processor (e.g., the first processor in the active control end 230, the second processor in the operation end effector 240, etc.) may output a third signal. The third signal may be used to control the scanning table 242 to move from the third position to the fourth position, facilitating a subsequent surgical procedure. In some embodiments, the fourth position is related to the surgical mode. When the surgical mode includes a real-time control mode or a breakpoint control mode, the fourth position is within the scanning area of the medical radiation imaging system 220 (e.g., the position of the scanning table 242 when the corresponding target point is at the collimation center line of the imaging scanning plane S). When the surgical mode includes a manual control mode, the fourth position is outside the scanning area, and the third position and the fourth position may be the same or different. In some embodiments, the data for the fourth position may be automatically acquired or manually input by the user via the operation interface. In some embodiments, after receiving the data for the fourth position, the imaging host 221 may control the scanning table 242 to move to the fourth position.

In 2380, the surgical procedure is performed.

In some embodiments, the user may control the execution host 231 and the surgical execution system 241 to perform the surgical procedure on the corresponding entry point. In some embodiments, this entry point corresponds to the entry point at the third position in step 2350.

In some embodiments, after moving the scanning table 242 to the fourth position, a processor (e.g., the first processor in the active control end 230, the second processor in the operation end effector 240, etc.) may unlock the surgical execution arm 2411.

In some embodiments, after the surgical execution arm 2411 is unlocked, the user may, based on first prompt information on the operation interface, operate the motion enable switch 245. The processor may then control the surgical execution arm 2411 to move from an initial pose to an installation pose. The first prompt information may be preset or input in real-time. The first prompt information may include information related to the installation pose.

After the surgical execution arm 2411 is moved to the installation pose, the user may install, remove, replace, etc., the end effector 2412 (e.g., execution instrument 24122) based on the actual situation. In some embodiments, the operation interface may display information about the end effector 2412 (e.g., the type, the model, etc.).

After the installation of the end effector 2412 (e.g., execution instrument 24122) is completed, the user may, based on second prompt information on the operation interface, operate the motion enable switch 245. The relevant processor may then control the surgical execution arm 2411 to adjust to an operation pose. The second prompt information may be preset or input in real-time. It may include information related to the surgical operation pose. In some embodiments, the surgical execution arm 2411 may first be adjusted from the installation pose to the initial pose, and then from the initial pose to the surgical operation pose. In some embodiments, if the end effector 2412 of the surgical execution arm 2411 does not require installation, removal, replacement, etc., the user can directly control the surgical execution arm 2411 to adjust to the surgical operation pose when the scanning table 242 is moved to the fourth position. In some embodiments, the surgical operation pose may be determined based on the target point and entry point corresponding to that fourth position.

In some embodiments, after the surgical execution arm 2411 is adjusted to the corresponding surgical operation pose, the user may move to the active control end 230 to avoid subsequent radiation exposure.

In some embodiments, the user may operate the control enable button 2324 on the master manipulator 232 to unlock the control of the master manipulator 232 over the surgical execution system 241.

In some embodiments, the user may operate the exposure switch 223 to cause the medical radiation imaging system 220 to scan the patient and obtain a medical image, the imaging host 221 or the execution host 231 may display the medical image in real-time of the patient. Based on the real-time medical image, the user may control the surgical execution arm 2411 via the master manipulator 232 to adjust the pose (angle) of the end effector 2412. When the end effector 2412 is at a preset pose (e.g., the end effector 2412 pointing at the entry point), the user may release the control enable button 2324, locking the master manipulator's control over the surgical execution system 241, thereby locking the pose of the end effector 2412. Once the end effector 2412 is at the preset pose, the relevant processor may control the end effector 2412 to perform the corresponding surgical procedure (e.g., puncture, etc.). After the surgical procedure is completed, the relevant processor may output a corresponding signal to the operation interface, prompting the user that the surgical procedure is done.

After the surgical procedure is completed, the user may operate the motion enable switch 245, and the relevant processor may control the surgical execution arm 2411 to adjust to another pose and the surgical execution arm 2411 is stowed and locked. The another pose may be the same as or different from the initial pose.

In some embodiments, when the patient has multiple lesions or a large lesion area, multiple surgical procedures may be required. That is, the surgical operation pose may include multiple target points or multiple entry points. Each entry point may correspond to a fourth position, and the multiple entry points may correspond to a single third position.

In some embodiments, when performing the multiple surgical procedures on a patient, an auxiliary procedure may be performed for each surgical procedure. In some embodiments, after completing the auxiliary procedure and the surgical procedure for one entry point, the auxiliary procedure and the surgical procedure for the next entry point may be performed. That is, steps 2350, 2360, 2370, and 2380 may be repeated sequentially until surgical procedures for all entry points are completed. In this case, there may be multiple fourth positions, and each surgical procedure corresponds to one fourth position. In some embodiments, the third signal may include multiple third sub-signals, each of the multiple third sub-signals corresponds to a fourth position. For example, third sub-signal-1 corresponds to fourth position-1, third sub-signal-2 corresponds to fourth position-2, ..., third sub-signal-n corresponds to fourth position-n. In some embodiments, after the scanning table 242 is moved to a fourth position, in response to the completion of step 2380, i.e., in response to completion of the surgical procedure at that fourth position, and if not all surgical procedures are completed, a processor (e.g., the first processor in the active control end 230, the second processor in the operation end effector 240, etc.) may output a fifth signal. The fifth signal is used to control the scanning table 242 to move from that fourth position back to the third position. That is, in response to the fifth signal, step 2350 is executed to control the scanning table 242 to move from the current fourth position (e.g., fourth position-1) to the third position. In some embodiments, when the scanning table 242 is moved to the third position, the auxiliary indicator 244 is controlled to indicate the next entry point. In some embodiments, the location information for the next entry point may be determined by the processor (e.g., the first processor in the active control end 230, the second processor in the operation end effector 240, etc.) or selected by the user. In some embodiments, the location information for the next entry point may also be included in the fifth signal. For example, the fifth signal may include multiple fifth sub-signals. The number of the multiple fifth sub-signals is one less than the number of the auxiliary procedures. Each auxiliary procedure except the one auxiliary procedure corresponding to the second signal corresponds to a fifth sub-signal. For example, the first entry point may correspond to the second signal; the second entry point may correspond to fifth sub-signal-1; the third entry point may correspond to fifth sub-signal-2; ...; the mth entry point may correspond to fifth sub-signal-(m-1). That is, the second signal, the first entry point, third sub-signal-1, and fourth position-1 (first target point) correspond to each other; fifth sub-signal-1, the second entry point, third sub-signal-2, and fourth position-2 (second target point) correspond to each other; ...; fifth sub-signal-(n-1), nth entry point, third sub-signal-n, and fourth position-n (nth target point) correspond to each other. Based on the indication of the auxiliary indicator 244, step 2360 is executed to perform the auxiliary procedure on the selected entry point (e.g., the second entry point). In some embodiments, in response to acquiring a signal indicating completion of an auxiliary procedure, the processor may output the third sub-signal (e.g., third sub-signal-2) corresponding to the auxiliary procedure. In response to this third sub-signal, step 2370 is executed, controlling the scanning table 242 to move from the third position to a fourth position (e.g., fourth position-2) corresponding to the auxiliary procedure. When the scanning table 242 is at that fourth position, step 2380 is executed, and the surgical execution system 241 performs a surgical procedure on the entry point (e.g., the second entry point) corresponding to the aforementioned third sub-signal. This process is repeated until all multiple surgical procedures are completed.

In some embodiments, when performing multiple surgical procedures on a patient, an auxiliary procedure may be performed for each surgical procedure. In some embodiments, the auxiliary procedures for all entry points may be completed first, and then the surgical procedure for each entry point may be performed separately. That is, step 2360 may be repeated until the auxiliary procedures for all entry points are completed, and then steps 2370 and 2380 may be repeated sequentially until the surgical procedures for all entry points are completed. In this case, there may be multiple fourth positions, and each surgical procedure corresponds to one fourth position. In some embodiments, when the scanning table 242 is at the third position, the processor (e.g., the first processor in the active control end 230, the second processor in the operation end effector 240, etc.) may acquire the position signals of all entry points and the corresponding pose information of the auxiliary indicator 244, thereby controlling the auxiliary indicator 244 to adjust its pose sequentially to indicate the multiple entry points in turn, thus performing auxiliary procedures on the multiple entry points sequentially. In some embodiments, in response to acquiring a signal indicating completion of all auxiliary procedures, the processor may output the third signal, and based on the third signal, control the scanning table 242 to move to one fourth position (e.g., fourth position-1). After the scanning table 242 is moved to that fourth position, step 2380 is executed, and the surgical execution system 241 performs a surgical procedure on the entry point (e.g., the first entry point) corresponding to the third signal. In some embodiments, in response to completion of the surgical procedure when the scanning table 242 is at any fourth position (e.g., fourth position-1), and if not all surgical procedures are completed, the processor may output a sixth signal. The sixth signal is used to control the scanning table 242 to move to another fourth position. In some embodiments, the sixth signal may include multiple sixth sub-signals. The number of sixth sub-signals is one less than the number of surgical procedures. Each surgical procedure, except the one surgical procedure corresponding to the third signal, corresponds to a sixth sub-signal. That is, the third signal, first entry point, and fourth position-1 (first target point) correspond to each other; sixth sub-signal-1, second entry point, and fourth position-2 (second target point) correspond to each other; ...; sixth sub-signal-(n-1), nth entry point, and fourth position-n (nth target point) correspond to each other. In some embodiments, in response to a sixth sub-signal (e.g., sixth sub-signal-1), step 2370 is executed to control the scanning table 242 to move from the current fourth position (e.g., fourth position-1) to another fourth position (e.g., fourth position-2). When the scanning table 242 is at the another fourth position, step 2380 is executed, and the surgical execution system 241 performs a surgical procedure on the entry point (e.g., the second entry point) corresponding to the aforementioned sixth sub-signal. This process is repeated until all multiple surgical procedures are completed.

In some embodiments, because the auxiliary procedure has a certain effective area, when performing multiple surgical procedures on a patient, only one auxiliary procedure may be performed. That is, one auxiliary procedure and the multiple surgical procedures are performed on the patient. For example, for multiple entry points, an auxiliary procedure may be performed only once at the center point of the distribution of the multiple entry points, and then the multiple surgical procedures are performed separately on each entry point. In this case, after completing step 2360, steps 2370 and 2380 may be repeated sequentially until surgical procedures for all entry points are completed. For more details on multiple surgical procedures, please refer to the previous related description, which will not be repeated here.

In some embodiments, after completing a portion of a surgical procedure, it may be necessary to move the scanning table 242 out of the medical radiation imaging system 220 for an intermediate operation (e.g., checking the patient, operating the medical radiation imaging system 220, etc.) or for an intermission, before resuming the surgical procedure. That is, after completing a portion of the surgical procedure, the processor may output a fourth signal, which may control the scanning table 242 to move from the fourth position to a fifth position. When a preset condition is met (e.g., the intermediate operation is completed, the intermission is over, or the scanning table 242 has been at the fifth position for a preset duration), the user may input a continue signal or the processor may output the continue signal. In response to the continue signal, the processor (e.g., the first processor in the active control end 230, the second processor in the operation end effector 240, etc.) may output a seventh signal, which is used to control the scanning table 242 to move from the fifth position back to the fourth position. It should be noted that during the movement of the scanning table 242 from the fourth position to the fifth position and back to the fourth position, the patient's position remains unchanged.

In 2390, a surgery ends.

After the surgical procedure is completed, the user may adjust the navigation support arm 2413 to stow the navigation support arm 2413. After confirming that the navigation support arm 2413 is stowed, the surgery ends. In some embodiments, in response to at least partial completion of the surgical procedure, a processor (e.g., the first processor in the active control end 230, the second processor in the operation end effector 240, etc.) may output a fourth signal. The fourth signal may be used to control the scanning table 242 to move from the fourth position to a fifth position. The fifth position is located outside the imaging area of the medical radiation imaging system 220. In some embodiments, the fifth position may be the same as or different from the first position. In some embodiments, in response to completion of all surgical procedures, the processor may output the fourth signal, which may be used to control the scanning table 242 to move from the fourth position to the fifth position.

It should be understood that the illustrated system and its modules may be implemented in various ways. For example, in some embodiments, the system and its modules may be implemented by hardware, software, or a combination of software and hardware. The hardware portion may be implemented using dedicated logic; the software portion may be stored in a memory and executed by an appropriate instruction execution system, such as a microprocessor or dedicated design hardware. Those skilled in the art will appreciate that the above methods and systems may be implemented using computer-executable instructions and/or embodied in processor control code, such as code provided on a carrier medium such as a magnetic disk, CD, or DVD-ROM, a programmable memory such as read-only memory (firmware), or a data carrier such as an optical or electronic signal carrier. The system and its modules of the present application may be implemented not only by hardware circuits such as very large scale integrated circuits or gate arrays, semiconductors such as logic chips, transistors, or the like, or programmable hardware devices such as field programmable gate arrays, programmable logic devices, or the like, but also by software executed by various types of processors, or by a combination of the above hardware circuits and software (e.g., firmware).

The basic concepts have been described above, and it is apparent to those skilled in the art that the foregoing detailed disclosure serves only as an example and does not constitute a limitation of the present disclosure. While not expressly stated herein, a person skilled in the art may make various modifications, improvements, and amendments to the present disclosure. Such modifications, improvements, and amendments are suggested in the present disclosure, so such modifications, improvements, and amendments remain within the spirit and scope of the exemplary embodiments of the present disclosure.

Also, the present disclosure uses specific words to describe embodiments of the present disclosure. Such as "an embodiment", "an embodiment", and/or "some embodiment" means a feature, structure, or characteristic associated with at least one embodiment of the present disclosure. Accordingly, it should be emphasized and noted that "one embodiment" or "an embodiment" or "an alternative embodiment" in different places in the present disclosure do not necessarily refer to the same embodiment. In addition, certain features, structures, or characteristics of one or more embodiments of the present disclosure may be suitably combined.

Similarly, it should be noted that in order to simplify the presentation of the present disclosure of the disclosure, and thereby aid in the understanding of one or more embodiments of the invention, the foregoing descriptions of embodiments of the present disclosure sometimes combine a variety of features into a single embodiment, accompanying drawings, or descriptions thereof. However, this method of disclosure does not imply that more features are required for the objects of the present disclosure than are mentioned in the claims. Rather, claimed subject matter can lie in less than all features of a single foregoing disclosed embodiment.

Some embodiments use numbers to describe the number of components, attributes, and it should be understood that such numbers used in the description of the embodiments are modified in some examples by the modifiers "about", "approximately", or "substantially". ", "approximately", or "generally" is used in some examples. Unless otherwise noted, the terms "about," "approximate," or "approximately" indicates that a ±20% variation in the stated number is allowed. Correspondingly, in some embodiments, the numerical parameters used in the disclosure and claims are approximations, which approximations are subject to change depending on the desired characteristics of individual embodiments. In some embodiments, the numerical parameters should take into account the specified number of valid digits and utilize a general digit retention method. While the numerical domains and parameters used to confirm the breadth of their ranges in some embodiments of the present disclosure are approximations, in specific embodiments such values are set to be as precise as possible within a feasible range.

For each of the patents, patent applications, patent application disclosures, and other materials cited in the present disclosure, such as articles, books, disclosure sheets, publications, documents, or the like, are hereby incorporated by reference in their entirety into the present disclosure. Application history documents that are inconsistent with or conflict with the contents of the present disclosure are excluded, as are documents (currently or hereafter appended to the present disclosure) that limit the broadest scope of the claims of the present disclosure. It should be noted that in the event of any inconsistency or conflict between the descriptions, definitions, and/or use of terms in the materials appended to the present disclosure and those set forth herein, the descriptions, definitions and/or use of terms in the present disclosure shall control use shall prevail. Finally, it should be understood that the embodiments described in the present disclosure are intended only to illustrate the principles of the embodiments of the present disclosure. Other deformations can also fall within the scope of the present disclosure. As such, alternative configurations of embodiments of the present disclosure may be considered to be consistent with the teachings of the present disclosure as an example, not as a limitation. Correspondingly, embodiments of the present disclosure are not limited to those expressly presented and described herein.

The described embodiments are only meant to illustrate the principles of the application. Other variations may still fall within the scope of the application. Alternative configurations consistent with the teachings of the application are possible (as examples, not limitations). Therefore, the embodiments are not limited to those explicitly described.

## Claims

1. An interventional medical system, comprising a processor and an operation end effector, wherein the operation end effector comprises a surgical execution system, and the processor is configured to:
perform a spatial registration on the interventional medical system to obtain registration information;
acquire relevant information and surgical information of a patient, and
determine a procedure plan for the surgical execution system based on the registration information, the relevant information, and the surgical information;
control the surgical execution system to perform one or more procedures based on the surgical information and the procedure plan.

2. The interventional medical system according to claim 1, wherein the interventional medical system is configured to be used with a medical radiation imaging system, and the surgical execution system comprises a base and a surgical operation assembly disposed on the base; the performing spatial registration on the interventional medical system to obtain registration information comprises:
establishing a first coordinate system of the base, a second coordinate system of the medical radiation imaging system, and a third coordinate system of a reference point; and
determining a first mapping relationship between the first coordinate system and the second coordinate system based on a mapping relationship between the first coordinate system and the third coordinate system and a mapping relationship between the second coordinate system and the third coordinate system.

3. The interventional medical system according to claim 2, wherein the surgical operation assembly comprises a surgical execution arm and an end effector disposed at a distal end of the surgical execution arm, and the reference point is disposed at the distal end of the surgical execution arm.

4. The interventional medical system according to 2, wherein the surgical operation assembly comprises a surgical execution arm and an end effector disposed at a distal end of the surgical execution arm, the end effector comprises an execution mount, the execution mount being detachably connected with the distal end of the surgical execution arm, and the reference point is disposed on the execution mount;
the processor is further configured to perform the spatial registration on the interventional medical system after the execution mount is installed onto the surgical execution arm.

5. The interventional medical system according to 2, wherein the surgical operation assembly comprises a surgical execution arm and an end effector disposed at a distal end of the surgical execution arm, the end effector comprises an execution mount and an execution instrument, the execution mount being detachably connected with the surgical execution arm, the execution instrument being detachably connected with the execution mount, and the reference point is disposed on the execution instrument;
the processor is further configured to perform the spatial registration on the interventional medical system after the execution mount is installed onto the surgical execution arm and the execution instrument is installed onto the execution mount.

6. The interventional medical system according to claim 2, wherein the surgical operation assembly comprises a surgical execution arm and an end effector disposed at a distal end of the surgical execution arm, the end effector comprises an execution mount, and the reference point is disposed on a registration tool, the registration tool being detachably connected with the execution mount;
the registration tool is installed onto the execution mount before the spatial registration, and is detached from the execution mount after the spatial registration is completed;
the processor is further configured to perform the spatial registration on the interventional medical system after the execution mount is installed onto the surgical execution arm and the registration tool is installed onto the execution mount.

7. The interventional medical system according to claim 2, wherein the surgical operation assembly comprises a surgical execution arm and an end effector disposed at a distal end of the surgical execution arm, and the performing spatial registration on the interventional medical system to obtain registration information further comprises:
determining a second mapping relationship between a patient coordinate system of the patient and the second coordinate system; and
establishing a fourth coordinate system of the end effector; and
determining a third mapping relationship between the fourth coordinate system and the first coordinate system.

8. The interventional medical system according to claim 2, wherein the interventional medical system comprises an auxiliary indicator, and the performing spatial registration on the interventional medical system to obtain registration information further comprises:
determining a second mapping relationship between a patient coordinate system of the patient and the second coordinate system;
establishing a fifth coordinate system of the auxiliary indicator; and
determining a fourth mapping relationship between the fifth coordinate system and the first coordinate system.

9. The interventional medical system according to claim 7 or 8, wherein the surgical operation assembly comprises the surgical execution arm, and the procedure plan comprises an operation pose and an executable path for the surgical execution arm; the determining a procedure plan for the surgical execution system based on the registration information, the relevant information, and the surgical information comprises:
determining the operation pose based on the relevant information and the surgical information;
determining target joint parameters of the surgical execution arm based on the operation pose; and
determining the executable path based at least on the target joint parameters.

10. The interventional medical system according to claim 9, wherein the interventional medical system is configured to be used with a medical radiation imaging system, the interventional medical system comprises an optical imaging system, and the relevant information comprises a medical image and a first optical image of the patient;
the acquiring relevant information of a patient and surgical information comprises:
acquiring the medical image of the patient from the medical radiation imaging system; and
controlling the optical imaging system to acquire the first optical image of the patient.

11. The interventional medical system according to claim 10, wherein the determining a procedure plan for the surgical execution system based on the registration information, the relevant information, and the surgical information comprises:
determining a contour model of the patient based on the medical image and the first optical image;
determining an initial planned pose based on the contour model and the surgical information;
determining a simulated execution path for the surgical execution arm based on the initial planned pose;
verifying the simulated execution path based on the contour model and the initial planned pose; and
when verification passes, determining the initial planned pose as the operation pose, and determining the simulated execution path as the executable path.

12. The interventional medical system according to claim 11, wherein the determining the initial planned pose based on the contour model and the surgical information comprises:
determining an entry point and a target point based on the contour model and the surgical information; and
determining the initial planned pose based on the entry point and the target point.

13. The interventional medical system according to claim 12, wherein
the medical radiation imaging system further comprises a scanning table, the scanning table having a translational degree of freedom to move relative to an imaging area of the medical radiation imaging system;
the surgical operation assembly comprises the surgical execution arm and an end effector disposed at the distal end of the surgical execution arm,
the one or more procedures include a surgical procedure, the operation pose of the surgical execution arm comprises a surgical operation pose of the surgical execution arm, the initial planned pose comprises an initial planned surgical pose, the simulated execution path comprises a simulated surgical execution path, and the executable path comprises a surgical executable path; when verification passes, the initial planned surgical pose is determined as the surgical operation pose, and the simulated surgical execution path is determined as the surgical executable path;
the determining the initial planned pose based on the entry point and the target point comprises:
acquiring first position information of the scanning table when the scanning table is at a first position, the first position being a position of the scanning table when performing the spatial registration on the interventional medical system;
acquiring fourth position information of the scanning table when the scanning table is at a fourth position, the fourth position being determined based on the target point;
determining first translation information based on the first position information and the fourth position information; and
determining the initial planned surgical pose based on the entry point, the target point, the first translation information, the first mapping relationship, the second mapping relationship, and the third mapping relationship.

14. The interventional medical system according to claim 13, wherein the determining a simulated execution path for the surgical execution arm based on the initial planned pose comprises:
determining an initial positioning point based on the entry point and the initial planned surgical pose, wherein when verification passes, the initial positioning point is determined as a navigation positioning point; and
determining the simulated surgical execution path based on the navigation positioning point and the initial planned surgical pose.

15. The interventional medical system according to claim 12, wherein
the medical radiation imaging system further comprises a scanning table, the scanning table having a translational degree of freedom to move relative to an imaging area of the medical radiation imaging system;
the interventional medical system comprises the auxiliary indicator, the surgical operation assembly comprises the surgical execution arm and the end effector disposed at the distal end of the surgical execution arm, the auxiliary indicator is disposed on the surgical execution arm or on the end effector,
the one or more procedures include an auxiliary procedure, the operation pose of the surgical execution arm comprises an auxiliary operation pose of the surgical execution arm, the initial planned pose comprises an initial planned auxiliary pose, the simulated execution path comprises a simulated auxiliary execution path, and the executable path comprises an auxiliary executable path; when verification passes, the initial planned auxiliary pose is determined as the auxiliary operation pose, and the simulated auxiliary execution path is determined as the auxiliary executable path;
the determining the initial planned pose based on the entry point and the target point comprises:
acquiring first position information of the scanning table when the scanning table is at a first position, the first position being a position of the scanning table when performing spatial registration on the interventional medical system;
acquiring third position information of the scanning table when the scanning table is at a third position, the third position being within an indication area of the auxiliary indicator;
determining second translation information based on the fourth position information and the third position information; and
determining the initial planned auxiliary pose based on the entry point, the target point, the second translation information, the first mapping relationship, the second mapping relationship, and the fourth mapping relationship.

16. The interventional medical system according to claim 13 or 14, wherein the interventional medical system comprises the auxiliary indicator, and the surgical execution system further comprises a navigation support arm, the auxiliary indicator is mounted on the navigation support arm ;
the determining a procedure plan for the surgical execution system based on the registration information, the relevant information, and the surgical information further comprises:
determining an auxiliary indication pose of the navigation support arm based on the surgical operation pose;
wherein determining the auxiliary indication pose of the navigation support arm based on the surgical operation pose comprises:
determining a third position of the scanning table based on the surgical operation pose;
acquiring third position information of the scanning table when the scanning table is at the third position, wherein the third position is within an indication area of the auxiliary indicator;
determining third translation information based on the fourth position information and the third position information; and
determining the auxiliary indication pose based on the fourth mapping relationship and the third translation information.

17. The interventional medical system according to claim 1, wherein the one or more procedures include a surgical procedure, the interventional medical system is configured to be used with a medical radiation imaging system, the medical radiation imaging system comprises a scanning table, the surgical execution system comprises a surgical execution arm and an end effector mounted at a distal end of the surgical execution arm, and the procedure plan comprises a surgical operation pose of the surgical execution arm;
the controlling the surgical execution system to perform the one or more procedures based on surgical information and the procedure plan comprises:
in response to completion of the spatial registration, controlling the scanning table to move to a second position, wherein the second position is a position of the scanning table when the medical radiation imaging system scans the patient;
in response to completion of scanning the patient by the medical radiation imaging system, controlling the scanning table to move to a fourth position, the fourth position being a position of the scanning table when the patient undergoes the surgical procedure;
in response to the scanning table moving to the fourth position, controlling the surgical execution arm to adjust to the surgical operation pose; and
in response to the surgical execution arm adjusting to the surgical operation pose, controlling the end effector to perform the surgical procedure.

18. The interventional medical system according to claim 17, wherein the surgical execution system comprises an end effector disposed at a distal end of the surgical execution arm, the end effector comprises an execution mount detachably connected with the surgical execution arm, the execution mount being configured to mount an execution instrument required for the surgical procedure;
the controlling the surgical execution arm to adjust to the surgical operation pose in response to the scanning table moving to the fourth position comprises:
in response to the scanning table moving to the fourth position, controlling the surgical execution arm to adjust to an installation pose, the installation pose being a feasible pose for the surgical execution arm to avoid the patient;
in response to the surgical execution arm adjusting to the installation pose, controlling the execution mount to install the execution instrument; and
in response to completion of installation of the execution instrument, controlling the surgical execution arm to adjust to the surgical operation pose.

19. The interventional medical system according to claim 17, wherein the interventional medical system comprises an auxiliary indicator, and the one or more procedure comprises an auxiliary procedure;
the controlling the scanning table to move to a fourth position in response to completion of scanning the patient by the medical radiation imaging system comprises:
in response to completion of scanning the patient by the medical radiation imaging system, controlling the scanning table to move to a third position, wherein the third position is a position of the scanning table when the patient undergoes the auxiliary procedure;
in response to the scanning table moving to the third position, controlling the auxiliary indicator to provide an indication for the auxiliary procedure; and
in response to completion of the auxiliary procedure, controlling the scanning table to move to the fourth position.

20. The interventional medical system according to claim 19, wherein the auxiliary indicator is disposed on the surgical execution arm or on the end effector, and the procedure plan comprises an auxiliary operation pose of the surgical execution arm;
the controlling the auxiliary indicator to provide an indication for the auxiliary procedure in response to the scanning table moving to the third position comprises:
in response to the scanning table moving to the third position, controlling the surgical execution arm to adjust to the auxiliary operation pose; and
in response to the surgical execution arm adjusting to the auxiliary operation pose, controlling the auxiliary indicator to provide the indication for the auxiliary procedure.

21. The interventional medical system according to claim 19, wherein the surgical execution system further comprises a navigation support arm, the auxiliary indicator is disposed on the navigation support arm, and the procedure plan comprises an auxiliary indication pose of the navigation support arm;
the controlling the auxiliary indicator to provide the indication for the auxiliary procedure in response to the scanning table moving to the third position comprises:
in response to the scanning table moving to the third position, controlling the navigation support arm to adjust to the auxiliary indication pose;
in response to the navigation support arm adjusting to the auxiliary indication pose, controlling the auxiliary indicator to provide the indication for the auxiliary procedure.

22. The interventional medical system according to any one of claims 17-21, wherein the controlling the surgical execution system to perform one or more procedures based on surgical information and the procedure plan further comprises:
in response to at least partial completion of the surgical procedure, controlling the scanning table to move to a fifth position, wherein the fifth position is outside an imaging area of the medical radiation imaging system.

23. The interventional medical system according to claim 1, wherein the interventional medical system further comprises an active control end, and the processor is further configured to:
acquire a control command from the active control end; and
control the surgical execution system to perform the one or more procedures based on the control command.

24. The interventional medical system according to claim 23, wherein the acquiring a control command from an active control end, and controlling the surgical execution system to perform the one or more procedures based on the control command comprises:
monitoring whether the active control end receives enable information;
in response to the active control end receiving the enable information, acquiring the control command from the active control end; and
controlling the surgical execution system to perform the one or more procedures based on the control command.

25. The interventional medical system according to claim 24, wherein
the active control end comprises an exposure switch and a master manipulator connected with the surgical execution system,
the master manipulator comprises a control enable button, and the enable information comprises first enable information sent by the exposure switch and second enable information sent by the control enable button;
the acquiring the control command from the active control end in response to the active control end receiving enable information comprises:
in response to both the first enable information and the second enable information being acquired, acquiring the control command from the active control end.

26. The interventional medical system according to claim 1, wherein
the surgical execution system comprises a surgical execution arm and an end effector disposed at a distal end of the surgical execution arm, the end effector comprises an execution mount detachably connected with the surgical execution arm;
the processor is configured to:
at least after the execution mount is installed onto the surgical execution arm, start the interventional medical system; and
after the interventional medical system satisfies a preset condition, perform the spatial registration on the interventional medical system to obtain registration information.

27. The interventional medical system according to claim 1, wherein
the surgical execution system comprises a surgical execution arm and a motion enable switch,
the motion enable switch is configured to permit or prohibit movement of the surgical execution arm.

28. The interventional medical system according to claim 1, wherein
the interventional medical system further comprises a surgical field camera,
the surgical field camera is configured to acquire a second optical image of the patient and present the second optical image when the surgical execution system performs the one or more procedures.

29. The interventional medical system according to claim 1, wherein
the surgical information comprises a surgical mode,
the procedure plan comprises a navigation positioning point,
the one or more procedures comprises a surgical procedure,
the surgical execution system comprises a surgical operation assembly,
the controlling the surgical execution system to perform the one or more procedures based on the surgical information and the procedure plan comprises:
controlling the surgical operation assembly to move to the navigation positioning point;
after the surgical operation assembly moves to the navigation positioning point, controlling the surgical operation assembly to perform the surgical procedure based on the surgical mode.

30. An interventional medical system, comprising a processor, wherein the interventional medical system is configured to be used with a medical radiation imaging system, the medical radiation imaging system comprises a scanning table, and the processor is configured to:
in response to a start signal, perform a spatial registration on the interventional medical system;
in response to completion of the spatial registration, control the scanning table to move into an imaging area of the medical radiation imaging system to a second position, the second position being a position of the scanning table when the medical radiation imaging system scans a patient; and
in response to completion of scanning the patient by the medical radiation imaging system, control the scanning table to move to an execution position, the patient undergoing one or more procedures at the execution position.

31. The interventional medical system according to claim 30, wherein
the interventional medical system comprises an end effector, the one or more procedures include one or more surgical procedures, and the execution position comprises one or more fourth positions;
the controlling the scanning table to move to an execution position in response to completion of scanning the patient by the medical radiation imaging system, the patient undergoing one or more procedures at the execution position comprises:
in response to completion of scanning the patient by the medical radiation imaging system, controlling the scanning table to move to the one or more fourth positions, the fourth positions being positions of the scanning table when the patient undergoes the one or more surgical procedures; and
in response to the scanning table moving to the one or more fourth positions, controlling the end effector to perform the one or more surgical procedures.

32. The interventional medical system according to claim 31, wherein the processor is further configured to:
in response to completion of one of the one or more surgical procedures when the scanning table is at one of the one or more fourth positions, and the one or more surgical procedures not being fully completed, control the scanning table to move to a fifth position, the fifth position being outside the imaging area of the medical imaging system;
in response to a continue signal, control the scanning table to move to another fourth position, and control the end effector to continue performing the one or more surgical procedures.

33. The interventional medical system according to claim 31, wherein
the interventional medical system further comprises a surgical execution arm and an auxiliary indicator,
the end effector is disposed at a distal end of the surgical execution arm,
the one or more procedures further include one or more auxiliary procedures, and
the execution position further comprises one or more third positions;
the controlling the scanning table to move to the one or more fourth positions in response to completion of scanning the patient by the medical radiation imaging system comprises:
in response to completion of scanning the patient by the medical radiation imaging system, controlling the scanning table to move to the one or more third positions, wherein the one or more third positions is one or more positions of the scanning table when the patient undergoes the one or more auxiliary procedures;
in response to the scanning table moving to the one or more third positions, controlling the auxiliary indicator to provide an indication for the one or more auxiliary procedures;
in response to completion of the auxiliary procedures, controlling the scanning table to move to one of the one or more fourth positions.

34. The interventional medical system according to claim 33, wherein the processor is further configured to:
in response to completion of the one of the one or more surgical procedures when the scanning table is at the one of the one or more fourth positions, and the one or more surgical procedures not being fully completed, control the scanning table to move to the one or more third positions.

35. The interventional medical system according to claim 33, wherein the processor is further configured to:
in response to completion of the one of the one or more surgical procedures when the scanning table is at the one of the fourth positions, and the one or more surgical procedures not being fully completed, control the scanning table to move to another fourth position.

36. The interventional medical system according to any one of claims 30-35, wherein the processor is further configured to:
in response to completion of all of the one or more surgical procedures, control the scanning table to move to a fifth position, the fifth position being outside the imaging area of the medical imaging system.

37. The interventional medical system according to claim 31, wherein
the interventional medical system comprises a surgical execution arm and the end effector disposed at a distal end of the surgical execution arm, the end effector comprises an execution mount detachably connected with the surgical execution arm;
the performing spatial registration on the interventional medical system in response to a start signal comprises:
in response to the start signal, controlling the surgical execution arm to install the execution mount; and
in response to completion of installation of the execution mount, performing the spatial registration on the interventional medical system.

38. The interventional medical system according to claim 37, wherein the execution mount is configured to mount an execution instrument required for the surgical operation;
the controlling the end effector to perform the surgical operation in response to the scanning table moving to the one or more fourth positions comprises:
in response to the scanning table moving to the one of the fourth positions, controlling the surgical execution arm to adjust to an installation pose, the installation pose being a feasible pose for the surgical execution arm to avoid the patient;
in response to the surgical execution arm adjusting to the installation pose, controlling the execution mount to install the execution instrument;
in response to completion of the installation of the execution instrument, controlling the surgical execution arm to adjust to a surgical operation pose;
in response to the surgical execution arm adjusting to the surgical operation pose, controlling the execution instrument to perform the one of the one or more surgical procedures.

39. The interventional medical system according to claim 38, wherein the controlling the surgical execution arm to adjust to a surgical operation pose in response to completion of installation of the execution instrument comprises:
in response to completion of the installation of the execution instrument, controlling the surgical execution arm to adjust to an initial pose; and
in response to the surgical execution arm adjusting to the initial pose, controlling the surgical execution arm to adjust to the surgical operation pose.

40. The interventional medical system according to claim 38, wherein the controlling the execution instrument to perform the one of the one or more surgical procedures in response to the surgical execution arm adjusting to the surgical operation pose comprises:
in response to the surgical execution arm adjusting to the surgical operation pose, controlling the execution mount to adjust a pose of the execution instrument;
in response to completion of the adjustment of the pose of the execution instrument, controlling the execution instrument to perform the one of the one or more surgical procedures.

41. The interventional medical system according to claim 38, wherein
the end effector further comprises an instrument confirmation control,
the instrument confirmation control is configured to output a confirmation signal in response to the completion of the installation of the execution instrument;
the controlling the surgical execution arm to adjust to a surgical operation pose in response to the completion of the installation of the execution instrument comprises:
in response to the completion of the installation of the execution instrument, controlling the instrument confirmation control to output the confirmation signal;
controlling the surgical execution arm to adjust to the surgical operation pose based on the confirmation signal.

42. The interventional medical system according to claim 38, wherein the end effector further comprises a manual control, the manual control being configured to control the execution mount to lock or release the execution instrument.

43. The interventional medical system according to claim 38, wherein the processor is further configured to:
in response to completion of all of the one or more surgical procedures, controlling the surgical execution arm to adjust to an end pose, the end pose being the same as or different from an initial pose of the surgical execution arm.

44. An interventional medical system, comprising a processor and an operation end effector, wherein the operation end effector comprises a surgical execution system, and the processor is configured to:
acquire relevant information and surgical information of a patient ;
determine a procedure plan based at least on the relevant information and the surgical information;
control the surgical execution system to perform one or more procedures based on the surgical information and the procedure plan;
wherein the surgical information comprises a surgical mode, the procedure plan comprises a navigation positioning point, the one or more procedures include one or more surgical procedures, the surgical execution system comprises a surgical operation assembly, and the controlling the surgical execution system to perform one or more procedures based on the surgical information and the procedure plan comprises:
controlling the surgical operation assembly to move to the navigation positioning point; and
after the surgical operation assembly moves to the navigation positioning point, controlling the surgical operation assembly to perform the one or more surgical procedures based on the surgical mode.

45. The interventional medical system according to claim 44, wherein
the interventional medical system is configured to be used with a medical radiation imaging system,
the surgical mode comprises at least one of a real-time control mode and a breakpoint control mode;
the surgical operation assembly comprises a surgical execution arm, an end effector disposed at a distal end of the surgical execution arm, and an execution instrument disposed at the end effector;
the controlling the surgical execution system to perform one or more procedures based on the surgical information and the procedure plan comprises:
determining the execution instrument based on the surgical information and the procedure plan;
installing the execution instrument onto the end effector; and
after installation of the execution instrument is completed, controlling the surgical execution arm to drive the end effector to move to the navigation positioning point.

46. The interventional medical system according to claim 45, wherein
the interventional medical system further comprises an active control end connected with the operation end effector,
the active control end comprises an exposure switch and a master manipulator connected with the surgical execution system,
when the surgical mode comprises the real-time control mode, the processor is configured to:
acquire a control command issued by the master manipulator, and control the execution instrument to perform the one or more surgical procedures based on the control command;
acquire first enable information sent by the exposure switch; and
based on the real-time control mode and the first enable information, scan the patient in real-time via the medical radiation imaging system to acquire a real-time image of the patient;
the controlling the surgical operation assembly to perform the one or more surgical procedures based on the surgical mode comprises:
controlling the execution instrument to perform the one or more surgical procedures via the master manipulator based on the real-time control mode and the real-time image.

47. The interventional medical system according to claim 45, wherein
the interventional medical system further comprises an active control end connected with the operation end effector, and the controlling the surgical operation assembly to perform the one or more surgical procedures based on the surgical mode comprises:
controlling the execution instrument to perform the one or more surgical procedures via the active control end based on the real-time control mode or the breakpoint control mode.

48. The interventional medical system according to claim 45, wherein when the surgical mode comprises the breakpoint control mode, the controlling the surgical operation assembly to perform the one or more surgical procedures based on the surgical mode comprises:
based on the breakpoint control mode, scanning the patient via the medical radiation imaging system to obtain a first surgical image;
based on the breakpoint control mode and the first surgical image, controlling the execution instrument to perform a portion of the one or more surgical procedures corresponding to the first surgical image;
after the portion of the one or more surgical procedures is completed, continuing to scan the patient via the medical radiation imaging system to obtain a second surgical image;
based on the breakpoint control mode and the second surgical image, controlling the execution instrument to perform a next portion of the one or more surgical procedures corresponding to the second surgical image.

49. The interventional medical system according to claim 48, wherein
the end effector comprises a clamping structure,
the execution instrument is installed in the clamping structure,
the active control end comprises a master manipulator connected with the surgical execution system,
the master manipulator comprises a control enable button, and when the surgical mode comprises the breakpoint control mode, the control enable button is used to control a clamping state of the clamping structure on the execution instrument;
when the surgical mode comprises the breakpoint control mode, the processor is configured to:
acquire a control command issued by the master manipulator;
control the execution instrument to perform the one or more surgical procedures based on the control command;
acquire second enable information sent by the control enable button;
control the clamping structure to adjust to a clamped state based on the second enable information, the second enable information enabling the control command to take effect;
acquire a scan signal, and scan the patient via the medical radiation imaging system to obtain surgical images, the surgical images comprising at least the first surgical image and the second surgical image;
the controlling the surgical operation assembly to perform the one or more surgical procedures based on the surgical mode comprises:
based on the breakpoint control mode and the surgical images, controlling the execution instrument to perform the portion of the one or more surgical procedures corresponding to the surgical images via the master manipulator.

50. The interventional medical system according to claim 45, wherein
the surgical mode comprises the breakpoint control mode and the real-time control mode, and the processor is further configured to switch the surgical mode between the breakpoint control mode and the real-time control mode.

51. The interventional medical system according to claim 50, wherein the controlling the surgical operation assembly to perform the one or more surgical procedures based on the surgical mode comprises:
based on the breakpoint control mode, scanning the patient via the medical radiation imaging system to obtain a first surgical image;
based on the breakpoint control mode and the first surgical image, controlling the execution instrument to perform a portion of the one or more surgical procedures corresponding to the first surgical image;
after the portion of the one or more surgical procedures is completed, controlling the processor to switch from the breakpoint control mode to the real-time control mode;
based on the real-time control mode, scanning the patient in real-time via the medical radiation imaging system to acquire a real-time image of the patient;
based on the real-time control mode and the real-time image, controlling the execution instrument to continue performing the one or more surgical procedures.

52. The interventional medical system according to claim 50, wherein the controlling the surgical operation assembly to perform the one or more surgical procedures based on the surgical mode comprises:
based on the real-time control mode, scanning the patient in real-time via the medical radiation imaging system to acquire a real-time image of the patient;
based on the real-time control mode and the real-time image, controlling the execution instrument to perform a portion of the one or more surgical procedures;
after the portion of the one or more surgical procedures is completed, controlling the processor to switch from the real-time control mode to the breakpoint control mode;
based on the breakpoint control mode, scanning the patient via the medical radiation imaging system to obtain a first surgical image;
based on the breakpoint control mode and the first surgical image, controlling the execution instrument to perform a portion of the one or more surgical procedures corresponding to the first surgical image among a remaining portion of the one or more surgical procedures;
after the portion of the one or more surgical procedures is completed, continuing to scan the patient via the medical radiation imaging system to obtain a second surgical image;
based on the breakpoint control mode and the second surgical image, controlling the execution instrument to perform a next portion of the one or more surgical procedures corresponding to the second surgical image.

53. The interventional medical system according to claim 44, wherein
the surgical mode comprises a manual control mode;
the surgical operation assembly comprises a surgical execution arm, an end effector disposed at a distal end of the surgical execution arm, and an execution instrument disposed at the end effector;
the controlling the surgical execution system to perform one or more procedures based on the surgical information and the procedure plan comprises:
determining the execution instrument based on the surgical information and the procedure plan;
controlling the surgical execution arm to drive the end effector to move to the navigation positioning point; and
installing the execution instrument onto the end effector.

54. The interventional medical system according to claim 53, wherein the controlling the surgical operation assembly to perform the one or more surgical procedures based on the surgical mode comprises:
based on the manual control mode, manually controlling the execution instrument to perform the one or more surgical procedures.

55. The interventional medical system according to claim 44, wherein
the one or more procedures comprises multiple surgical procedures,
the surgical mode comprises a real-time control mode or a breakpoint control mode, and
the surgical operation assembly comprises a surgical execution arm, an end effector disposed at a distal end of the surgical execution arm, and an execution instrument disposed at the end effector;
the controlling the surgical operation assembly to perform the one or more surgical procedures based on the surgical mode comprises:
installing an execution instrument corresponding to a current surgical procedure onto the end effector;
controlling the surgical execution arm to drive the end effector to move to a navigation positioning point corresponding to the current surgical procedure;
controlling the execution instrument to perform the current surgical procedure based on the real-time control mode or the breakpoint control mode;
after the current surgical procedure is completed, moving the surgical execution arm to an installation pose;
installing an execution instrument corresponding to a next surgical procedure onto the end effector;
controlling the surgical execution arm to drive the end effector to move to a navigation positioning point corresponding to the next surgical procedure;
controlling the execution instrument to perform the next surgical procedure based on the real-time control mode or the breakpoint control mode.

56. The interventional medical system according to claim 55, wherein
the medical radiation imaging system further comprises a scanning table,
each of the multiple surgical procedures corresponds to a fourth position of the scanning table, the fourth position being within a scanning area of the medical radiation imaging system;
the controlling the surgical operation assembly to perform the one or more surgical procedures based on the surgical mode comprises:
moving the scanning table to the fourth position corresponding to a first surgical procedure;
when the fourth position corresponding to the current surgical procedure is different from another fourth position corresponding to the next surgical procedure,
the controlling the surgical operation assembly to perform the surgical procedure based on the surgical mode further comprises:
after the current surgical procedure is completed, moving the scanning table to the other fourth position corresponding to the next surgical procedure.

57. The interventional medical system according to claim 44, wherein
the one or more procedure include multiple surgical procedures,
the surgical mode comprises a manual control mode,
the medical radiation imaging system further comprises a scanning table,
the multiple surgical procedures collectively correspond to a single fourth position of the scanning table,
the fourth position is outside a scanning area of the medical radiation imaging system;
the surgical operation assembly comprises a surgical execution arm, an end effector disposed at a distal end of the surgical execution arm, and an execution instrument disposed at the end effector;
the controlling the surgical operation assembly to perform the one or more surgical procedures based on the surgical mode comprises:
moving the scanning table to the fourth position;
controlling the surgical execution arm to drive the end effector to move to a navigation positioning point corresponding to a current surgical procedure;
installing an execution instrument corresponding to the current surgical procedure onto the end effector;
controlling the execution instrument to perform the current surgical procedure based on the manual control mode;
after the current surgical procedure is completed, controlling the surgical execution arm to drive the end effector to move to a navigation positioning point corresponding to a next surgical procedure;
installing an execution instrument corresponding to the next surgical procedure onto the end effector; and
controlling the execution instrument to perform the next surgical procedure based on the manual control mode.

58. The interventional medical system according to claim 57, wherein the controlling the surgical operation assembly to perform the one or more surgical procedures based on the surgical mode further comprises:
after all or part of the current surgical procedure is completed, moving the patient to a second position, the second position being a position of the scanning table when the medical radiation imaging system scans the patient;
after the scanning table moves to the second position, acquiring a new medical image of the patient via the medical radiation imaging system;
determining a new procedure plan for remaining surgical procedures based on the new medical image and the procedure plan for the remaining surgical procedures; and
completing the remaining surgical procedures based on the new procedure plan.

59. The interventional medical system according to claim 58, wherein when the current surgical procedure is fully completed, the completing the remaining surgical procedures based on the new procedure plan comprises:
based on the new procedure plan, manually controlling the execution instrument to perform the remaining surgical procedures.

60. The interventional medical system according to claim 58, wherein when the current surgical procedure is partially completed, the completing the remaining surgical procedures based on the new procedure plan comprises:
based on the new procedure plan, manually completing an uncompleted portion of the current surgical procedure; and
after the current surgical procedure is completed, manually controlling the execution instrument to perform the remaining surgical procedures.

61. The interventional medical system according to claim 44, wherein
the one or more procedures further comprises one or more auxiliary procedures,
the interventional medical system further comprises an auxiliary indicator, and
the surgical operation assembly comprises a surgical execution arm and an end effector disposed at a distal end of the surgical execution arm;
the controlling the surgical execution system to perform one or more procedures based on the surgical information and the procedure plan comprises:
based on the surgical information and the procedure plan, controlling the auxiliary indicator to provide an indication for the one or more auxiliary procedures;
after the one or more auxiliary procedures is completed, controlling the surgical operation assembly to perform the one or more surgical procedures.

62. The interventional medical system according to claim 44, wherein
the interventional medical system further comprises an active control end,
the active control end comprises an operation interface configured to achieve at least one of the following:
displaying and processing the relevant information and the surgical information of the patient;
displaying and inputting the procedure plan;
displaying prompt information.

63. The interventional medical system according to claim 44, wherein the surgical operation assembly comprises a surgical execution arm and an end effector disposed at a distal end of the surgical execution arm, the end effector comprises an execution mount detachably connected with the surgical execution arm, and the processor is configured to
initiate the interventional medical system at least after the execution mount is installed onto the surgical execution arm; and
perform a spatial registration on the interventional medical system to obtain registration information after the interventional medical system satisfies a preset condition.
